# EUROPÄISCHE PATENTSCHRIFT

(11) **EP 0 525 584 B1**
(45) Veröffentlichungstag und Bekanntmachung des Hinweises auf die Patenterteilung: **10.12.1997**
(21) Anmeldenummer: 92112431.9
(22) Anmeldetag: 21.07.1992
(51) Int. Cl.: C07D 487/06, C07D 209/10, A61K 31/55

(54) **6-Oxo-azepinoindol-Verbindungen sowie Verfahren und Zwischenprodukte zu ihrer Herstellung und diese Verbindungen enthaltende Arzneimittel**
6-Oxo-azepinoindole derivatives, process and intermediates for their preparation and medicaments containing them
Dérivés du 6-oxoazépinoindole, procédé et intermédiaires pour leur préparation et compositions pharmaceutiques les contenant

(30) Priorität: 31.07.1991 DE 4125292
(43) Veröffentlichungstag der Anmeldung: 03.02.1993
(73) Patentinhaber: Solvay Pharmaceuticals GmbH, 30173 Hannover (DE)
(72) Erfinder: Benson, Werner, W-3016 Seelze OT Letter (DE); Van Charldorp, Karin, NL-1223 AK Hilversum (NL); Gregory, Peter Colin, W-3000 Hannover 71 (DE); Wolf, Klaus-Ullrich, W-3162 Hänigsen (DE); Preuschoff, Ulf, W-3110 Uelzen 5 (DE); Tulp, Martin, NL-1399 GJ Muiderberg (NL); Hulkenberg, Ton, NL-3752 HC Bunschoten (NL); Van Wijngaarden, Ineke, B-2360 Oud-Turnhout (BE)
(74) Vertreter: Lauer, Dieter, Dr.

(56) Entgegenhaltungen:
- US-A- 4 910 193
- JOURNAL OF MEDICINAL CHEMISTRY, Bd. 33, 1990, WASHINGTON, US; Seiten 633 - 641, R. D. CLARK ET AL.: '1,9-Alkano-bridged 2,3,4,5-tetrahydro-1H-3-benzazepines with affinity for the alpha-2-adrenoceptor and the 5-HT1A receptor'

## Beschreibung

Die vorliegende Erfindung betrifft neue, in 3- oder 4-Stellung des Ringgerüstes einen gegebenenfalls substituierten Aminoalkylrest tragende 6-Oxo-3,4,5,6-tetrahydro-1H-azepino[5,4,3-cd]indol-derivate und deren Salze sowie diese Verbindungen enthaltende pharmazeutische Zubereitungen und Verfahren und Zwischenprodukte zur Herstellung dieser Verbindungen.

In der japanischen Patentanmeldung Nr. 1 034 988 werden 6-Oxo-tetrahydroazepinoindole als Zwischenprodukte für die Herstellung von diuretisch wirksamen Tetrahydroazepinoindolen beschrieben. Aus der japanischen Patentanmeldung Nr. 57 144 286 sind Tetrahydroazepinoindole mit coronaren vasodilatorischen Eigenschaften bekannt.

Der vorliegenden Erfindung liegt die Aufgabe zugrunde, neue 6-Oxo-3,4,5,6-tetrahydro-1H-azepino[5,4,3-cd] indol-Verbindungen mit wertvollen pharmakologischen Eigenschaften herzustellen.

Es wurde nun gefunden, daß die neuen 6-Oxo-3,4,5,6-tetrahydro-1H-azepino[5,4,3-cd]indol-derivate, welche in 3- oder 4-Stellung des Ringgerüstes eine gegebenenfalls substituierte Aminomethyl- oder Aminoethylgruppe tragen, wertvolle pharmakologische Eigenschaften besitzen und insbesondere eine günstige pharmakologische Wirkung im gastrointestinalen Trakt zeigen und sich durch eine die Magenmotilität fördernde Wirkung auszeichnen. Die Verbindungen besitzen außerdem serotonin-agonistische Wirkungen an 5-HT₁-artigen Rezeptoren, welche ein Indiz für Migränezustände günstig beeinflussende Eigenschaften sind. Aufgrund ihrer Wirkungen eignen sich die erfindungsgemäßen Verbindungen zur Behandlung von Magenmotilitätsstörungen sowie zur Behandlung von Migräne.

Die vorliegende Erfindung betrifft daher neue Verbindungen der allgemeinen Formel I
(siehe Formel I)
worin
- R¹: für Wasserstoff, für eine niedere Alkyl- oder Cycloalkylalkylgruppe oder für eine Phenylniederalkylgruppe, welche gegebenenfalls im Phenylring durch niederes Alkoxy, Hydroxy, Halogen oder niederes Alkyl mono- oder disubstituiert sein kann, steht,
- R²: Wasserstoff oder eine gegebenenfalls in α-Stellung zu dem Stickstoffatom durch niederes Alkoxy substituierte niedere Alkylgruppe bedeutet,
- R³: Wasserstoff, niederes Alkyl, niederes Alkoxy, Halogen oder, falls die Substituenten R¹, R², R⁴ und/oder R⁵ keine niederen Alkoxygruppen enthalten, auch Hydroxy bedeutet,
- n: für 1 oder, falls die -(CH₂)ₙ-Kette in 4-Stellung des Ringgerüstes angeordnet ist, auch für 2 steht,
- R⁴: Wasserstoff, Alkyl mit 1 bis 5 Kohlenstoffatomen, Cycloalkyl mit 3 bis 6 Kohlenstoffatomen, Cycloalkylalkyl mit 4 bis 9 Kohlenstoffatomen oder eine gegebenenfalls im Phenylring durch niederes Alkyl, niederes Alkoxy, Hydroxy oder Halogen mono- oder disubstituierte Phenylniederalkylgruppe bedeutet, und
- R⁵: Wasserstoff, Alkyl mit 1 bis 5 Kohlenstoffatomen, Cycloalkyl mit 3 bis 6 Kohlenstoffatomen, Cycloalkylalkyl mit 4 bis 9 Kohlenstoffatomen oder eine gegebenenfalls im Phenylring durch niederes Alkyl, niederes Alkoxy, Hydroxy oder Halogen mono- oder disubstituierte Phenylniederalkylgruppe bedeutet, oder
- R⁴: und R⁵ gemeinsam mit dem Stickstoffatom, an welches sie gebunden sind, einen Heterocyclus der allgemeinen Formel a
(siehe Formel a)
bilden, worin
B für eine Bindung, die Methylengruppe, Sauerstoff oder eine Iminogruppe -NR⁶-, worin R⁶ Wasserstoff, niederes Alkyl oder gegebenenfalls im Phenylring durch niederes Alkyl, niederes Alkoxy, Hydroxy oder Halogen substituiertes Phenyl oder Benzyl bedeutet, steht, und
- D: eine Bindung oder, falls R⁴ und R⁵ nicht Wasserstoff bedeuten, auch die -N=CH-Gruppe darstellt,
und deren physiologisch verträgliche Säureadditionssalze.

Sofern in den Verbindungen der Formel I die Substituenten niedere Alkylgruppen enthalten, können diese Alkylgruppen gerade oder verzweigt sein und insbesondere 1 bis 4, vorzugsweise 1 bis 2 Kohlenstoffatome enthalten und stellen bevorzugt Methyl dar. Sofern die Substituenten Halogen bedeuten oder Halogensubstituenten enthalten, kommen insbesondere Fluor, Chlor oder Brom, vorzugsweise Chlor, in Frage.

Der Substituent R¹ stellt vorzugsweise Wasserstoff dar. Sofern R¹ eine niedere Alkylgruppe darstellt, kann diese geradkettig, verzweigt oder cyclisch sein und vorzugsweise 1 bis 4 Kohlenstoffatome enthalten und stellt insbesondere Methyl dar. Sofern R¹ eine Phenylniederalkylgruppe darstellt, kann deren Alkylenkette 1 bis 4 Kohlenstoffatome enthalten. Der Phenylring kann unsubstituiert sein oder durch niederes Alkoxy, vorzugsweise Methoxy, oder auch durch Halogen, Hydroxy oder niederes Alkyl mono- oder disubstituiert sein. Cycloalkylalkylreste R¹ können Cycloalkylgruppen mit 3 bis 6 Kohlenstoffatomen und eine Alkylenkette mit 1 bis 3 Kohlenstoffatomen enthalten.

Der Substituent R² stellt vorzugsweise Wasserstoff dar. Sofern R² eine niedere Alkylgruppe darstellt, kann diese 1 bis 4, insbesondere 1 oder 2 Kohlenstoffatome enthalten und ist vorzugsweise durch niederes Alkoxy, insbesondere Methoxy, substituiert und stellt beispielsweise die Methoxymethylgruppe dar.

Der Substituent in dem Azepinring ist vorzugsweise in 3-Stellung angeordnet. R⁴ und/oder R⁵ können Wasserstoff oder geradkettiges oder verzweigtes Alkyl mit bis zu 5 Kohlenstoffatomen, Cycloalkyl mit 3 bis 6 Kohlenstoffatomen, beispielsweise Cyclopropyl oder Cyclohexyl, oder Cycloalkylalkyl mit 4 bis 9, insbesondere 4 bis 7, Kohlenstoffatomen, beispielsweise Cyclopropylmethyl oder Cyclohexylmethyl, darstellen oder auch eine gegebenenfalls substituierte Phenylniederalkylgruppe bedeuten, deren Alkylenkette 1 bis 4 Kohlenstoffatome enthalten kann und deren Phenylring unsubstituiert oder durch niederes Alkoxy, insbesondere Methoxy, oder auch Halogen, Hydroxy oder niederes Alkyl mono- oder disubstituiert sein kann. Als günstig erweisen sich Substituenten des Azepin-Ringes worin D eine Bindung bedeutet und einer der Substituenten R⁴ und R⁵ Wasserstoff ist, beispielsweise R⁴ Wasserstoff oder eine Alkyl-, Cycloalkyl- oder Cycloalkylalkylgruppe mit bis zu 5 Kohlenstoffatomen bedeutet und R⁵ Wasserstoff bedeutet. Sofern R⁴ eine gegebenenfalls substituierte Phenylniederalkylgruppe, insbesondere gegebenenfalls substituiertes Benzyl, darstellt, ist R⁵ bevorzugt Wasserstoff. Sofern R⁴ und R⁵ gemeinsam mit dem Stickstoffatom, an welches sie gebunden sind, einen Heterocyclus bilden, kann dies ein Pyrrolidin-, Piperidin-, Morpholin- oder gegebenenfalls substituierter Piperazinring sein. So eignen sich beispielsweise unsubstituierte oder durch gegebenenfalls substituiertes Phenyl substituierte Piperazinringe.

Erfindungsgemäß werden die neuen Verbindungen der Formel I und deren Säureadditionssalze erhalten, indem man in an sich bekannter Weise
a) zur Herstellung von Verbindungen der allgemeinen Formel Ia
   (siehe Formel Ia)
   worin R¹ obige Bedeutung besitzt und R³' die für R³ angegebene Bedeutung mit Ausnahme von Hydroxy besitzt, Verbindungen der allgemeinen Formel II
   (siehe Formel II)
   worin R¹ und R³' obige Bedeutung besitzen und R⁷ eine niedere Alkoxycarbonylgruppe oder die CN-Gruppe bedeutet, unter reduzierenden Bedingungen cyclisiert, oder
b) zur Herstellung von Verbindungen der allgemeinen Formel Ib
   (siehe Formel Ib)
   worin R¹, R³', R⁴, R⁵ und n obige Bedeutung besitzen und R²' Wasserstoff oder niederes Alkyl darstellt, Verbindungen der allgemeinen Formel III
   (siehe Formel III)
   worin R¹, R²', R³' und n obige Bedeutung besitzen und X eine nukleophil abspaltbare Fluchtgruppe darstellt, mit Verbindungen der allgemeinen Formel IV
   (siehe Formel IV)
   worin R⁴ und R⁵ obige Bedeutung besitzen, umsetzt, oder
c) zur Herstellung von Verbindungen der allgemeinen Formel Ic
   (siehe Formel Ic)
   worin R¹, R²', R^{3'} und n obige Bedeutung besitzen, in Verbindungen der allgemeinen Formel V
   (siehe Formel V)
   worin R¹, R²', R³' und n obige Bedeutung besitzen und Y für eine Azid- oder Phthalimidgruppe oder, falls n 1 ist, auch für die Cyangruppe steht, die Gruppe Y in die Aminogruppe überführt, oder
d) zur Herstellung von Verbindungen der allgemeinen Formel Id
   (siehe Formel Id)
   worin R¹, R², R³', R⁵ und n obige Bedeutung besitzen und R⁴'' die für R⁴ angegebene Bedeutung mit Ausnahme von Wasserstoff besitzt,
   Verbindungen der allgemeinen Formel VI
   (siehe Formel VI)
   worin R¹, R², R³' und n obige Bedeutung besitzen und R⁸ Wasserstoff, Alkyl mit 1 bis 5 Kohlenstoffatomen, Cycloalkyl mit 3 bis 6 Kohlenstoffatomen, Cycloalkylalkyl mit 4 bis 9 Kohlenstoffatomen, eine gegebenenfalls im Phenylring durch niederes Alkyl, niederes Alkoxy, Hydroxy oder Halogen mono- oder disubstituierte Phenylniederalkylgruppe oder eine Aminoschutzgruppe bedeutet, alkyliert und eine allfällige Aminoschutzgruppe R⁸ wieder abspaltet, oder
e) zur Herstellung von Verbindungen der allgemeinen Formel Ie
   (siehe Formel Ie)
   worin R¹, R³', R⁴, R⁵ und n obige Bedeutung besitzen und R²'' eine gegebenenfalls in α-Stellung zum Stickstoffatom durch niederes Alkoxy substituierte niedere Alkylgruppe bedeutet, in Verbindungen der allgemeinen Formel VII
   (siehe Formel VII)
   worin R^{3'} und n obige Bedeutung besitzen, R¹' die für R¹ angegebene Bedeutung besitzt oder für eine Aminoschutzgruppe steht, R⁴' und R⁵' die für R⁴ und R⁵ angegebenen Bedeutungen besitzen, wobei jedoch eine NR⁴R⁵-Gruppe, worin R⁴ und/oder R⁵ Wasserstoff bedeuten, durch mindestens eine leicht abspaltbare Aminoschutzgruppe derart geschützt ist, daß sie nicht mit Acylierungs- oder Alkylierungsmitteln reagiert, und R⁹ eine niedere 1-Hydroxyalkylgruppe darstellt, die 1-Hydroxyalkylgruppe in den Rest R²'' überführt und allfallige Aminoschutzgruppen wieder abspaltet, oder
f) zur Herstellung von Verbindungen der allgemeinen Formel If
   (siehe Formel If)
   worin R¹, R³', R⁴, R⁵ und n obige Bedeutung besitzen und R²''' niederes Alkyl bedeutet,
   Verbindungen der allgemeinen Formel VIII
   (siehe Formel VIII)
   worin R³' und n obige Bedeutung besitzen und R¹''', R⁴''' und R⁵''' die für R¹, R⁴ und R⁵ angegebenen Bedeutungen mit Ausnahme von Wasserstoff besitzen oder eine Aminoschutzgruppe darstellen, mit Verbindungen der allgemeinen Formel XII
   (siehe Formel XII)
   worin R²''' und X obige Bedeutung besitzen, umsetzt und anschließend allfällige Aminoschutzgruppen wieder abspaltet, oder
g) zur Herstellung von Verbindungen der allgemeinen Formel Ig
   (siehe Formel Ig)
   worin R², R³', R⁴, R⁵ und n obige Bedeutung besitzen und R¹'' die für R¹ angegebene Bedeutung mit Ausnahme von Wasserstoff besitzt, Verbindungen der allgemeinen Formel IX
   (siehe Formel IX)
   worin R², R³', R⁴', R^{5'} und n obige Bedeutung besitzen, mit Verbindungen der allgemeinen Formel X
   (siehe Formel X)
   worin R¹'' und X obige Bedeutung besitzen, umsetzt und anschließend allfällige Aminoschutzgruppen wieder abspaltet, oder
h) zur Herstellung von Verbindungen der allgemeinen Formel Ih
   (siehe Formel Ih)
   worin R¹, R², R³', R⁴'' und n obige Bedeutung besitzen und R⁵'' die für R⁵ angegebene Bedeutung mit Ausnahme von Wasserstoff besitzt, in Verbindungen der allgemeinen Formel Ii
   (siehe Formel Ii)
   worin R¹, R², R³' und n obige Bedeutung besitzen, den Aminomethylenrest der allgemeinen Formel b
   (siehe Formel b)
   worin R⁴'' und R⁵'' obige Bedeutung besitzen, einführt,
und gewünschtenfalls in erhaltenen Verbindungen der Formel I, worin R³' Methoxy bedeutet und/oder R¹, R⁴ und/oder R⁵ eine Methoxyphenylgruppe enthalten, die Methoxygruppe zur Hydroxygruppe spaltet und/oder in erhaltenen Verbindungen der Formel I, worin R¹, R⁴, R⁵ und/oder R⁶ eine gegebenenfalls substituierte Benzylgruppe darstellen, diese Benzylgruppe hydrogenolytisch abspaltet und gewünschtenfalls freie Verbindungen der Formel I in ihre Säureadditionssalze überführt oder die Säureadditionssalze in die freien Verbindungen der Formel I überführt.

Die reduktive Cyclisierung von Verbindungen der Formel II zu Verbindungen der Formel Ia gemäß Verfahrensvariante a) kann auf sich bekannte Weise erfolgen, indem man Verbindungen der Formel II mit einem Reduktionsmittel, welches fähig ist, selektiv aliphatische Nitrogruppen zu Aminogruppen zu reduzieren ohne den Cyan- oder Alkoxycarbonylrest reduktiv anzugreifen, in einem unter den Reaktionsbedingungen inerten Lösungsmittel behandelt. Als Reduktionsmittel eignen sich zum Beispiel Hydrazin in Gegenwart von Raney-Nickel oder, sofern R⁷ nicht Cyano ist, auch Wasserstoff in Gegenwart eines Hydrierungskatalysators, insbesondere Palladium/Kohle. Als Lösungsmittel eignen sich insbesondere niedere Alkohole wie Methanol oder Ethanol, welche im Falle einer hydrierenden Behandlung mit Wasserstoff auch im Gemisch mit Wasser eingesetzt werden können. Die Reduktion mit Hydrazin in Gegenwart von Raney-Nickel-Katalysator kann bei Temperaturen zwischen Raumtemperatur und ca. 80 °C, vorzugsweise bei Siedetemperatur des Lösungsmittels erfolgen. Die Reduktion durch katalytische Hydrierung kann bei einem Wasserstoffdruck von 3 bis 120 bar und Temperaturen zwischen Raumtemperatur und ca. 120 °C durchgeführt werden. Je nach Art der Hydrierbedingungen kann ein gegebenenfalls substituierter Benzylrest R¹ bei einer katalytischen Hydrierung ebenfalls mitabgespalten werden, so daß in diesem Fall zweckmäßigerweise die Reduktion mit Hydrazin gewählt wird.

Die Umsetzung von Verbindungen der Formel III mit Aminoverbindungen der Formel IV gemäß Verfahrensvariante b) kann nach an sich zur Aminoalkylierung üblichen Methoden durchgeführt werden. Die Umsetzung wird zweckmäßigerweise in einem unter den Reaktionsbedingungen inerten organischen Lösungsmittel unter basischen Bedingungen durchgeführt.

Als nukleophil abspaltbare Reste X in den Verbindungen der Formel III eignen sich Halogene wie Chlor, Brom oder Jod oder auch ein Acyloxyrest O-E, worin E einen niederen Alkanoylrest oder einen organischen Sulfonsäurerest darstellt, beispielsweise den Rest von einer Niederalkansulfonsäure wie zum Beispiel Methansulfonsäure oder von aromatischen Sulfonsäuren wie Benzolsulfonsäure oder durch niederes Alkyl oder durch Halogen substituierten Benzolsulfonsäuren, zum Beispiel Toluolsulfonsäuren oder Brombenzolsulfonsäuren. Als inerte organische Lösungsmittel eignen sich insbesondere Dimethylformamid, niedere Alkanole wie Ethanol, cyclische Ether wie Dioxan oder Tetrahydrofuran, halogenierte Kohlenwasserstoffe, aromatische Kohlenwasserstoffe oder Gemische aus den vorgenannten Lösungsmitteln. Zum Abfangen der während der Reaktion gebildeten Säure wird die Umsetzung zweckmäßigerweise unter Zusatz einer organischen oder anorganischen Base durchgeführt. Es kann jedoch auch ein Überschuß des Amins der Formel IV verwendet und dieser als interne Base benutzt werden. Beispiele organischer Basen sind tertiäre organische Amine, insbesondere tertiäre Niederalkylamine wie Triethylamin, Tripropylamine, N-Niederalkylmorpholine oder N-Niederalkylpiperidine. Geeignete anorganische Basen sind insbesondere Alkalimetallcarbonate oder -bicarbonate. Die Reaktionstemperatur kann zwischen Raumtemperatur und 100 °C liegen, vorzugsweise wird bei erhöhter Temperatur, beispielsweise bei Temperaturen zwischen 50 und 80 °C gearbeitet.

Die Gewinnung von Aminverbindungen der Formel Ic gemäß Verfahrensvariante c) aus entsprechenden Aziden, Cyaniden oder Phthalimiden der Formel V kann auf an sich bekannte Weise nach zur Umwandlung von Aziden, Cyaniden oder Phthalimiden zu entsprechenden Aminen üblichen Methoden erfolgen. So können beispielsweise Phthalimide der Formel V auf an sich bekannte Weise hydrolysiert werden und beispielsweise durch Behandeln mit Hydrazin zu den Verbindungen der Formel Ic gespalten werden. Azide und Cyanide der Formel V können auf an sich bekannte Weise in einem unter den Reaktionsbedingungen inerten Lösungsmittel zu den entsprechenden Aminverbindungen reduziert werden. Hierbei müssen die Reduktionsbedingungen so gewählt werden, daß die Lactamfunktion des Ringgerüstes nicht angegriffen wird. Zur Reduktion von Aziden der Formel V eignen sich zum Beispiel Behandeln mit Hydrazin in Gegenwart von Raney-Nickel, Behandeln mit Zinkchlorid in Methanol, Behandeln mit Natriumborhydrid in einem Zweiphasensystem aus einer wäßrigen Phase und einem mit Wasser nicht mischbaren organischen Lösungsmittel, z.B. einem halogenierten Kohlenwasserstoff wie Dichlormethan oder einem aromatischen Kohlenwasserstoff wie Toluol, in Gegenwart eines Phasentransferkatalysators, z.B. eines Tetraalkylammoniumsalzes wie Tetraoctylammoniumacetat, Behandeln mit Triphenylphosphin in wäßrigem Medium oder katalytische Hydrierung. Die katalytische Hydrierung kann auf an sich bekannte Weise in einem unter den Reaktionsbedingungen inerten Lösungsmittel, z.B. einem niederen Alkanol, in Gegenwart eines Hydrierkatalysators bei einem Wasserstoffdruck im Bereich von 1 - 50 bar durchgeführt werden. Als Hydrierkatalysatoren eignen sich Raney-Nickel oder, falls R¹ keine gegebenenfalls substituierte Benzylgruppe darstellt, auch Palladium auf Kohle. Die Reduktion eines Cyanids der Formel V wird vorzugsweise durch katalytische Hydrierung in Gegenwart von Raney-Nickel in einem Gemisch aus einem niederen Alkohol und Ammoniak bei einem Wasserstoffdruck zwischen 50 und 150 bar durchgeführt.

Die Herstellung von Verbindungen der Formel Id durch Alkylierung von Verbindungen der Formel VI gemäß Verfahrensvariante d) kann nach an sich zur Alkylierung von Aminen üblichen Methoden erfolgen. So können Verbindungen der Formel Id erhalten werden durch Umsetzen von Verbindungen der Formel VI mit Verbindungen der allgemeinen Formel XIIIa oder, falls R⁸ in den Verbindungen der Formel VI Wasserstoff bedeutet, auch mit Verbindungen der allgemeinen Formel XIIIb
(siehe Formeln XIIIa und XIIIb)
worin B und X obige Bedeutung besitzen und R^{4IV} Alkyl mit 1 bis 5 Kohlenstoffatomen, Cycloalkyl mit 3 bis 6 Kohlenstoffatomen, Cycloalkylalkyl mit 4 bis 9 Kohlenstoffatomen oder eine gegebenenfalls im Phenylring durch niederes Alkyl, niederes Alkoxy, Hydroxy oder Halogen mono- oder disubstituierte Phenylniederalkylgruppe bedeutet, unter zur Aminoalkylierung üblichen Bedingungen oder durch reduktive Alkylierung der Verbindungen der Formel VI mit Aldehyden oder Ketonen der Formel XIIIc
(siehe Formel XIIIc)
worin R^{4V} einen dem Rest R^{4IV} entsprechenden jedoch ein Wasserstoffatom weniger enthaltenden Rest mit mindestens 2 Kohlenstoffatomen darstellt.

Die Umsetzung von Verbindungen der Formel VI mit Verbindungen der Formel XIIIa oder XIIIb kann nach an sich zur Alkylierung von Aminen üblichen Methoden in einem unter den Reaktionsbedingungen inerten Lösungsmittel unter basischen Bedingungen ausgeführt werden. Sie kann beispielsweise in der für die Umsetzung der Verbindungen der Formel III mit Verbindungen der Formel IV beschriebenen Weise erfolgen. Sofern R⁸ eine Aminoschutzgruppe darstellt, kann dies eine an sich bekannte hydrogenolytisch oder hydrolytisch abspaltbare Schutzgruppe sein. Als hydrogenolytisch abspaltbare Schutzgruppen eignen sich insbesondere gegebenenfalls substituierte Benzyl- oder Benzylhydrylgruppen, welche anschließend leicht, z.B. in Gegenwart eines Palladium/Kohle-Katalysators, wieder abhydriert werden können. Als Beispiele von hydrolytischen abspaltbaren Aminoschutzgruppen eignen sich niedere Acylgruppen wie Formyl, Acetyl oder Trifluoracetyl. Sofern R⁸ in den Verbindungen der Formel VI Wasserstoff darstellt, wird im allgemeinen bei der Umsetzung mit Verbindungen der Formel XIIIa ein Gemisch von mono- und disubstituierten Verbindungen erhalten, worin der Anteil an disubstituierten Verbindungen je nach der eingesetzten Menge an Verbindung der Formel XIIIa und den Reaktionsbedingungen variieren kann. Die monosubstituierten und disubstituierten Verbindungen können auf an sich bekannte Weise, beispielsweise durch Chromatographie an Kieselgel, voneinander getrennt werden.

Die reduktive Alkylierung von Verbindungen der Formel VI kann auf an sich bekannte Weise durch Umsetzen der Verbindungen der Formel VI mit einem Aldehyd oder Keton der Formel XIIIc unter reduzierenden Bedingungen erfolgen. Zum Beispiel können die Verbindungen der Formel VI mit Verbindungen der Formel XIIIc in einem unter den Reaktionsbedingungen inerten Lösungsmittel in Gegenwart eines Reduktionsmittels, beispielsweise Ameisensäure, einem Boran-Diniederalkylamin-Komplex oder Natriumcyanoborhydrid, umgesetzt werden. Die Verbindungen der Formel VI können jedoch auch mit den Verbindungen der Formel XIIIa zunächst in einem unter den Reaktionsbedingungen inerten Lösungsmittel umgesetzt werden und die intermediär entstandenen Schiff'schen Basen anschließend in situ oder nach Isolierung reduziert werden durch Behandeln mit einem die Lactamfunktion des Ringgerüstes nicht angreifenden Reduktionsmittel. Die Reduktion der intermediären Iminverbindungen kann beispielsweise auf an sich bekannte Weise durch Behandeln mit einem Boran-Diniederalkylamin-Komplex, z.B. Boran-Dimethylamin, oder einem Diboran/Pyridin-Komplex, mit Natriumborhydrid in Eisessig oder mit Natriumcyanoborhydrid in saurem oder neutralem Medium, zum Beispiel in Essigsäure oder einem niederen Alkohol, erfolgen. Gewünschtenfalls kann die Reduktion der Schiff'schen Basen auch durch katalytische Hydrierung erfolgen. Die katalytische Hydrierung kann beispielsweise in Gegenwart von Raney-Nickel oder Palladium/Kohle in einem niederen Alkohol bei milden Bedingungen, z.B. bei einem Wasserstoffdruck von 1 - 3 bar bei Raumtemperatur, durchgeführt werden.

Die Überführung des 1-Hydroxyalkyl-Restes R⁹ der Verbindungen der Formel VII in einen Rest R²'' gemäß Verfahrensvariante e) zur Herstellung von Verbindungen der Formel Ie kann nach an sich bekannten Methoden erfolgen. So kann eine Hydroxyalkylgruppe R⁹ auf an sich bekannte Weise zu der entsprechenden Alkylgruppe reduziert werden oder die Hydroxyalkylgruppe R⁹ kann auf an sich bekannte Weise unter sauren Bedingungen mit einem niederen Alkohol verethert werden.

Die Reduktion der Hydroxyalkylgruppe zur Alkylgruppe kann in einem unter den Reaktionsbedingungen inerten organischen Lösungsmittel mit Hilfe eines Hydridreduktionsmittels erfolgen, welches befähigt ist, die Hydroxyalkylgruppe zu reduzieren ohne die Lactamfunktion anzugreifen. Als Reduktionsmittel eignet sich insbesondere Natriumborhydrid in Gegenwart von starken organischen Säuren, z.B. Halogenessigsäuren wie Trifluoressigsäure, oder auch Triethylsilan. Als Lösungsmittel eignen sich beispielsweise offenkettige oder cyclische Ether, insbesondere cyclische Ether wie Tetrahydrofuran oder Dioxan, oder halogenierte Kohlenwasserstoffe wie Dichlormethan. Die Reaktion kann bei leicht erhöhter Temperatur, beispielsweise bei Temperaturen zwischen ca. 30 und 100 °C, vorzugsweise bei Rückflußtemperatur des Reaktionsgemisches durchgeführt werden.

Die Veretherung der Hydroxyalkylgruppe R⁹ mit einem niederen Alkohol kann unter an sich zur durch Säuren katalysierten Etherbildung üblichen Bedingungen durchgeführt werden. So kann die Verbindung der Formel VII beispielsweise in einem niederen Alkohol unter Zusatz von katalytisch wirksamen Mengen einer starken Säure umgesetzt werden. Die Umsetzung erfolgt zweckmäßigerweise bei erhöhter Temperatur, beispielsweise durch Erhitzen der Verbindung der Formel VII in dem niederen Alkohol bei Rückflußtemperatur des Reaktionsgemisches. Als Säuren eignen sich anorganische Säuren wie Schwefelsäure oder starke organische Säuren, zweckmäßigerweise organische Sulfonsäuren, beispielsweise Niederalkansulfonsäuren wie zum Beispiel Methansulfonsäure oder aromatische Sulfonsäuren wie Benzolsulfonsäure oder durch niederes Alkyl oder durch Halogen substituierte Benzolsulfonsäuren oder auch Halogenessigsäuren.

Nach erfolgter Umwandlung des Restes R⁹ in einen Rest R²'' werden allfällige Aminoschutzgruppen auf an sich bekannte Weise abgespalten.

Die Umsetzung von Verbindungen der Formel VIII mit Verbindungen der Formel XII gemäß Verfahrensvariante f) kann auf an sich bekannte Weise unter zur Alkylierung von Amiden üblichen Bedingungen erfolgen. So kann die Umsetzung in einem unter den Reaktionsbedingungen inerten Lösungsmittel in Gegenwart einer starken Base, welche befähigt ist, das Stickstoffatom der Lactam-Gruppe zu deprotonieren, durchgeführt werden. Als Basen eignen sich beispielsweise lithiumorganische Basen, z.B. ein Niederalkyllithium, insbesondere Butyllithium, oder Lithiumdiisopropylamid, oder Kalium-tert.Butylat. Als Lösungsmittel eignen sich beispielsweise offenkettige oder cyclische Ether wie Diethylether, Tetrahydrofuran oder Dioxan. Die Umsetzung wird zweckmäßigerweise bei Temperaturen im Bereich von - 78 °C bis Raumtemperatur durchgeführt. Nach beendeter Reaktion werden allfällige Aminoschutzgruppen auf an sich bekannte Weise wieder abgespalten.

Die Umsetzung von Verbindungen der Formel IX mit Verbindungen der Formel X gemäß Verfahrensvariante g) kann nach an sich zur Alkylierung von Indolen üblichen Methoden erfolgen. In den Verbindungen der Formel X kann der nukleophil abspaltbare Rest X die vorstehend für die Verbindungen der Formel III angegebenen Bedeutungen besitzen. Insbesondere eignen sich Halogene, vorzugsweise Jod oder Brom, oder auch organische Sulfonsäurereste. Die Umsetzung wird zweckmäßigerweise in einem unter den Reaktionsbedingungen inerten organischen Lösungsmittel in Gegenwart einer starken Base durchgeführt. Als starke Basen eignen sich beispielsweise Alkalimetallhydride wie Natriumhydrid oder lithiumorganische Basen, z.B. ein Niederalkyllithium, insbesondere Butyllithium, oder Lithiumdiisopropylamid, oder Kalium-tert.Butylat. Sofern R² Wasserstoff bedeutet, reagiert primär der stärker nukleophile Indolstickstoff. Um eine zusätzliche Alkylierung am Lactamstickstoff zu vermeiden, wird die Menge an Base zweckmäßig so begrenzt, daß sie für eine Zweitalkylierung nicht ausreicht. Gewünschtenfalls kann eine freie Amidgruppe auch auf an sich bekannte Weise durch Einführen einer Formylschutzgruppe, welche bei der anschließenden Aufarbeitung wieder hydrolytisch abgespalten wird, geschützt werden. Als Lösungsmittel eignen sich Dimethylformamid oder offenkettige oder cycliche Ether wie Diethylether, Tetrahydrofuran oder Dioxan. Die Umsetzung wird zweckmäßigerweise bei erhöhter Temperatur beispielsweise bei Temperaturen zwischen 30 und 100 °C durchgeführt. Allfällige Aminoschutzgruppen können nach der Umsetzung auf an sich bekannte Weise abgespalten werden.

Die Einführung des Aminomethylenrestes der Formel b in die Aminoverbindungen der Formel Ii gemäß Verfahrensvariante h) kann nach an sich zur Amidinbildung üblichen Methoden erfolgen. So können die Verbindungen der Formel Ii auf an sich bekannte Weise mit Halogeniminiumsalzen der allgemeinen Formel XIa
(s. Formel XIa)
worin R^{4''} und R^{5''} obige Bedeutung besitzen, Hal Chlor oder Brom bedeutet und A⁻ für ein Säureanion steht, oder mit Acetalen der allgemeinen Formel XIb
(s. Formel XIb)
worin R^{4''} und R^{5''} obige Bedeutung besitzen und R¹³ niederes Alkyl bedeutet, umgesetzt werden.

Die Umsetzung von Verbindungen der Formel Ii mit Halogeniminiumsalzen der Formel XIa zu den Verbindungen der Formel Ih kann auf an sich bekannte Weise unter zur Bildung von Amidinen üblichen Bedingungen erfolgen. In den Salzen der Formel XIa kann A⁻ das Anion einer Halogenwasserstoff-Säure, insbesondere Chlorid, darstellen. Die Umsetzung erfolgt zweckmäßigerweise in einem unter den Reaktionsbedingungen inerten organischen Lösungsmittel, beispielsweise Dimethylformamid, einem offenkettigen oder cyclischen Ether, einem halogenierten Kohlenwasserstoff oder einem Gemisch dieser Lösungsmittel, bei Temperaturen von Raumtemperatur bis 100 °C.

Die Umsetzung von Verbindungen der Formel Ii mit Acetalen der Formel XIb kann auf an sich bekannte Weise unter zur Bildung von Amidinen üblichen Bedingungen, beispielsweise den vorstehend für Umsetzung der Verbindungen der Formel Ii mit den Verbindungen der Formel XIa angegebenen Bedingungen, erfolgen.

Als Aminoschutzgruppen in den vorstehend angegebenen Verbindungen können an sich bekannte Schutzgruppen gewählt werden, welche anschließend auf an sich bekannte Weise solvolytisch oder hydrogenolytisch wieder abgespalten werden können. Geeignete leicht wieder abspaltbare Schutzgruppen für Aminogruppen sind zum Beispiel bekannt aus E. Mc Omie "Protective Groups in Organic Chemistry" Plenum Press 1971. Als Aminoschutzgruppen eignen sich beispielsweise hydrolytisch abspaltbare Acylgruppen, vorzugsweise die Trifluoracetylgruppe, oder auch gegebenenfalls substituierte Benzylgruppen, welche auf an sich bekannte Weise hydrogenolytisch wieder abgespalten werden können. Die Aminoschutzgruppen müssen selbstverständlich unter Berücksichtigung der übrigen in den zu schützenden Verbindungen enthaltenen Reste jeweils so ausgewählt werden, daß die Aminogruppe unter den zur Herstellung und/oder Weiterverarbeitung der Verbindungen herrschenden Reaktionsbedingungen ausreichend geschützt ist, und daß die Schutzgruppen anschließend leicht unter Bedingungen, unter denen andere im Molekül enthaltene Reste nicht angegriffen werden, abspaltbar sind. Als hydrogenolytisch abspaltbare Schutzgruppe wird vorzugsweise die Benzylgruppe verwendet. Als hydrolytisch abspaltbare Gruppe wird vorzugsweise die Trifluoracetylgruppe eingesetzt. Sofern die NR⁴R⁵-Gruppe eine NH₂-Gruppe darstellt, ist es bei Verwendung der Trifluoracetylschutzgruppe ausreichend, wenn eines der Wasserstoffatome durch diese Schutzgruppe ersetzt wird. Zur Einführung einer Trifluoracetylschutzgruppe kann die zu schützende Verbindung mit Trifluoracetanhydrid in an sich bekannter Weise umgesetzt werden. Sofern R² Wasserstoff bedeutet, kann bei dieser Acylierung auch teilweise eine Acylierung an dem Amid-Stickstoff stattfinden, wobei eine Lactamimidfunktion gebildet wird. Eine derartige Lactamimidfunktion wird jedoch bei einer anschließenden Behandlung des Acylierungsproduktes mit wäßriger gesättigter Natriumhydrogencarbonatlösung zur Neutralisation von gebildeter Säure und von überschüssigem Säureanhydrid wieder gespalten. Sofern die Substituenten R¹, R⁴ und/oder R⁵ phenolische Hydroxygruppen enthalten, können diese gewünschtenfalls bei den vorstehenden Umsetzungen durch anschließend wieder abspaltbare an sich bekannte Etherschutzgruppen, beispielsweise Benzyl, geschützt werden.

In Verbindungen der Formel I, worin R³ Methoxy bedeutet und/oder R¹ und/oder R⁴ und/oder R⁵ eine Methoxyphenylgruppe enthalten, kann die Methoxygruppe mit zur Spaltung von Methoxyarylethern geeigneten Methoden auf an sich bekannnte Weise zur Hydroxygruppe gespalten werden. Beispielsweise kann die Etherspaltung durch Behandeln mit Jodwasserstoff oder Bromwasserstoff in einem unter den Reaktionsbedingungen inerten Lösungsmittel, zum Beispiel Acetanhydrid oder Essigsäure, oder durch Behandeln mit Jodtrimethylsilan in Gegenwart einer Base oder mit Bortribromid in einem halogeniertem Kohlenwasserstoff wie Dichlormethan erfolgen.

In Verbindungen der Formel I, worin R¹, und/oder R⁴ und/oder R⁵ und/oder R⁶ eine gegebenenfalls im Phenylring substituierte Benzylgruppe darstellen, kann diese Gruppe gewünschtenfalls auf an sich bekannte Weise hydrogenolytisch abgespalten werden. Die Hydrogenolyse kann in einem unter den Reaktionsbedingungen inerten Lösungsmittel, beispielsweise einem niederen Alkohol, durch katalytische Hydrierung bei einem Wasserstoffdruck von 3 bis 50 bar und Temperaturen zwischen Raumtemperatur und ca. 120 °C in Gegenwart eines Hydrierungskatalysators, beispielsweise Palladium/Kohle, erfolgen. Die Benzylabspaltung kann auch durch Behandeln mit Ameisensäure in Gegenwart von Palladium/Kohle in einem niederen Alkohol oder durch Behandeln mit Natrium in flüssigem Ammoniak erfolgen.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit anorganischen Säuren, zum Beispiel Halogenwasserstoffsäuren, insbesondre Chlorwasserstoffsäure, Schwefelsäure oder Phosphorsäuren, oder mit organischen Säuren, beispielsweise niederen aliphatischen Mono- oder Dicarbonsäuren wie Maleinsäure, Fumarsäure, Milchsäure, Weinsäure oder Essigsäure oder Sulfonsäuren, beispielsweise Niederalkansulfonsäuren wie Methansulfonsäure oder gegebenenfalls im Benzolring durch Halogen oder niederes Alkyl substituierte Benzolsulfonsäuren wie p-Toluolsulfonsäure, oder Cyclohexylaminsulfonsäure.

Die Verbindungen der Formel I enthalten ein Chiralitätszentrum in der Position, in welcher die Seitenkette -(CH₂)ₙ-D-NR⁴R⁵ an den Azepinring gebunden ist, und können in mehreren optisch aktiven enantiomeren Formen oder als Racemat vorliegen. Die vorliegende Erfindung umfaßt sowohl die racemischen Gemische wie auch die reinen optischen Isomeren der Verbindungen der Formel I.

Falls bei der Synthese Racemate der Ausgangsverbindungen der Formeln III, V, VI, VII, VIII oder IX eingesetzt werden, werden die Verbindungen der Formel I in Form von Racematen erhalten. Ausgehend von optisch aktiven Formen dieser Ausgangsverbindungen können optisch aktive Verbindungen der Formel I erhalten werden. Die optisch aktiven Verbindungen der Formel I können aus den racemischen Gemischen in an sich bekannter Weise erhalten werden, zum Beispiel durch chromatographische Trennung an chiralen Trennmaterialien oder durch Umsetzung mit geeigneten optisch aktiven Säuren, beispielsweise Weinsäure oder Äpfelsäure, und anschließende Auftrennung in ihre optisch aktiven Antipoden durch fraktionierte Kristallisation der gewonnenen Salze.

Die Ausgangsverbindungen der Formel II stellen wertvollen Zwischenprodukte zur Herstelllung von pharmakologisch wirksamen Verbindungen, beispielsweise den Verbindungen der Formel I, dar. Die Verbindungen der Formel II können auf an sich bekannte Weise ausgehend von Indolverbindungen der allgemeinen Formel XIV
(siehe Formel XIV)
worin R¹, R³' und R⁷ obige Bedeutung besitzen, erhalten werden, indem man die Verbindungen der Formel XIV zunächst zu Aldehyden der allgemeinen Formel XV
(siehe Formel XV)
worin R¹, R³' und R⁷ obige Bedeutung besitzen, formyliert und die erhaltenen Aldehydverbindungen der Formel XV mit Nitromethan umsetzt.

Die Formylierung der Indolverbindungen der Formel XIV kann durch Umsetzen mit bekannten Formylierungsmitteln nach zur Formylierung von Aromaten üblichen Methoden erfolgen. Als zweckmäßig erweist sich die Formylierung mit einem N,N-disubstituierten Formamid wie Dimethylformamid oder N-Methylformanilid in Gegenwart von Phosphoroxychlorid oder Phosgen nach der Vilsmeier-Methode. Als Lösungsmittel kann hierbei ein Überschuß an Dimethylformamid dienen oder es können auch aromatische Kohlenwasserstoffe wie Benzol oder Chlorbenzol eingesetzt werden. Die Umsetzung kann bei Temperaturen zwischen Raumtemperatur und ca. 80 °C durchgeführt werden.

Erhaltene Aldehydverbindungen der Formel XV, worin R¹ Wasserstoff bedeutet, können gewünschtenfalls durch Umsetzung mit Verbindungen der Formel X in solche Verbindungen der Formel XV überführt werden, worin R¹ für einen R¹''-Rest steht. Die Umsetzung kann nach an sich zur Alkylierung von Indolen üblichen Methoden erfolgen und kann beispielsweise unter den vorstehend für die Umsetzung von Verbindungen der Formel IX mit Verbindungen der Formel X angegebenen Bedingungen durchgeführt werden.

Die Umsetzung von Verbindungen der Formel XV mit Nitromethan kann in Gegenwart einer Base unter zur Umsetzung von Aldehyden mit C-H-aciden Verbindungen üblichen Bedingungen durchgeführt werden. Als Lösungsmittel kann ein Überschuß Nitromethan dienen. Gewünschtenfalls können auch weitere organische Lösungsmittel wie zum Beispiel niedere Alkohole, halogenierte Kohlenwasserstoffe wie Dichlormethan oder cyclische Ether wie Tetrahydrofuran zugesetzt werden. Die Umsetzung kann bei Temperaturen zwischen Raumtemperatur und ca. 80 °C erfolgen. Als Basen können anorganische oder organische basisch reagierende Verbindungen eingesetzt werden. So eignen sich beispielsweise Alkalimetallhydroxyde, -carbonate oder -acetate, Ammoniumacetat oder -carbonat, basische Ionenaustauscher oder tertiäre organische Basen wie beispielsweise Tri-niederalkylamine oder insbesondere 1,8-Diazabicyclo[5.4.0]undec-7-en (1,5-5)(=DBU) oder 1,5-Diazabicyclo[4.3.0]non-5-en (= DBN). Bei der Umsetzung kann ein Gemisch aus den 3-(1,3-Dinitropropan-2-yl)-indol-Verbindungen der Formel II und den entsprechenden 3-(2-Nitroethylen)-indol-Verbindungen der allgemeinen Formel XVI
(siehe Formel XVI)
worin R¹, R³' und R⁷ obige Bedeutung besitzen, gebildet werden. Ein Gemisch aus Verbindungen der Formel II und Verbindungen der Formel XVI kann auf an sich bekannte Weise beispielsweise destillativ oder durch Säulenchromatographie, aufgetrennt werden. Das Verhältnis von Verbindungen der Formel XVI zu Verbindungen der Formel II in dem Reaktionsgemisch kann je nach Art und Stärke der eingesetzten Base und den Reaktionsbedingungen variiert werden. Um direkt Verbindungen der Formel II oder Gemische mit einem sehr hohen Anteil an Verbindungen der Formel II zu erhalten, werden als Basen zweckmäßigerweise 1,8-Diazabicyclo[5.4.0]undec-7-en(1,5-5) oder 1,5-Diazabicyclo[4.3.0]non-5-en eingesetzt. Um Verbindungen der Formel XVI oder Gemische mit einem sehr hohen Anteil an Verbindungen der Formel XVI zu erhalten, werden zweckmäßigerweise schwache Basen, insbesondere Ammoniumacetat, eingesetzt.

Verbindungen der Formel XVI, worin R¹ Wasserstoff bedeutet, können gewünschtenfalls durch Umsetzen mit Verbindungen der Formel X in solche Verbindungen der Formel XVI, worin R¹ den Rest R¹'' bedeutet, überführt werden. Die Umsetzung kann nach an sich zur Alkylierung von Indolen üblichen Methoden erfolgen und beispielsweise unter den für die Umsetzung von Verbindungen der Formel IX mit Verbindungen der Formel X vorstehend beschriebenen Bedingungen durchgeführt werden.

Verbindungen der Formel XVI können durch Umsetzung mit weiterem Nitromethan in Gegenwart einer der vorstehend für die Herstellung von Verbindungen der Formel II als besonders geeignet angegebenen Basen in Verbindungen der Formel II überführt werden.

Verbindungen der Formel XVI können auch erhalten werden, indem man Verbindungen der Formel XIV mit 1-Nitro-2-dimethylaminoethylen umsetzt.

Die Indol-Verbindungen der Formel XIV sind bekannt oder können nach an sich bekannten Methoden oder analog zu an sich bekannten Methoden hergestellt werden. Die Herstellung von Verbindungen der Formel XIV wird beispielsweise in dem US-Patent Nr. 3 732 245 beschrieben.

Die Ausgangsverbindungen der Formel III sind neue Verbindungen, welche wertvolle Zwischenprodukte zur Herstellung von pharmakologisch wirksamen Verbindungen, beispielsweise den Verbindungen der Formel I, darstellen. Verbindungen der Formel III können auf an sich bekannte Weise aus entsprechenden Alkoholen der allgemeinen Formel XVII
(siehe Formel XVII)
worin R¹, R³', R²' und n obige Bedeutung besitzen, erhalten werden, indem die Hydroxygruppe in an sich bekannter Weise in eine Fluchtgruppe X überführt wird. So können die Verbindungen der Formel XVII beispielsweise zur Einführung eines Halogenrestes X mit Thionylchlorid oder mit Phosphorhalogeniden, zum Beispiel Phosphortribromid, auf an sich bekannte Weise in einem unter den Reaktionsbedingungen inerten Lösungsmittel, zum Beispiel einem halogenierten Kohlenwasserstoff wie Chloroform, umgesetzt werden. Sulfonsäurereste X können auf an sich bekannte Weise eingeführt werden, indem man Verbindungen der Formel XVII mit einem entsprechenden Sulfonsäurehalogenid acyliert. So können die Alkohole der Formel XVII mit einem Sulfonsäurehalogenid, vorzugsweise einem Sulfonsäurechlorid, unter an sich zur Esterbildung üblichen Methoden umgesetzt werden. Beispielsweise kann die Umsetzung in Gegenwart einer Base bei Temperaturen zwischen ca. Raumtemperatur und 100 °C in einem unter den Reaktionsbedingungen inerten Lösungsmittel durchgeführt werden. Als Basen eignen sich beispielsweise tertiäre organische Amine wie Triethylamin oder Pyridin, welches gleichzeitig als Lösungsmittel für die Umsetzung dienen kann.

Verbindungen der Formel XVII sind neue Verbindungen, welche wertvolle Zwischenprodukte zur Herstellung von pharmakologisch wirksamen Verbindungen, beispielsweise Verbindungen der Formel I, darstellen. Die Alkohole der allgemeinen Formel XVIIa
(siehe Formel XVIIa)
worin R¹, R^{2'} und R^{3'} obige Bedeutung besitzen,
können auf an sich bekannte Weise erhalten werden, indem man in entsprechenden Estern der allgemeinen Formel XVIII
(siehe Formel XVIII)
worin R¹, R²' und R³' obige Bedeutung besitzen und R¹⁰ niederes Alkyl bedeutet, die Estergruppe selektiv reduziert. Als Reduktionsmittel eignen sich beispielsweise zur Reduktion von Estern befähigte Hydridreduktionsmittel, welche jedoch die Lactamfunktion des Ringgerüstes nicht angreifen. So erweist sich beispielsweise die Reduktion mit Diisobutylaluminiumborhydrid oder mit Alkalimetallborhydriden wie Natriumborhydrid, Lithiumborhydrid oder Lithiumtriniederalkylborhydrid oder mit Natriumtriethoxyaluminiumhydrid in einem Lösungsmittelgemisch aus einem cyclischen Ether wie Tetrahydrofuran oder Dioxan und einem niederen Alkohol bei Temperaturen zwischen Raumtemperatur und Rückflußtemperatur des Reaktionsgemisches als zweckmäßig. Bei Verwendung von Diisobutylaluminiumhydrid als Reduktionsmittel kann die Reduktion in einem cyclischen Ether wie Tetrahydrofuran oder aromatischen Kohlenwasserstoffen wie Benzol oder Toluol bei Temperaturen zwischen -20 °C und Raumtemperatur erfolgen.
Alkohole der allgemeinen Formel XVIIb
(siehe Formel XVIIb)
worin R¹, R^{2'} und R^{3'} obige Bedeutung besitzen, können ausgehend von solchen Verbindungen der Formel V, worin Y die Cyangruppe bedeutet, erhalten werden, indem man die Cyangruppe auf an sich bekannte Weise in eine Alkoxycarbonylgruppe überführt und diese anschließend selektiv zur Hydroxymethylgruppe reduziert. Als Reduktionsmittel eignen sich die vorstehend zur selektiven Reduzierung der Estergruppe in den Verbindungen der Formel XVIII angegebenen Hydridreduktionsmittel.

Verbindungen der allgemeinen Formel XVIIIa
(siehe Formel XVIIIa)
worin R¹, R³' und R¹⁰ obige Bedeutung besitzen, können auf an sich bekannte Weise ausgehend von Verbindungen der allgemeinen Formel XIX
(siehe Formel XIX)
worin R¹, R³' und R¹⁰ obige Bedeutung besitzen und R^{7'} eine niedere Alkoxycarbonylgruppe bedeutet, hergestellt werden, indem man die Verbindungen der Formel XIX unter cyclisierenden Bedingungen reduziert. Hierzu werden die Verbindungen der Formel XIX in einem unter den Reaktionsbedingungen inerten Lösungsmittel mit einem Reduktionsmittel, welches fähig ist, selektiv aliphatische Nitrogruppen zu Aminogruppen zu reduzieren ohne die Alkoxycarbonylreste reduktiv anzugreifen, behandelt. Als Reduktionsmittel eignen sich zum Beispiel Wasserstoff in Gegenwart eines Hydrierungskatalysators oder Hydrazin in Gegenwart von Raney-Nickel. Die Reduktion durch katalytische Hydrierung kann zweckmäßigerweise bei einem Wasserstoffdruck von 3 bis 50 bar und Temperaturen zwischen Raumtemperatur und ca. 120 °C durchgeführt werden. Als Hydrierungskatalysator eignet sich insbesondere Palladium auf Kohle. Als Lösungsmittel eignen sich insbesondere aromatische Kohlenwasserstoffe wie Toluol oder Xylol. Je nach Art der Hydrierbedingungen kann ein gegebenenfalls substituierter Benzylring R¹ bei einer katalytischen Hydrierung ebenfalls mitabgespalten werden, so daß in diesem Fall zweckmäßigerweise die Reduktion mit Hydrazin gewählt wird. Bei der Reduktion der Verbindungen der Formel XIX unter den angegebenen Bedingungen entsteht im allgemeinen ein Gemisch aus Verbindungen der allgemeinen Formel XX
(siehe Formel XX)
worin R¹, R³', R⁷' und R¹⁰ obige Bedeutung besitzen, und den entsprechenden cyclisierten Verbindungen der Formel XVIIIa. Zur Vervollständigung der Cyclisierung wird dieses Gemisch zweckmäßigerweise während einer Zeitdauer von 0,5 bis 3 Stunden auf Temperaturen zwischen 100 und 150 °C erhitzt.

Verbindungen der Formel XVIIIa, worin R¹ Wasserstoff bedeutet, können gewünschtenfalls durch Umsetzen mit Verbindungen der Formel X in solche Verbindungen der Formel XVIIIa, worin R¹ den Rest R¹'' bedeutet, überführt werden. Die Umsetzung kann nach an sich zur Alkylierung von Indolen üblichen Methoden erfolgen und beispielsweise unter den für die Umsetzung von Verbindungen der Formel IX mit Verbindungen der Formel X vorstehend beschriebenen Bedingungen durchgeführt werden.

Gewünschtenfalls kann man in die Verbindungen der Formel XVIIIa eine Alkylgruppe R2''^{'} auf an sich bekannte Weise einführen. So können die Verbindungen der Formel XVIIIa mit Verbindungen der Formel XII unter zur Alkylierung von Amiden üblichen Bedingungen umgesetzt werden. Die Umsetzung kann in einem unter den Reaktionsbedingungen inerten Lösungsmittel in Gegenwart einer starken Base, welche befähigt ist, das Stickstoffatom der Lactam-Gruppe zu deprotonieren, durchgeführt werden. Als Basen eignen sich beispielsweise die vorstehend für die Alkylierung von Verbindungen der Formel VIII gemäß Verfahren f) genannten Basen, insbesondere Butyllithium. Als Lösungsmittel eignen sich beispielsweise cyclische oder offenkettige Ether wie vorzugsweise Tetrahydrofuran oder Dioxan oder auch Diethylether. Die Umsetzung wird zweckmäßigerweise bei Temperaturen im Bereich von - 80 °C bis Raumtemperatur durchgeführt. Sofern R¹ Wasserstoff bedeutet, muß die Indolfunktion auf an sich bekannte Weise durch eine übliche Aminoschutzgruppe geschützt werden, welche anschließend wieder abgespalten wird.

Verbindungen der Formel XIX können auf an sich bekannte Weise ausgehend von Verbindungen der allgemeinen Formel XXI
(siehe Formel XXI)
worin R¹, R³' und R⁷' obige Bedeutung besitzen und R¹¹ und R¹² je niederes Alkyl bedeuten oder gemeinsam eine Alkylenkette mit bis zu 4 Kohlenstoffatomen darstellen, erhalten werden, indem man die NR¹¹R¹²-Gruppe der Verbindungen der Formel XXI in eine nukleophil abspaltbare Fluchtgruppe überführt und das Reaktionsprodukt in situ mit einem Nitroessigsäureniederalkylester umsetzt. So kann beispielsweise die Verbindung der Formel XXI mit dem Nitroessigsäurealkylester in einem unter den Reaktionsbedingungen inerten Lösungsmittel in Gegenwart von einem Triniederalkylphosphin, insbesondere Tributylphosphin, welches intermediär mit der Aminogruppe der Verbindung der Formel XXI zu einem nucleophil abspaltbaren Rest reagiert, umgesetzt werden. Als Lösungsmittel eignen sich beispielsweise Acetonitril, Dimethylformamid oder cyclische Ether. Die Umsetzung erfolgt zweckmäßigerweise bei erhöhter Temperatur, beispielsweise bei Temperaturen im Bereich von 50 - 80 °C. Gewünschtenfalls kann die Verbindung der Formel XXI auch zunächst durch Umsetzen mit einem Niederalkyljodid auf an sich bekannte Weise in das entsprechende quartäre Ammoniumsalz überführt werden und dieses anschließend mit dem Nitroessigsäureniederalkylester umgesetzt werden.

Verbindungen der Formel XXI können aus Indolverbindungen der allgemeinen Formel XIVa
(siehe Formel XIVa)
worin R¹, R³' und R⁷' obige Bedeutung besitzen, auf an sich bekannte Weise erhalten werden, indem man die Verbindungen der Formel XIVa mit Formaldehyd und einem Amin HNR¹¹R¹², worin R¹¹ und R¹² obige Bedeutung besitzen, nach zur Aminomethylierung üblichen Methoden, beispielsweise unter den Bedingungen einer Mannich-Reaktion, umsetzt.

Verbindungen der Formel XIX können auch aus Verbindungen der allgemeinen Formel XXII
(siehe Formel XXII)
worin R¹, R³', R⁷' und R¹⁰ obige Bedeutung besitzen, erhalten werden, indem man die Doppelbindung in den Verbindungen der Formel XXII auf an sich bekannte Weise reduziert, z.B. indem man die Verbindung der Formel XXII mit in situ aus Natriumborhydrid und Methanol in Tetrahydrofuran hergestelltem Natriumtrimethoxyborhydrid behandelt. Verbindungen der Formel XXII können auf an sich bekannte Weise erhalten werden, indem man Verbindungen der allgemeinen Formel XVa
(siehe Formel XVa)
worin R¹, R³' und R⁷' obige Bedeutung besitzen mit einem Nitroessigsäureniederalkylester umsetzt. Die Umsetzung kann nach an sich bekannten Methoden in Gegenwart einer Base unter zur Umsetzung von Aldehyden mit C-H-aciden Verbindungen üblichen Bedingungen, beispielsweise den vorstehend für die Umsetzung von Verbindungen der Formel XV mit Nitromethan angegebenen Bedingungen, durchgeführt werden.

Verbindungen der Formel V sind neu und stellen wertvolle Zwischenprodukte zur Herstellung von pharmakologisch aktiven Verbindungen, beispielsweise Verbindungen der Formel I, dar.

Verbindungen der Formel V können erhalten werden, indem man entsprechende Verbindungen der Formel III auf an sich bekannte Weise mit einem Alkalimetallazid, einem Alkalimetallphthalimid oder einem Alkalimetallcyanid umsetzt.

Verbindungen der Formel VI umfassen solche Verbindungen der Formel I, worin R⁴ Wasserstoff bedeutet, D eine bindung darstellt und R³ nicht für Hydroxy steht oder können aus entsprechenden Verbindungen der Formel I, worin R⁵ Wasserstoff bedeutet, durch Einführung einer Aminoschutzgruppe erhalten werden.

Verbindungen der Formel VII stellen neue Verbindungen dar, welche wertvolle Zwischenprodukte zur Herstellung von pharmakologisch wirksamen Verbindungen, beispielsweise den Verbindungen der Formel I, darstellen. Verbindungen der Formel VII können erhalten werden, indem man Verbindungen der allgemeinen Formel XXIII
(siehe Formel XXIII)
worin R¹', R³', R⁴', R⁵' und n obige Bedeutung besitzen, auf an sich bekannte Weise zu Verbindungen der allgemeinen Formel XXIV
(siehe Formel XXIV)
worin R¹', R³', R⁴', R⁵' und n obige Bedeutung besitzen und R⁹' eine dem Rest R⁹ entsprechende Acylgruppe darstellt, acyliert und die erhaltenen Verbindungen der Formel XXIV anschließend zu den Verbindungen der Formel VII reduziert.

Zur Acylierung können die Verbindungen der Formel XXIII mit einem Acylierungsmittel der allgemeinen Formel XXV
(siehe Formel XXV)
worin R^{9'} obige Bedeutung besitzt und X' Halogen oder eine niedere Alkoxycarbonylgruppe bedeutet, bei erhöhten Temperaturen, beispielsweise Temperaturen zwischen 30 und 100 °C, umgesetzt werden. Als Lösungsmittel kann ein Überschuß des Acylierungsmittels dienen, dem gewünschtenfalls weitere organische Lösungsmittel wie offenkettige oder cyclische Äther, chlorierte Kohlenwasserstoffe wie Dichlormethan oder aromatische Kohlenwasserstoffe wie Benzol zugesetzt werden können. Vorzugsweise wird als Acylierungsmittel das entsprechende Säureanhydrid eingesetzt. Zur Formylierung wird zweckmäßigerweise ein in situ aus Ameisensäure und einem niederen Carbonsäureanhydrid, vorzugsweise Acetanhydrid, gebildetes gemischtes Anhydrid verwendet.

Die Reduktion von Verbindungen der Formel XXIV zu Verbindungen der Formel VII kann auf an sich bekannte Weise in einem unter den Reaktionsbedingungen inerten organischen Lösungsmittel mit Hilfe eines Hydridreduktionsmittels erfolgen, welches befähigt ist den Alkanoylrest R^{9'} der gemischten Imidgruppierung der Verbindungen der Formel XXIV zur Hydroxyalkylgruppe zu reduzieren ohne die Lactam-Gruppe des Oxo-azepin-Ringgerüstes oder allfällige Amidschutzgruppen anzugreifen. Als zweckmäßig erweist sich beispielsweise die Reduktion mit Diniederalkylaluminiumhydriden, insbesondere Diisobutylaluminiumhydrid, Diboran oder Diniederalkylborhydriden bei tiefen Temperaturen, beispielsweise Temperaturen zwichen - 80 °C und Raumtemperatur. Als Lösungsmittel eignen sich beispielsweise offenkettige oder cyclische Äther wie Diethylether oder Tetrahydrofuran, aromatische Kohlenwasserstoffe wie Benzol oder Toluol oder halogenierte Kohlenwasserstoffe wie Dichlormethan oder Gemische derartiger Lösungsmittel.

Verbindungen der Formel XXIII umfassen Verbindungen der Formel I worin R² Wasserstoff bedeutet, D eine Bindung darstellt und R³ nicht Hydroxy darstellt und R⁴ und/oder R⁵ nicht Wasserstoff bedeuten, oder können aus entsprechenden Verbindungen der Formel I worin R⁴, R⁵ und/oder R¹ Wasserstoff bedeuten, durch Einführung von Aminoschutzgruppen auf an sich bekannte Weise erhalten werden.

Verbindungen der Formel VIII umfassen Verbindungen der Formel I, worin R² Wasserstoff bedeutet, D eine Bindung darstellt und R³ nicht Hydroxy darstellt und die Reste R¹, R⁴ und R⁵ nicht Wasserstoff bedeuten, oder können aus entsprechenden Verbindungen der Formel I worin R¹, R⁴ und/oder R⁵ Wasserstoff bedeuten, durch Einführung von Aminoschutzgruppen auf an sich bekannte Weise erhalten werden.

Verbindungen der allgemeinen Formel IX umfassen Verbindungen der Formel I, worin R¹ Wasserstoff bedeutet, D eine Bindung darstellt und R³ nicht Hydroxy darstellt und die Reste R⁴ und R⁵ nicht Wasserstoff bedeuten, oder können aus entsprechenden Verbindungen der Formel I, worin R⁴ und/oder R⁵ Wasserstoff bedeuten, durch Einführung einer Aminoschutzgruppe auf an sich bekannte Weise erhalten werden.

Verbindungen der allgemeinen Formel XXVI
(s. Formel XXVI)
worin R¹, R^{2'}, R^{3'} und n obige Bedeutung besitzen und Z für Hydroxy oder für einen der vorstehend definierten Reste X oder Y steht, umfassen die Verbindungen der Formeln III, V und XVII und stellen wertvolle Zwischenprodukte zur Herstellung von pharmakologisch aktiven Verbindungen, z.B. den Verbindungen der Formel I, dar.

Die Verbindungen der Formel I und ihre pharmakologisch akzeptablen Säureadditionssalze besitzen interessante pharmakologische Eigenschaften und zeigen eine selektive Affinität zu 5-HT₁-Rezeptoren. Sie zeichnen sich insbesondere durch eine günstige Wirkung auf die Motilität des gastrointestinalen Traktes, insbesondere des Magens, aus. So werden im Tierexperiment unter dem Einfluß von Verbindungen der Formel I die peristaltischen Wellen des Magens verstärkt, wobei sich die Frequenz der Bewegungen nicht signifikant verändert. Außerdem besitzen die Verbindungen eine serotonin-agonistische stimulierende Wirkung auf 5-HT₁-artige Rezeptoren in der Arteria basilaris, welche ein gutes Indiz für eine Antimigränewirksamkeit der Verbindungen darstellt.

Beschreibung der pharmakologischen Untersuchungsmethoden:

### 1. Bestimmung der minimalen toxischen Dosis.

Männlichen Mäusen von 20-25 g Gewicht werden p.o. Maximaldosen von 300 mg/kg der Testsubstanz verabreicht. Die Tiere werden 3 Stunden lang sorgfältig auf Toxizitätssymptome beobachtet. Über einen Zeitraum von 72 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird in der nachfolgenden Tabelle A als minimale toxische Dosis angegeben. Die in Tabelle A angegebenen Beispielsnummern beziehen sich auf die nachstehenden Herstellungsbeispiele.

### 2. Bestimmung der Wirkung auf die Motilität des Magens in narkotisierten Ratten.

Für die Tests werden jeweils Gruppen von 5-6 nüchternen Ratten des Stammes SIV 50 mit einem Körpergewicht von 220-290 g eingesetzt, welche mit einer Ketamin/Xylazin-Mischung anästhesiert werden. Die Tiere erhalten eine Anfangsdosis von 1 ml/kg einer Lösung von 50 mg/ml Ketamin und 10 mg/ml Xylazin i.p. appliziert und der Anästhetica-Spiegel wird durch eine kontinuierliche intraperitonale Infusion der gleichen Lösung mit einer Infusionsrate von 1 ml/kg/h aufrecht erhalten. Die Tiere werden tracheotomiert und laparotomiert. Nach Anlegen einer Pylorusligatur wird eine Magensonde in den Magen eingeführt und am anderen Ende über einen Drei-Wege-Hahn mit einem geeichten Druckaufnehmer (Statham-Element P 23 ID) verbunden. Eine entsprechende Sonde wird rektal 8 - 9 cm tief in das Colon eingeführt und ebenfalls in gleicher Weise mit einem geeichten Druckaufnehmer des gleichen Typs verbunden. Anschließend wird der Magen der Tiere über die Sonde mit 2 ml Wasser gefüllt. Nach einer Stabilisierungsphase von 40 min werden die Druckschwankungen im Magen und Colon über eine Testdauer von 2 Stunden und 20 Minuten gemessen und mit Hilfe eines Watanabe-Multirecorders (MC 6621) werden die durch die phasische Magenmotorik erzeugten Amplituden aufgezeichnet. Für die einzelnen Tiere werden die geometrischen Mittelwerte der Amplituden während der ersten 20 min bestimmt und dienen als Kontrollamplitudenwerte. Nach diesen 20 min werden die Testsubstanzen i.p. appliziert. Die durch die Testsubstanzen bewirkte maximale Amplitudensteigerung (=Mittelwert aus demjenigen 20-Minutenzeitraum, in welchem die höchste Amplitudensteigerung auftritt) wird in % Steigerung der vor der Applikation der Testsubstanzen ermittelten Kontrollamplitudenwerte bestimmt, und in der nachfolgenden Tabelle A wird der Mittelwert für die Tiergruppe angegeben. Außerdem kann die durch die Testsubstanzen während der Testdauer bewirkte maximale Steigerung des mittleren Magentonus gegenüber dem vor der Applikation der Testsubstanzen vorhandenen mittleren Magentonus aufgezeichnet werden. Diese Magentonussteigerung wird in Tabelle A in cm H₂O (Mittelwert der Tiergruppe) angegeben. Im Colon bewirken die Teststubstanzen eine Amplitudendämpfung.

**Tabelle A**

| Testsubstanz Beispiel Nr. | Magenmotilitätsfördernde Wirkung an der Ratte | | | Minimale toxische Dosis mg/kg Maus p.o. |
|---|---|---|---|---|
| | Dosis µMol/ kg i.p. | maximale Amplitudensteigerung= max. Amplitude in %-Steigerung der Kontrollamplitude | Magentonussteigerung in cm H₂O | |
| 1 | 10 | 449 | 7,4 | > 300 |
| 2 | 100 | 239 | | > 300 |
| 4 | 100 | 532 | 9,0 | > 300 |
| 5a | 100 | 1129 | 9,5 | > 300 |
| 5b | 100 | 449 | 3,8 | > 300 |
| 6 | 100 | 609 | 15,4 | > 300 |
| 8 | 100 | 569 | | > 300 |
| 9 | 10 | 334 | 5,5 | > 300 |
| 10 | 100 | 663 | 10,5 | > 300 |
| 13 | 100 | 332 | 7,2 | > 300 |
| 14 | 100 | 1115 | 3,0 | > 300 |
| 15 | 100 | 308 | 3,1 | > 300 |
| 17 | 100 | 293 | | > 300 |
| 24 | 100 | 195 | | > 300 |
| 25 | 100 | 206 | | 300 |
| 28 | 100 | 17 | 0,5 | > 300 |
| 29 | 100 | 154 | | > 300 |
| 31 | 100 | 928 | | > 300 |
| 32 | 100 | 922 | | > 300 |
| 34 | 100 | 467 | | |
| 36 | 100 | 351 | 11,5 | > 300 |
| 37 | 100 | 950 | 4,0 | > 300 |
| 38 | 100 | 559 | 12,4 | > 300 |
| 39 | 100 | 569 | 1,8 | > 300 |
| 40 | 100 | 997 | 6,5 | > 300 |
| 41 | 100 | 234 | 2,7 | > 300 |
| 45 | 100 | 580 | 3,5 | > 300 |
| 47 | 100 | 1795 | 6,7 | |

### 3. Untersuchung der auf eine Antimigränewirksamkeit der Testsubstanzen deutenden Eigenschaften in vitro.

Migräneschmerzen sind mit einer übermäßigen Dilatation des cranialen Gefäßsystems assoziiert.

Die Testsubstanzen besitzen Gefäßkontraktionen stimulierende Serotonin-agonistische Wirkungen auf 5-HT₁-artige Rezeptoren in der Arteria basilaris, auf Grund deren auf eine Antimigräne-Wirkung der Substanzen zu schließen ist. Die Serotonin-agonistische Wirkung der Substanzen an 5-HT₁-artigen Rezeptoren kann in vitro an isolierten Organstreifen aus der Basilararterie des Schweines bestimmt werden. Serotonin ruft durch Stimulation von 5HT₁-artigen Rezeptoren eine konzentrationsabhängige Kontraktion am isolierten Organstreifen der Basilararterie des Schweines hervor. Eine derartige Kontraktion wird auch von den Testsubstanzen hervorgerufen und stellt ein gutes Indiz für die Antimigräne-Wirksamkeit der Substanzen dar. Die Untersuchung wird nach der von van Charldorp et al beschriebenen Methode (Naunyn-Schmiedeb. Arch. Pharmacol. supp. zu Band 341, R89, 1990 und Eur.J.Pharmacol. 183, 1106 - 1107, 1990) durchgeführt.

Versuchsbeschreibung für die in vitro Bestimmung der Serotonin-agonistischen Kontraktionen induzierenden Wirkung an Segmenten der isolierten Basilararterie des Schweines.

Für den Versuch werden spiralförmige Segmente der Basilararterie von Schweinen verwendet, welche aus vom Schlachthof gelieferten Schweinehirnen isoliert wird.

Jeder Streifen wird in einem Organbad aus 10 ml einer auf pH=7,4 eingestellten modifizierten Tyrode-Lösung (= Tyrode-Lösung* mit Zusatz von 10⁻⁸ Mol/l Indomethacin, 10⁻⁷ Mol/l Atropin und 10⁻⁷ Mol/l Propanolol) so befestigt, daß das Gewebe unter einer Spannung von 10 mN steht. Das Bad wird mit einer Mischung aus 95 % O₂ und 5 % CO₂ begast. Nach einer Äquilibrierungsphase von 1 Stunde wird zweimal jeweils durch Zugabe von Serotonin bei einer Konzentration von 10⁻⁵ Mol/l eine Kontraktion des Gewebes hervorgerufen und anschließend das Präparat ausgewaschen. Dann wird die Kontraktionswirkung bei ansteigender Serotonin-Konzentration gemessen und eine cumulative Konzentrationswirkungskurve für Serotonin aufgestellt. An dem gleichen Präparat wird (nach nochmaligem Auswaschen) eine cumulative Konzentrationswirkungskurve für die Testsubstanz aufgestellt.
* Tyrodelösung = wäßrige Lösung enthaltend pro Liter 150,0 mMol NaCl, 4,0 mMol KCl, 1,8 mMol CaCl₂·2H₂O, 1,1 mMol MgCl₂·6H₂O, 25,0 mMol NaHCO₃, 0,3 mMol NaH₂PO₄·H₂O und 11,1 mMol Glucose.

In der nachfolgenden Tabelle B werden die maximale durch die Testsubstanzen bewirkte Kontraktion im Verhältnis zu der maximalen durch Serotonin hervorgerufenen Kontraktion, sowie der negative Logarithmus derjenigen Konzentration der Testsubstanz, welche 50 % der durch diese Testsubstanz erzeugbaren Maximalkontraktion hervorruft (= pD₂), und die relative Wirkpotenz berechnet als pD₂ der Testsubstanz bezogen auf den an dem selben Arterienstück ermittelten pD₂ von Serotonin angegeben.

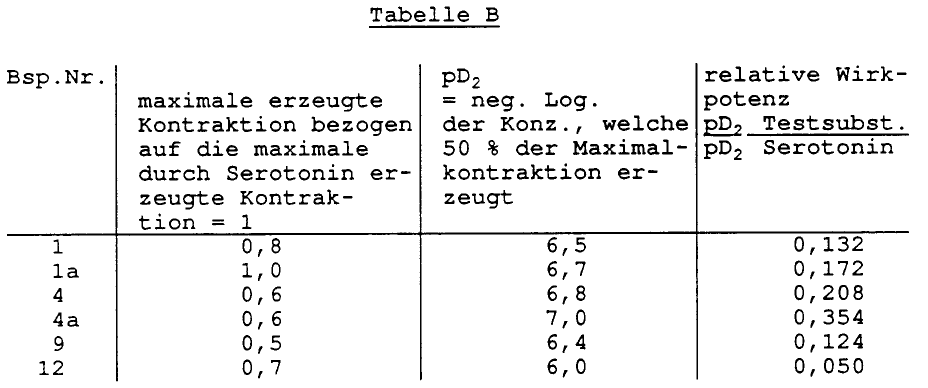

Aufgrund ihrer Wirkungen sind die Verbindungen der Formel I und ihre physiologisch verträglichen Säureadditionssalze in der Gastroenterologie als Arzneimittel für größere Säugetiere, insbesondere Menschen, zur Prophylaxe und Behandlung von Motilitätsstörungen im Magen-Darm-Trakt geeignet. So sind die Substanzen beispielsweise nützlich zur Behandlung von verschiedenen durch Motilitätsstörungen des Magen-Darm-Traktes hervorgerufenen Beschwerden wie Übelkeit, Völlegefühl, Bauchschmerzen oder Reizdarmsyndrom. Ferner sind die Verbindungen bei der Behandlung und Prophylaxe von Migräne und verwandten durch Dilatation des cranialen Gefäßsystems verursachten Störungen und Kopfschmerzen nützlich und haben dabei auch schmerzlindernde Wirkungen.

Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich jedoch für Applikationen an größeren Säugetieren, insbesondere Menschen, Arzneiformen mit einem Wirkstoffgehalt von 25 bis 300 mg pro peroraler Einzeldosis.

Als Heilmittel können die Verbindungen der Formel I mit üblichen pharmazeutischen Hilfsstoffen in galenischen Zubereitungen, wie z.B. Tabletten, Kapseln, Suppositorien oder Lösungen, z.B. Injektionslösungen oder Lösungen für orale Einnahme oder sublinguale Einnahme, beispielsweise in Sprayform, enthalten sein. Diese galenischen Zubereitungen können nach an sich bekannten Methoden hergestellt werden unter Verwendung üblicher fester Trägerstofffe wie z.B. Milchzucker, Stärke oder Talkum oder flüssigen Paraffinen und unter Verwendung von üblichen pharmazeutischen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

### Beispiel 1:

### 3-Aminomethyl-3,4,5,6-tetrahydro-6-oxo-1H-azepino[5,4,3-cd]indol.

A) Zu einer Lösung von 100 g Indol-4-carbonsäuremethylester in 1000 ml Dimethylformamid wurden unter Eiskühlung 90 ml Phosphoroxychlorid innerhalb von 30 Minuten zugetropft. Dann wurde das Eisbad entfernt und das Reaktionsgemisch weitere 3 Stunden bei Raumtemperatur gerührt. Zur Aufarbeitung wurde das Reaktionsgemisch sodann unter Eiskühlung und starkem Rühren mit 1000 ml Dichlormethan verdünnt. Dann wurden 200 ml Wasser und 100 ml 40 %-ige Natriumhydroxydlösung so langsam zugesetzt, daß die Temperatur nicht über 40 °C anstieg. Die organische Phase wurde abgetrennt, zweimal mit je 50 ml 10 %-iger Natriumhydroxydlösung gewaschen, über Natriumsulfat getrocknet und eingeengt. Der als öliger Rückstand verbleibende rohe 3-Formylindol-4-carbonsäuremethylester wurde in Diethylether aufgenommen. Aus der etherischen Lösung kristallisierte das Produkt aus und wurde abfiltriert. Nach Trocknen wurden 83,4 g 3-Formylindol-4-carbonsäuremethylester mit einem Schmelzpunkt von 134-135 °C erhalten.
B) 60 g des vorstehend erhaltenen Produktes wurden bei Raumtemperatur unter Rühren mit 350 ml Nitromethan und 5 ml 1,8-Diazabicyclo[5.4.0]undec-7-en(1,5-5) (= DBU) gemischt und das Reaktionsgemisch wurde 6 Stunden bei einer Temperatur von 65 °C reagieren gelassen. Zur Aufarbeitung wurde das Reaktionsgemisch anschließend zur Trockene eingeengt und der Rückstand in Essigsäureethylester gelöst. Die Lösung wurde zweimal mit Wasser gewaschen, über Natriumsulfat getrocknet, filtriert und eingeengt. Der als öliger Rückstand verbleibende rohe 3-(1,3-Dinitro-prop-2-yl)-indol-4-carbonsäuremethylester wurde in Diethylether gelöst. Aus der etherischen Lösung kristallisierte das Produkt aus. Das Kristallisat wurde abfiltriert, mit Diethylether nachgewaschen und getrocknet. Es wurden 67 g 3-(1,3-Dinitroprop-2-yl)-indol-4-carbonsäuremethylester mit einem Schmelzpunkt von 110 bis 112 °C erhalten.
C) 67 g des vorstehend erhaltenen Produktes wurden in einem Liter Methanol gelöst. Zu der Lösung wurden 5 g Raney-Nickel hinzugegeben. Dann wurden zu dem Reaktionsgemisch 150 ml Hydrazin-hydrat zugetropft. Hierbei stieg die Temperatur unter heftiger Gasentwicklung auf 40 °C an. Anschließend wurde das Reaktionsgemisch 1 ½ Stunden lang auf eine Temperatur von 55 °C erwärmt. Zur Aufarbeitung wurde das Reaktionsgemisch abgekühlt, vom Katalysator abfiltriert und zur Trockene eingeengt. Die als Rückstand verbleibende rohe Titelverbindung wurde mittels Säulenchromatographie an Kieselgel unter leicht erhöhtem Druck (Flash-Chromatographie) unter Verwendung von Methanol/25 %-ige wäßrige Ammoniaklösung 96:4 als Elutionsmittel gereinigt. Der nach Abdampfen des Lösungsmittelgemisches verbleibende schaumige Rückstand wurde in Methanol aufgenommen und kristallisiert. Das Kristallisat wurde abgesaugt und mit einem Gemisch Methanol/Diethylether 9:1 nachgewaschen und getrocknet. Es wurden 36,4 g 3-Aminomethyl-3,4,5,6-tetrahydro-6-oxo-1H-azepino[5,4,3-cd]indol-hydrat mit einem Schmelzpunkt von 119 bis 120 °C erhalten.
   Zur Überführung in das Hydrochlorid wurden 13 g des vorstehend erhaltenen Hydrates der Titelverbindung in 200 ml Methanol gelöst. Zu der Lösung wurde unter Rühren tropfenweise ein Überschuß an etherischer Salzsäurelösung zugegeben. Das bei Raumtemperatur gebildete Kristallisat wurde abfiltriert und mit einem Gemisch Methanol/Diethylether 9:1 nachgewaschen und getrocknet. Es wurden 12 g 3-Aminomethyl-3,4,5,6-tetrahydro-6-oxo-1H-azepino[5,4,3-cd]indol-hydrochlorid-hydrat mit einem Schmelzpunkt von 193 bis 204 °C erhalten.
D) Enantiomerentrennung:
   Die racemische Titelverbindung wurde wie folgt in ihre optischen Antipoden
   1a) 3R(+)-3-Aminomethyl-3,4,5,6-tetrahydro-6-oxo-1H-azepino[5,4,3-cd]indol
      und
   1b) 3S(-)-3-Aminomethyl-3,4,5,6-tetrahydro-6-oxo-1H-azepino[5,4,3-cd]indol
   aufgetrennt.
Da1) 8,2 g racemisches 3-Aminomethyl-3,4,5,6-tetrahydro-6-oxo-1H-azepino[5,4,3-cd]indol-hydrat wurden in 50 ml Methanol bei Rückflußtemperatur des Methanols gelöst und zu der Lösung wurden unter Rühren 5,12 g D(-)-Weinsäure zugegeben. Dann wurden 5 ml Wasser zugesetzt und das Reaktionsgemisch bei 70 °C Wasserbadtemperatur gerührt, bis eine klare Lösung erhalten wurde. Diese Lösung wurde 24 Stunden in einem offenen Gefäß bei Raumtemperatur gerührt. Das gebildete Kristallisat wurde von der Mutterlauge abgetrennt und mit wenig Methanol gewaschen. Nach Trocknung wurden 5,9 g Kristallisat erhalten, welches aus Methanol/Wasser 9:1 umkristallisiert und getrocknet wurde. Es wurden 3,5 g 3R(+)-3-Aminomethyl-3,4,5,6-tetrahydro-6-oxo-1H-azepino[5,4,3-cd]indol-D-tartrat mit einem spezifischen optischen Drehwert [α]²⁵_{D}= + 92,8 ° (c = 0,25 in Wasser) erhalten.
Da2) 2,12 g des vorstehend erhaltenen Tartrates wurden in 6,3 ml Wasser bei einer Temperatur von 95 °C gelöst. Die Lösung hatte einen pH-Wert von 3,5. Durch Zugabe von 25-%iger isopropanolischer Salzsäurelösung wurde der pH-Wert der Lösung auf 0,5 gesenkt und die Lösung wurde anschließend unter vermindertem Druck (Wasserstrahlpumpenvakuum) auf 4 ml eingeengt. Das gebildete Kristallisat wurde nach 24 Stunden aus dem wäßrigen Reaktionsgemisch abfiltriert, mit wenig Ethanol gewaschen und getrocknet. Es wurden 0,94 g 3R(+)-3-Aminomethyl-3,4,5,6-tetrahydro-6-oxo-1H-azepino[5,4,3-cd]indol-hydrochlorid mit einem Schmelzpunkt von 295 °C (Zersetzung) und einem spezifischen optischen Drehwert von [α]²⁵_{D} = + 232,4 ° (c = 0,25 in Wasser) erhalten.
Da3) Das in Da2) verbleibende wäßrige Filtrat wurde durch Zugabe von gesättigter wäßriger Natriumcarbonatlösung auf einen pH-Wert von 11 gebracht und anschließend unter vermindertem Druck (Wasserstrahlpumpenvakuum) eingedampft. Der verbleibende Rückstand wurde durch Chromatographie unter leicht erhöhtem Druck (Flash-Chromatographie) an Kieselgel unter Verwendung von Methanol/25-%ige wäßrige Ammoniaklösung 96:4 als Elutionsmittel gereinigt. Es wurden 0,32 g amorphes 3R(+)-3-Aminomethyl-3,4,5,6-tetrahydro-6-oxo-1H-azepino[5,4,3-cd]indol mit einem spezifischen optischen Drehwert von [α]²²_{D} = + 104° (c = 1 in Methanol) erhalten.
Db1) Die in der Verfahrensstufe Da1) erhaltene Mutterlauge wurde eingedampft und der Rückstand wurde in 20 ml Wasser bei einer Temperatur von 80 °C gelöst. Nach Abkühlen der Lösung auf Raumtemperatur wurden 4 ml Isopropanol zugesetzt. Die Lösung wurde 48 Stunden bei Raumtemperatur in einem offenen Gefäß gerührt. Das gebildete Kristallisat wurde abfiltriert, getrocknet und zweimal aus Wasser/Isopropanol 20:4 umkristallisiert. Es wurden 1,05 g 3S(-)-3-Aminomethyl-3,4,5,6-tetrahydro-6-oxo-1H-azepino[5,4,3-cd]indol-D-tartrat mit einem spezifischen optischen Drehwert von [α]²⁵_{D} = - 143 ° (c = 1 in Methanol) erhalten.
Db2) 0,95 g des vorstehend erhaltenen Tartrates wurden analog der in Verfahrensstufe Da2) beschriebenen Methode direkt in das entsprechende Hydrochlorid überführt. Es wurden 0,3 g 3S(-)-3-Aminomethyl-3,4,5,6-tetrahydro-6-oxo-1H-azepino[5,4,3-cd]indol-hydrochlorid mit einem spezifischen optischen Drehwert von [α]²⁵_{D} = - 222 ° (c = 0,25 in Wasser) erhalten.
Db3) Aus dem in der Verfahrensstufe Db2) verbleibenden wäßrigen Filtrat wurde analog der in Verfahrensstufe Da3) beschriebenen Methode die Base freigesetzt und gereinigt. Es wurden 0,15 g amorphes 3S(-)-3-Aminomethyl-3,4,5,6-tetrahydro-6-oxo-1H-azepino[5,4,3-cd]indol mit einem spezifischen optischen Drehwert von [α]²⁵_{D} = - 103 ° (c = 1 in Methanol) erhalten.

### Beispiel 2:

### 3-Benzylaminomethyl-3,4,5,6-tetrahydro-6-oxo-1H-azepino[5, 4,3-cd]indol.

6,2 g 3-Aminomethyl-3,4,5,6-tetrahydro-6-oxo-1H-azepino[5, 4,3-cd]indol-hydrat (Herstellung siehe Beispiel 1C) wurden in 120 ml Benzol gegeben und dem Gemisch wurden 3,3 ml Benzaldehyd zugesetzt. Dieses Reaktionsgemisch wurde 3 Stunden am Wasserabscheider zum Sieden erhitzt. Anschließend wurde das Reaktionsgemisch zur Trockene eingeengt. Das als öliger Rückstand verbleibende rohe 3-Benzylidenaminomethyl-3,4,5,6-tetrahydro-6-oxo-1H-azepino[5,4,3-cd]indol wurde sofort weiterverarbeitet. Hierzu wurde es in 40 ml Eisessig gelöst und der Lösung wurden unter Eiskühlung und Rühren 1,2 g Natriumborhydrid portionsweise zugesetzt. Dann wurde das Reaktionsgemisch 30 Minuten bei Raumtemperatur gerührt. Zur Aufarbeitung wurde der Eisessig unter vermindertem Druck (Wasserstrahlvakuum) abdestilliert. Der verbleibende ölige Rückstand wurde in 100 ml Wasser aufgenommen und die Mischung wurde durch Zugabe von gesättigter wäßriger Natriumcarbonatlösung bis auf pH 9 alkalisiert. Die sich als Öl abscheidende rohe Titelverbindung wurde abgetrennt und die wäßrige Phase wurde noch dreimal mit je 100 ml Methylenchlorid extrahiert. Die organischen Phasen wurden vereinigt, über Natriumsulfat getrocknet, filtriert und zur Trockene eingeengt. Das verbleibende Rohprodukt wurde mittels Säulenchromatographie an Kieselgel unter leicht erhöhtem Druck (Flash-Chromatographie) unter Verwendung von Essigsäureethylester/Methanol 4:1 als Elutionsmittel gereinigt. Nach Abdampfen des Elutionsmittels wurden 7 g 3-Benzylaminomethyl-3,4,5,6-tetrahydro-6-oxo-1H-azepino[5,4,3-cd]indol als schaumiges Produkt erhalten.

3 g der vorstehend erhaltenen Titelverbindung wurden in Methanol gelöst und wie in Beispiel 1C) beschrieben in ihr Hydrochlorid überführt. Es wurden 2,9 g 3-Benzylaminomethyl-3,4,5,6-tetrahydro-6-oxo-1H-azepino[5,4,3-cd]indol-hydrochlorid mit einem Schmelzpunkt von 179 bis 181 °C erhalten.

### Beispiel 3:

### 3-(N-Benzyl-N-methylaminomethyl)-3,4,5,6-tetrahydro-6-oxo-1H-azepino[5,4,3-cd]indol.

6,2 g 3-Benzylaminomethyl-3,4,5,6-tetrahydro-6-oxo-1H-azepino[5,4,3-cd]indol (Herstellung siehe Beispiel 2) wurden in 250 ml Dimethylformamid gelöst. Zu der Lösung wurden bei Raumtemperatur unter Rühren 0,81 ml Methyljodid und 1,81 g Kaliumcarbonat gegeben. Das Reaktionsgemisch wurde 3 Stunden bei Raumtemperatur gerührt. Anschließend wurde zur Aufarbeitung das Dimethylformamid bei 50 °C unter vermindertem Druck (Ölpumpenvakuum) abgezogen. Der verbleibende ölige Rückstand wurde in einer Mischung aus gleichen Teilen Wasser und Dichlormethan aufgenommen, die Dichlormethanphase abgetrennt und die wäßrige Phase noch zweimal mit Dichlormethan extrahiert. Die organischen Phasen wurden vereinigt, über Natriumsulfat getrocknet, filtriert und eingedampft. Als Rückstand verblieben 3,84 g öliges Rohprodukt. Dieses wurde durch Chromatographie unter mäßig erhöhtem Druck (= Niederdruckchromatographie = LPLC bei 3 bis 6 bar) an Kieselgel (Handelsprodukt LiChroprep^{R} Si 60) unter Verwendung von Dichlormethan/Methanol 9:1 als Elutionsmittel gereinigt. Es wurden 3,26 g öliges 3-(N-Benzyl-N-methylaminomethyl)-3,4,5,6-tetrahydro-6-oxo-1H-azepino[5,4,3-cd]indol erhalten.

0,88 g der vorstehend erhaltenen Titelverbindung wurden unter leichtem Erwärmen in 5 ml Isopropanol gelöst. Zu dieser Lösung wurde eine Lösung 0,44 g Fumarsäure in einem Gemisch aus 9 ml Isopropanol und 1 ml Methanol gegeben. Dann wurde die Mischung bis zur leichten Trübung mit 1 ml Methyl-tert.-butylether versetzt. Aus diesem Reaktionsgemisch kristallisierte das Fumarat aus. Nach Abfiltrieren des Kristallisates, Waschen mit Methyl-tert.-butylether und Trocknen wurden 1,07 g 3-(N-Benzyl-N-methylaminomethyl)-3,4,5,6-tetrahydro-6-oxo-1H-azepino[5,4,3-cd]indol-hydrogenfumarat-semihydrat mit einem Schmelzpunkt von 191 bis 193 °C erhalten.

### Beispiel 4:

### 3-Methylaminomethyl-3,4,5,6-tetrahydro-6-oxo-1H-azepino[5, 4,3-cd]indol.

A1) 2,38 g 3-(N-Benzyl-N-methylaminomethyl)-3,4,5,6-tetrahydro-6-oxo-1H-azepino[5,4,3-cd]indol (Herstellung siehe Beispiel 3) wurden in 150 ml Methanol gelöst. Zu der Lösung wurde unter einem Stickstoffstrom eine Spatelspitze eines Palladium/Kohlekatalysators (10 %-ig) zugesetzt. Anschließend wurde die Mischung in einem Autoklaven bei einem Wasserstoffdruck von 3,5 bar bei einer Temperatur von 50 °C unter Rühren hydriert. Nach 3 Stunden wurde der Wasserstoff abgelassen, das Reaktionsgemisch mit Stickstoff gespült und der Katalysator abfiltriert. Die verbleibende Lösung wurde eingedampft und das als Rückstand verbleibende ölige Rohprodukt wurde durch Niederdruckchromatorgraphie an Kieselgel unter Verwendung von Methanol/25 %-ige wäßrige Ammoniaklösung 96:4 als Elutionsmittel gereinigt. Es wurden 0,9 g 3-Methylaminomethyl-3,4,5,6-tetrahydro-6-oxo-1H-azepino[5,4,3-cd]indol als schaumiges Produkt erhalten.
   0,62 g der vorstehend erhaltenen Titelbase wurden wie in Beispiel 1C) beschrieben in das Hydrochlorid überführt. Es wurden 0,56 g 3-Methylaminomethyl-3,4,5,6-tetrahydro-6-oxo-1H-azepino[5,4,3-cd]indol-hydrochlori d mit einem Schmelzpunkt von 274 bis 277 °C erhalten.
A2) Anstelle von 3-(N-Benzyl-N-methylaminomethyl)-3,4,5,6-tetrahydro-6-oxo-1H-azepino[5,4,3-cd]indol kann als Ausgangsverbindung auch das 3-(N-Diphenylmethyl-N-methylamino)-3,4,5,6-tetrahydro-6-oxo-1H-azepino[5,4,3-cd]indol (Schmelzpunkt 192 °C unter Zersetzung) eingesetzt werden, welches analog Beispiel 3 ausgehend von 3-[N-(Diphenylmethyl)-aminomethyl]-3,4,5,6-tetrahydro-6-oxo-1H-azepino[5,4,3-cd]indol (Schmelzpunkt des Hydrochlorids 291 °C unter Zersetzung, Herstellung analog Beispiel 2 ausgehend von 3-Aminomethyl-3,4,5,6-tetrahydro-6-oxo-1H-azepino[5,4,3-cd]indol-hydrat und Benzophenon) erhalten werden kann.
B) Enantiomerentrennung:
   Die racemische Titelverbindung kann analog der in Beispiel 1D beschriebenen Methode in ihre optische Antipoden aufgetrennt werden. Es wurden
   4a) 3R(+)-3-Methylaminomethyl-3,4,5,6-tetrahydro-6-oxo-1H-azepino[5,4,3-cd]indol-Hydrochlorid, Schmelzpunkt 310 °C (Zersetzung), [α]_{D}²⁵ = 218,6 ° (c=1 in Methanol)
      und
   4b) 3S(-)-3-Methylaminomethyl-3,4,5,6-tetrahydro-6-oxo-1H-azepino[5,4,3-cd]indol-Hydrochlorid, Schmelzpunkt > 280 °C (Zersetzung), [α]_{D}²⁵ = - 224,9 ° (c=1 in Methanol) erhalten.
C) Enantiomerentrennung 2. Methode:
   Ca1) 2,14 g N-Ethoxycarbonyl-L-leucin und 2,94 ml Triethylamin wurden in 75 ml trockenem Tetrahydrofuran gelöst. Die Lösung wurde auf ca. - 15 °C abgekühlt. Dann wurde eine Lösung von 1,37 ml Chlorameisensäure-isobutylester in 10 ml trockenem Tetrahydrofuran über einen Zeitraum von 10 Minuten verteilt unter Stickstoffatmosphäre zugegeben. Das Reaktionsgemisch wurde weitere 10 Minuten gerührt. Dann wurden über einen Zeitraum von 15 Minuten verteilt 2,8 g racemisches 3-Methylaminomethyl-3,4,5,6-tetrahydro-6-oxo-1H-azepino[5,4,3-cd]indol portionsweise bei einer Temperatur von ca. - 15 °C zugegeben. Das Reaktionsgemisch wurde dann langsam auf Raumtemperatur erwärmt und weitere 3 Stunden gerührt. Anschließend wurde unter vermindertem Druck eingedampft und der Rückstand wurde durch Chromatographie unter leicht erhöhtem Druck (Flash-Chromatographie) unter Verwendung von Ethylacetat/Methanol 99:1 als Elutionsmittel gereinigt. 3 g des gereinigten Produktes wurden durch präparative Hochdruckflüssigkeitschromatographie (HPLC) in das (+)- und das (-)-Enantiomere getrennt.
   Ca2) 1,8 g des (+)-Enantiomeren wurden in 25 ml konzentrierter Salzsäure gelöst und die Lösung in einem Ölbad von 100 °C erhitzt. Nach 24 Stunden wurde die Lösung unter vermindertem Druck eingedampft, der Rückstand in Ethanol aufgenommen und wiederum eingedampft. Der verbleibende Rückstand wurde in wäßrigem Ethanol aufgenommen und es wurden 1,18 g Kaliumcarbonat zugegeben. Das Gemisch wurde unter vermindertem Druck eingeengt und der verbleibende Rückstand wurde durch Chromatographie unter leicht erhöhtem Druck (Flash-Chromatographie) unter Verwendung von Methanol/wäßrige Ammoniaklösung 99,5:0,5 als Elutionsmittel gereinigt. Das erhaltene sirupartige Produkt wurde in wenig absolutem Ethanol gelöst und zu der Lösung wurde ein Überschuß an ethanolischer Salzsäurelösung gegeben. Der gebildete Niederschlag wurde abgesaugt und mit absolutem Ethanol und Diethylether gewaschen. Das erhaltene 3R(+)-3-Methylaminomethyl-3,4,5,6-tetrahydro-6-oxo-1H-azepino[5,4,3-cd]indol-Hydrochlorid hatte einen Schmelzpunkt von 310 °C (Zersetzung) und einen spezifischen optischen Drehwert [α]²⁵_{D} = +218,6 ° (Methanol).
Cb) Das in der Verfahrensstufe Ca1) erhaltene (-)-Enantiomere wurde analog der in Verfahrensstufe Ca2) beschriebenen Methode in das 3S(-)-3-Methylaminomethyl-3,4,5,6-tetrahydro-6-oxo-1H-azepino[5,4,3-cd]indol-Hydrochlorid mit einem Schmelzpunkt von > 280 °C (Zersetzung) und einem spezifischen optischen Drehwert [α]²⁵_{D} = -224,9 ° (Methanol) überführt.

### Beispiel 5:

a) 3-Ethylaminomethyl-3,4,5,6-tetrahydro-6-oxo-1H-azepino [5,4,3-cd]indol.
b) 3-Diethylaminomethyl-3,4,5,6-tetrahydro-6-oxo-1H-azepino[5,4,3-cd]indol.
   A) 6 g 3-Aminomethyl-3,4,5,6-tetrahydro-6-oxo-1H-azepino [5,4,3-cd]indol-hydrat (Herstellung siehe Beispiel 1C) wurden in 60 ml Dimethylformamid gelöst. Zu der Lösung wurden insgesamt 2,5 ml Ethylbromid und 5,1 ml Triethylamin in vier gleichen Portionen über einen Zeitraum von 6,5 Stunden verteilt zugegeben. Während dieser Zeit wurde die Temperatur des Reaktionsgemisches auf 60 °C gehalten. Zur Aufarbeitung wurde das Reaktionsgemisch zur Trockene eingedampft und das verbleibende rohe Gemisch aus den beiden Titelverbindungen wurde durch Chromatographie unter leicht erhöhtem Druck (Flash-Chromatographie) an Kieselgel unter Verwendung von Dichlormethan/Methanol/Diethylamin 80:17:3 aufgetrennt. Es wurden 3,4 g öliges Monoethylaminomethyl-Rohprodukt (= Verbindung 5a) und 4,1 g öliges Diethylaminomethyl-Rohprodukt (= Verbindung 5b) erhalten.
      Das ölige Rohprodukt 5a) wurde in Methanol gelöst. Aus der Lösung wurden unter Zusatz von wenig Diethylether 2,3 g 3-Ethylaminomethyl-3,4,5,6-tetrahydro-6-oxo-1H-azepino[5,4,3-cd]indol mit einem Schmelzpunkt von 90 bis 92 °C kristallisiert.
      Das ölige Rohprodukt 5b) wurde in Ethanol gelöst und durch Zusatz von etherischer Salzsäurelösung wurde wie in Beispiel 1C) beschrieben das Hydrochlorid hergestellt. Es wurden 2,5 g 3-Diethylaminomethyl-3,4,5,6-tetrahydro-6-oxo-1H-azepino[5,4,3-cd]indol-hydrochlorid mit einem Schmelzpunkt von 150 bis 153 °C erhalten.

### Beispiel 6:

### 3-Isopropylaminomethyl-3,4,5,6-tetrahydro-6-oxo-1H-azepino [5,4,3-cd]indol.

Zu einer Lösung von 3-Aminomethyl-3,4,5,6-tetrahydro-6-oxo-1H-azepino[5,4,3-cd]indol-hydrat (Herstelung siehe Beispiel 1C) in 80 ml Tetrahydrofuran wurden unter Rühren 0,82 g Boran-Dimethylamin-Komplex zugesetzt. Die Lösung wurde 1 Stunde am Rückfluß erhitzt und anschließend wurden bei einer Temperatur von 45 °C 10 ml Aceton zugetropft. Das Reaktionsgemisch wurde 2,5 Stunden lang bei einer Temperatur von 60 °C gerührt. Zur Aufarbeitung wurde das Reaktionsgemisch anschließend zur Trockene eingedampft und der Rückstand wurde in einem Gemisch aus Wasser und Dichlormethan gelöst. Die Dichlormethanphase wurde abgetrennt und die wäßrige Phase wurde noch dreimal mit je 50 ml eines Gemisches aus Dichlormethan/Methanol 9:1 extrahiert. Die organischen Phasen wurden vereinigt und zur Trockene eingedampft. Das als Rückstand verbleibende Rohprodukt wurde durch Chromatographie unter leicht erhöhtem Druck (= Flash-Chromatographie) an Kieselgel unter Verwendung von Methanol/Essigsäureethylester 4:1 als Elutionsmittel gereinigt. Die Titelverbindung wurde als Öl erhalten.

Die ölige Titelverbindung wurde nach der in Beispiel 1C) beschriebenen Methode in ihr Hydrochlorid überführt. Es wurden 1,6 g 3-Isopropylaminomethyl-3,4,5,6-tetrahydro-6-oxo-1H-azepino[5,4,3-cd]indol-hydrochlorid mit einem Schmelzpunkt von 180 bis 183 °C erhalten.

### Beispiel 7:

### 4-Aminomethyl-3,4,5,6-tetrahydro-6-oxo-1H-azepino[5,4,3-cd]indol.

A) 100 g Indol-4-carbonsäuremethylester wurden in 500 ml Eisessig gelöst. Zu der Lösung wurde bei Raumtemperatur eine Mischung aus 86 ml einer 40 %-igen wäßrigen Dimethylaminlösung, 55 ml einer 37 %-igen wäßrigen Formaldehydlösung und 250 ml Eisessig getropft. Das Reaktiongsgemisch wurde anschließend 20 Stunden bei Raumtemperatur gerührt. Zur Aufarbeitung wurde der Reaktionsmischung unter Eiskühlung 20 %-ige wäßrige Natriumhydroxydlösung bis zum Erreichen von pH 9 zugesetzt. Dann wurde dreimal mit Dichlormethan extrahiert und die vereinigten organischen Extrakte wurden mit Natriumsulfat getrocknet, filtriert und eingeengt. Es wurden 127 g rohes 3-Dimethylaminomethylindol-4-carbonsäuremethylester als hellgelbes Öl erhalten.
B) 127 g des vorstehend erhaltenen Produktes wurden in 600 ml Acetonitril gelöst. Zu der Lösung wurden 68 ml Nitroessigsäureethylester zugefügt. Dann wurde bei Raumtemperatur eine Lösung von 40 ml Tributylphosphin in 350 ml Acetonitril zugetropft. Das Reaktionsgemisch wurde 2,5 Stunden auf Rückflußtemperatur erhitzt. Anschließend wurde das Reaktionsgemisch zur Aufarbeitung zur Trockene eingedampft und der Rückstand in 10 %-iger wäßriger Salzsäure gelöst. Die Lösung wurde dreimal mit Essigsäureethylester extrahiert und die vereinigten organischen Extrakte wurden über Natriumsulfat getrocknet, filtriert und eingeengt. Das verbleibende Rohprodukt wurde durch Säulenchromatographie an Kieselgel unter leicht erhöhtem Druck (Flash-Chromatographie) unter Verwendung von Cyclohexan/Essigsäureethylester 2:1 als Elutionsmittel gereinigt. Nach Eindampfen des Elutionsmittels wurde der verbleibende Rückstand aus Diethylether kristallisiert. Es wurden 66 g 3-(1-Ethoxycarbonyl-2-nitropropyl)-indol-4-carbonsäuremethylester mit einem Schmelzpunkt von 106 - 110 °C erhalten.
C) 66 g des vorstehend erhaltenen Produktes wurden in 2 l Toluol gelöst. Zu der Lösung wurden 5 g Palladium/Kohle-Katalysator (10 %-ig) zugegeben. Anschließend wurde das Reaktionsgemisch unter einem Wasserstoffdruck von 50 bar 6 Stunden lang bei einer Temperatur von 70 °C hydriert. Anschließend wurde abgekühlt, der Katalysator abfiltriert und das Filtrat eingedampft. Der verbleibende ölige Rückstand wurde zur Vervollständigung der Cyclisierung 1 Stunde lang auf 130 °C erhitzt. Das beim anschließenden Abkühlen sich bildende Kristallisat wurde mit Ethanol verrührt, abfiltriert und mit einer Mischung aus Diethylether/Ethanol 1:1 nachgewaschen. Nach dem Trocknen wurden 41,5 g 3,4,5,6-Tetrahydro-6-oxo-1H-azepino[5,4,3-cd]indol-4-carbonsäureethylester mit einem Schmelzpunkt von 185 - 188 °C erhalten.
D) 41,5 g des vorstehend erhaltenen Produktes wurden in 1,2 l Tetrahydrofuran gelöst. Zu der Lösung wurden 48,7 g Natriumborhydrid portionsweise unter Rühren bei Raumtemperatur zugegeben. Anschließend wurden 780 ml Ethanol zugetropft und das Reaktionsgemisch wurde für 1,5 Stunden auf 50 °C erhitzt. Zur Aufarbeitung wurde das Reaktionsgemisch mit Dichlormethan verdünnt und die organische Phase wurde mit Wasser extrahiert. Die wäßrige Phase wurde anschließend viermal mit einem Gemisch aus Dichlormethan/Methanol 95:5 extrahiert. Die vereinigten organischen Phasen wurden über Natriumsulfat getrocknet, filtriert und das Filtrat eingedampft. Der zurückbleibende feste Rückstand wurde in Methanol gelöst und kristallisiert. Das Kristallisat wurde abfiltriert, mit einer Mischung Methanol/Diethylether 8:2 nachgewaschen und getrocknet. Es wurden 31,3 g 4-Hydroxymethyl-3,4,5,6-tetrahydro-6-oxo-1H-azepino[5,4,3-cd]indol mit einem Schmelzpunkt von 186 bis 187 °C erhalten.
E) 31,3 g des vorstehend erhaltenen Produktes wurden in 190 ml Pyridin gelöst. Anschließend wurden zu der Lösung unter Eiskühlung und Rühren 33 g p-Toluolsulfonsäurechlorid gegeben. Das Reaktionsgemisch wurde 24 Stunden bei Raumtemperatur gerührt. Zur Aufarbeitung wurde das Reaktionsgemisch in 300 ml einer eisgekühlten, gesättigten, wäßrigen Zitronensäurelösung gegossen. Die wäßrige saure Phase wurde dreimal mit Dichlormethan extrahiert. Die vereinigten organischen Extrakte wurden über Natriumsulfat getrocknet, filtriert und eingedampft. Der verbleibende Rückstand wurde in Methanol gelöst und durch Zugabe von Diethylether kristallisiert. Das Kristallisat wurde abfiltriert, mit einer Mischung aus Methanol/Diethylether 7:3 gewaschen und getrocknet. Es wurden 36,5 g 4-(p-Toluolsulfonyloxymethyl)-3,4,5,6-tetrahydro-6-oxo-1H-azepino[5,4,3-cd]indol mit einem Schmelzpunkt von 175 - 178 °C erhalten.
F) 5 g des vorstehend erhaltenen Produktes wurden in 50 ml Dimethylformamid gelöst. Zu der Lösung wurden 5,3 g Natriumazid gegeben. Anschließend wurde das Reaktionsgemisch 2 Stunden unter Rühren auf 100 °C erhitzt. Zur Aufarbeitung wurde abgekühlt, das Reaktionsgemisch eingedampft, der Rückstand in Dichlormethan gelöst und die Dichlormethanphase zweimal mit Wasser gewaschen, über Natriumsulfat getrocknet, filtriert und eingedampft. Der verbleibende ölige Rückstand wurde in Essigsäureethylester gelöst und unter Zusatz von Diethylether kristallisiert. Das Kristallisat wurde abfiltriert und getrocknet. Es wurden 2,8 g 4-Azidomethyl-3,4,5,6-tetrahydro-6-oxo-1H-azepino[5,4,3-cd]indol mit einem Schmelzpunkt von 152 bis 153 °C erhalten.
G) 2,8 g des vorstehend erhaltenen Produktes wurden in 30 ml Methanol gelöst. Der Lösung wurde eine Spatelspitze Raney-Nickel zugesetzt, dann wurden unter Rühren 2,3 ml Hydrazin-hydrat zugegeben und das Reaktionsgemisch weitere 2 Stunden bei Raumtemperatur gerührt. Zur Aufarbeitung wurde der Katalysator über Asbestschlamm (Handelsprodukt Theorit^{R}) abgesaugt und das Filtrat eingedampft. Die als Rückstand verbleibende rohe Titelverbindung wurde in Methanol gelöst und wie in Beispiel 1C) beschrieben in ihr Hydrochlorid überführt. Das erhaltene Kristallisat wurde filtriert, mit einer Mischung aus Methanol/Diethylether 8:2 gewaschen und getrocknet. Es wurden 2,8 g 4-Aminomethyl-3,4,5,6-tetrahydro-6-oxo-1H-azepino[5,4,3-cd]indol-hydrochlorid mit einem Schmelzpunkt von 259 bis 265 °C erhalten.

### Beispiel 8:

### 4-(2-Aminoethyl)-3,4,5,6-tetrahydro-6-oxo-1H-azepino[5, 4,3-cd]indol.

A) 5 g 4-(p-Toluolsulfonyloxymethyl)-3,4,5,6-tetrahydro-6-oxo-1H-azepino[5,4,3-cd]indol (Herstellung siehe Beispiel 7E) wurden in 25 ml Dimethylformamid gelöst. Zu der Lösung wurden 970 mg Kaliumcyanid gegeben. Das Reaktionsgemisch wurde unter Rühren 3 Stunden auf 65 °C erhitzt. Zur Aufarbeitung wurde das Reaktionsgemisch nach Abkühlen mit Wasser verdünnt und dreimal mit Essigsäureethylester extrahiert. Die vereinigten organischen Phasen wurden über Natriumsulfat getrocknet, filtriert und eingedampft. Der ölige Rückstand wurde in Methanol gelöst und die Lösung wurde bis zur Kristallisation mit Diethylether versetzt. Das Kristallisat wurde abgesaugt, mit einem Gemisch aus Methanol/Diethylether 8:2 gewaschen und getrocknet. Es wurden 2,4 g 4-Cyanomethyl-3,4,5,6-tetrahydro-6-oxo-1H-azepino[5,4,3-cd]indol mit einem Schmelzpunkt von 197 bis 198 °C erhalten.
B) 1,8 g des vorstehend erhaltenen Produktes wurden in 250 ml bei 0 °C mit Ammoniak gesättigtem Methanol gelöst. Zu der Lösung wurde eine Spatelspitze Raney-Nickel gegeben. Das Reaktionsgemisch wurde anschließend in einen Autoklaven überführt und 5 Stunden bei 50 °C unter Rühren bei einem Wasserstoffdruck von 50 bar hydriert. Zur Aufarbeitung wurde vom Katalysator abfiltriert und das Filtrat eingedampft. Der verbleibende Rückstand wurde durch Niederdruckchromatographie an Kieselgel unter Verwendung von Methanol/Dichlormethan/Diethylamin 17:80:3 als Elutionsmittel gereinigt. Die Titelverbindung wurde als Öl erhalten.
   Die vorstehend erhaltene Titelbase wurde in Methanol gelöst und mit Maleinsäure analog der in Beispiel 3 beschriebenen Methode umgesetzt. Es wurden 1,4 g 4-(2-Aminoethyl)-3,4,5,6-tetrahydro-6-oxo-1H-azepino[5,4,3-cd]indol-hydrogenmaleinat mit einem Schmelzpunkt von 209 bis 210 °C erhalten.

### Beispiel 9:

### 1-Methyl-3-aminomethyl-3,4,5,6-tetrahydro-6-oxo-1H-azepino [5,4,3-cd]indol.

A) Zu einer Lösung von 7 g 3-Formylindol-4-carbonsäuremethylester (Herstellung siehe Beispiel 1A) in 250 ml trockenem Dimethylformamid wurden 2,1 g 80 %-iges Natriumhydrid portionsweise bei Raumtemperatur unter Stickstoffatmosphäre zugegeben. Das Reaktionsgemisch wurde 1 Stunde bei 50 °C gerührt. Anschließend wurden 7 ml Methyljodid durch einen Tropftrichter zugegeben, und das Reaktionsgemisch wurde weitere 2 Stunden bei 50 °C gerührt. Zur Aufarbeitung wurde das Reaktionsgemisch unter vermindertem Druck zur Trockene eingedampft und in einer Mischung von 50 ml Wasser und 50 ml Dichlormethan aufgenommen. Die Dichlormethanphase wurde abgetrennt und die wäßrige Phase nochmals mit 50 ml Dichlormethan extrahiert. Die organischen Phasen wurden vereinigt, über Natriumsulfat getrocknet, filtriert und eingedampft. Das zurückbleibende kristalline Rohprodukt wurde aus Diethylether umkristallisiert. Nach Trocknen des Kristallisates wurden 6,9 g 1-Methyl-3-formylindol-4-carbonsäuremethylester mit einem Schmelzpunkt von 128 - 129 °C erhalten.
B) 6,9 g des vorstehend erhaltenen Produktes wurden bei Raumtemperatur mit 70 ml Nitromethan und 1 ml 1,8-Diazabicyclo[5.4.0]undec-7-en(1,5-5) gemischt und das Reaktionsgemisch wurde 4 Stunden bei einer Temperatur von 65 °C gerührt. Anschließend wurde das Reaktionsgemisch wie in Beispiel 1B) beschrieben aufgearbeitet. Es wurden 5,1 g 1-Methyl-3-(1,3-dinitroprop-2-yl)-indol-4-carbonsäuremethylester mit einem Schmelzpunkt von 142 - 145 °C erhalten.
C) 5,1 g des vorstehend erhaltenen Produktes wurden in 60 ml Methanol gelöst und in Gegenwart einer Löffelspitze Raney-Nickel mit 10 ml Hydrazin-hydrat nach der in Beispiel 1C) beschriebenen Methode umgesetzt. Nach Beendigung der Reaktion wurde vom Katalysator abfiltriert und das Filtrat unter vermindertem Druck eingedampft. Die als schaumiger Rückstand verbleibende rohe Titelverbindung wurde in Methanol gelöst und wie in Beispiel 1C) beschrieben in ihr Hydrochlorid überführt. Es wurden 3,2 g 1-Methyl-3-aminomethyl-3,4,5,6-tetrahydro-6-oxo-1H-azepino[5,4,3-cd]indol-hydrochlorid mit einem Schmelzpunkt von 256 - 261 °C erhalten.

### Beispiel 10:

### 1-Benzyl-3-aminomethyl-3,4,5,6-tetrahydro-6-oxo-1H-azepino [5,4,3-cd]indol.

A) 28 g 3-Formylindol-4-carbonsäuremethylester (Herstellung siehe Beispiel 1A) wurden in 230 ml Nitromethan gelöst. Zu der Lösung wurden 4,2 g Ammoniumacetat zugefügt und das Reaktionsgemisch wurde 1 Stunde am Rückfluß gekocht. Zur Aufarbeitung wurde das Reaktionsgemisch auf Raumtemperatur abgekühlt, mit 150 ml eines Gemisches aus Essigsäureethylester/Methanol 9:1 verdünnt und anschließend in 150 ml Wasser gegossen. Die organische Phase wurde abgetrennt, über Natriumsulfat getrocknet, filtriert und unter leicht vermindertem Druck eingeengt. Das aus der eingeengten Lösung auskristallisierte Produkt wurde filtriert und getrocknet. Es wurden 25 g 3-(2-Nitrovinyl)-indol-4-carbonsäuremethylester mit einem Schmelzpunkt von 187 - 190 °C erhalten.
B) 12 g des vorstehend erhaltenen Produktes wurden in 200 ml trockenem Dimethylformamid gelöst. Zu der Lösung wurden bei Raumtemperatur portionsweise 3 g Natriumhydrid unter Stickstoffatmosphäre zugegeben. Anschließend wurde das Reaktionsgemisch auf 60 °C erhitzt und es wurden über einen Tropftrichter 6,3 ml Benzylbromid zugetropft. Das Reaktionsgemisch wurde sodann auf 80 °C erhitzt und 1 Stunde bei dieser Temperatur gerührt. Zur Aufarbeitung wurde das Reaktionsgemisch unter vermindertem Druck zur Trockene eingedampft und der Rückstand in einem Gemisch aus Wasser/Essigsäureethylester aufgenommen. Die organische Phase wurde abgetrennt und eingeengt. Der verbleibende Rückstand wurde mittels Säulenchromatographie an Kieselgel unter leicht erhöhtem Druck (Flash-Chromatographie) unter Verwendung von Essigsäureethylester als Elutionsmittel gereinigt. Der nach Einengen des Eluates verbleibende Rückstand wurde aus Dichlormethan/Diethylether 1:9 kristallisiert. Nach Trocknung wurden 10,8 g kristallines 1-Benzyl-3-(2-nitrovinyl)-indol-4-carbonsäuremethylester erhalten, welches direkt weiterverarbeitet wurde.
C) 10,8 g des vorstehend erhaltenen Produktes wurden in 150 ml Methanol gelöst. Zu der Lösung wurden bei Raumtemperatur 33 ml Nitromethan zugegeben und anschließend 2 ml Diazabicyclo[5.4.0]undec-7-en(1,5-5) langsam zugefügt. Das Reaktionsgemisch wurde anschließend 1 Stunde lang bei Raumtemperatur gerührt. Zur Aufarbeitung wurde das Reaktionsgemisch mit 150 ml Wasser verdünnt und dreimal mit je 100 ml Dichlormethan extrahiert. Die organischen Phasen wurden vereinigt, über Natriumsulfat getrocknet, filtriert und eingedampft. Das als Rückstand verbleibende ölige Rohprodukt wurde mittels Flash-Chromatographie an Kieselgel unter Verwendung von Essigsäureethylester als Elutionsmittel gereinigt. Der nach Einengen des Eluates verbleibende Rückstand wurde aus Isopropanol/Essigsäureethylester 2:8 kristallisiert. Nach Trocknen wurden 10,3 g kristallines 1-Benzyl-3-(1,3-dinitroprop-2-yl)-indol-4-carbonsäuremethylester erhalten, welches direkt weiterverarbeitet wurde.
D) 10,3 g des vorstehend erhaltenen Produktes wurden in 250 ml Methanol gelöst und in Gegenwart von 3 g mit Methanol gewaschenem Raney-Nickel mit 34 ml Hydrazinhydrat nach der in Beispiel 1C) beschriebenen Methode umgesetzt. Das Reaktionsgemisch wurde wie in Beispiel 1C) beschrieben aufgearbeitet. Die Titelverbindung wurde als Öl erhalten.
   Die vorstehend erhaltene ölige Titelbase wurde nach der in Beispiel 1C) beschriebenen Methode in ihr Hydrochlorid überführt. Es wurden 7,4 g 1-Benzyl-3-aminomethyl-3,4,5,6-tetrahydro-6-oxo-1H-azepino[5,4,3-cd]indol-hydrochlorid mit einem Schmelzpunkt von 167 bis 169 °C erhalten.

### Beispiel 11:

### 3-Aminomethyl-5-methyl-3,4,5,6-tetrahydro-6-oxo-1H-azepino [5,4,3-cd]indol.

A) Zu 50 ml Trifluoressigsäureanhydrid wurden unter Rühren und Eiskühlung 16 g 3-Aminomethyl-3,4,5,6-tetrahydro-6-oxo-1H-azepino[5,4,3-cd]indol-hydrat (Herstellung siehe Beispiel 1C) portionsweise innerhalb von 30 Minuten zugefügt. Das Reaktionsgemisch wurde 2 Stunden bei 20 °C unter Stickstoffatmosphäre gerührt. Anschließend wurde das Reaktionsgemisch unter vermindertem Druck (Wasserstrahlpumpenvakuum) zur Trockene eingedampft. Der Rückstand wurde in 200 ml Essigsäureethylester gelöst. Die Lösung wurde mit wäßriger gesättigter Natriumhydrogencarbonatlösung bis zur Erreichung eines pH-Wertes von 8,5 versetzt. Durch die Behandlung mit der wäßrigen gesättigten Natriumhydrogencarbonatlösung wurden Reste der gebildeten Trifluoressigsäure und überschüssiges Trifluoressigsäureanhydrid neutralisiert und gleichzeitig allfällige an dem Stickstoffatom in 5-Stellung des Ringgerüstes angelagerte Trifluoracetylreste wieder abgespalten. Anschließend wurde die organische Phase abgetrennt, die wäßrige Phase noch zweimal mit je 50 ml Essigsäureethylester extrahiert und die organischen Phasen vereinigt, über Natriumsulfat getrocknet, filtriert und eingedampft. Der verbleibende Rückstand wurde aus Essigsäureethylester unter Zusatz von Diethylether kristallisiert. Nach Trocknung wurde 18 g 3-Trifluoracetamidomethyl-3,4,5,6-tetrahydro-6-oxo-1H-azepino[5,4,3-cd]indol mit einem Schmelzpunkt von 228 - 232 °C erhalten.
B) 6 g des vorstehend erhaltenen Produktes wurden bei Raumtemperatur in 40 ml Ameisensäure (98 bis 100 %) gelöst. Zu der Lösung wurden bei einer Temperatur von 55 bis 65 °C über einen Zeitraum von 8 Stunden verteilt 95 ml Acetanhydrid zugetropft. Das Reaktionsgemisch wurde weitere 12 Stunden bei Raumtemperatur gerührt. Anschließend wurde die Lösung unter vermindertem Druck (Wasserstrahlpumpenvakuum) eingedampft und der Rückstand in Essigsäureethylester aufgenommen und wie in Beispiel 11A) beschrieben aufgearbeitet. Es wurden 5,3 g kristallines 3-Trifluoracetamidomethyl-5-formyl-3,4,5,6-tetrahydro-6-oxo-1H-azepino [5,4,3-cd]indol mit einem Schmelzpunkt von 198 - 205 °C erhalten.
C) 4 g des vorstehend erhaltenen Produktes wurden in 80 ml absolutem Tetrahydrofuran gelöst, und die Lösung wurde unter Stickstoffatmosphäre auf -78 °C abgekühlt. Bei dieser Temperatur wurden langsam 11,8 ml einer 1-molaren Lösung von Diisobutylaluminiumhydrid in Toluol hinzugetropft. Anschließend wurden die Kühlung entfernt. Nach 2 Stunden wurde die Lösung mit 80 ml Essigsäureethylester und 60 ml einer 10 %-igen wäßrigen Zitronensäurelösung versetzt. Die organische Phase wurde abgetrennt und die wäßrige Phase wurde noch zweimal mit je 80 ml Essigsäureethylester extrahiert. Die organischen Phasen wurden vereinigt, über Natriumsulfat getrocknet, filtriert und unter vermindertem Druck (Wasserstrahlpumpenvakuum) eingedampft. Der verbleibende Rückstand wurde aus Essigsäureethylester/ Diethylether kristallisiert. Nach Trocknen wurden 3,4 g 3-Trifluoacetamidomethyl-5-hydroxymethyl-3,4,5,6-tetrahydro-6-oxo-1H-azepino[5,4,3-cd]indol mit einem Schmelzpunkt von 150 - 151,5 °C erhalten.
D) Zu einer Suspension von 1,08 g Natriumborhydrid in 60 ml Tetrahydrofuran wurden unter Eiskühlung 25,2 ml Trifluoressigsäure unter starkem Rühren zugetropft. Zu dieser Mischung wurde eine Lösung von 1,8 g des vorstehend erhaltenen Produktes in 60 ml Tetrahydrofuran unter Eiskühlung innerhalb von 30 Minuten zugetropft. Anschließend wurde die Kühlung entfernt und das Reaktionsgemisch 1 Stunde lang auf 60 °C erhitzt. Anschließend wurde das Reaktionsgemisch im Wasserstrahlvakuum auf 10 ml eingeengt, und wie in Beispiel 11A) beschrieben weiterverarbeitet. Das erhaltene ölige Produkt wurde durch Chromatographie unter mäßig erhöhtem Druck (= Niederdruckchromatographie bei 3 bis 6 bar) an Kieselgel unter Verwendung von Essigsäureethylester als Elutionsmittel gereinigt. Das gereinigte Produkt wurde aus Essigsäureethylester/Diethylether kristallisiert. Es wurden 1,2 g 3-Trifluoracetamidomethyl-5-methyl-3,4,5,6-tetrahydro-6-oxo-1H-azepino[5,4,3-cd]indol mit einem Schmelzpunkt von 174 - 177 °C erhalten.
E) 1,2 g des vorstehend erhaltenen Produktes wurden in 40 ml Methanol gelöst. Zu der Lösung wurden 40 ml einer 5-%igen wäßrigen Kaliumcarbonatlösung zugefügt. Das Reaktionsgemisch wurde 8 Stunden bei Raumtemperatur gerührt. Zur Aufarbeitung wurde das Reaktionsgemisch anschließend mittels Chromatographie unter mäßig erhöhtem Druck (= Niederdruckchromatographie bei 3 bis 6 bar) an Kieselgel unter Verwendung von Methanol/25-%ige wäßrige Ammoniaklösung 97:3 als Elutionsmittel gereinigt. Das gereinigte Produkt wurde aus Methanol/Diethylether kristallisiert. Es wurden 0,82 g 3-Aminomethyl-5-methyl-3,4,5,6-tetrahydro-6-oxo-1H-azepino[5,4,3-cd]indol mit einem Schmelzpunkt von 85 bis 87 °C erhalten.
F) Das 3-Aminomethyl-5-methyl-3,4,5,6-tetrahydro-6-oxo-1H-azepino[5,4,3-cd]indol kann auch erhalten werden, indem man 1-Benzyl-3-Benzylaminomethyl-5-methyl-3,4,5,6-tetrahydro-6-oxo-1H-azepino[5,4,3-cd]indol (Herstellung siehe Beispiel 18) analog der in Beispiel 4 beschriebenen Methode jedoch bei einem Wasserstoffdruck von 10 bar hydrogenolytisch debenzyliert.

### Beispiel 12:

### 3-Aminomethyl-5-methoxymethyl-3,4,5,6-tetrahydro-6-oxo-1H-azepino[5,4,3-cd]indol.

Zu einer Lösung von 1,5 g 3-Trifluoracetamidomethyl-5-hydroxymethyl-3,4,5,6-tetrahydro-6-oxo-1H-azepino[5,4,3-cd]indol (Herstellung siehe Beispiel 11C) in 25 ml Methanol wurden 0,1 g p-Toluolsulfonsäure gegeben und die Lösung wurde 2 Stunden am Rückfluß erhitzt. Dann wurde das Reaktionsgemisch eingeengt und der Rückstand wurde mit gesättigter wäßriger Kaliumcarbonatlösung bis zur Erreichung eines pH-Wertes von 11 versetzt und 4 Stunden bei Raumtemperatur gerührt. Durch die Behandlung mit der wäßrigen gesättigten Kaliumcarbonatlösung wurde die Trifluoracetylschutzgruppe abgespalten und die p-Toluolsulfonsäure neutralisiert. Zur Aufarbeitung wurde das Reaktionsgemisch anschließend zur Trockene eingedampft, der Rückstand in Methanol aufgenommen und von unlöslichen Bestandteilen abfiltriert und der Filterrückstand noch zweimal mit je 30 ml Methanol gewaschen. Die vereinigten Methanolphasen wurden eingedampft und der Rückstand wurde durch Chromatographie unter mäßig erhöhtem Druck (= Niederdruckchromatographie bei 3 bis 6 bar) an Kieselgel unter Verwendung von Methanol/25-%ige wäßrige Ammoniaklösung 97:3 als Elutionsmittel gereinigt. Die nach Einengen des Eluates verbleibende Titelverbindung wurde aus Ethanol/Ether 9:1 kristallisiert. Es wurden 0,5 g 3-Aminomethyl-5-methoxy-methyl-3,4,5,6-tetrahydro-6-oxo-1H-azepino[5,4,3-cd]indol mit einem Schmelzpunkt von 160 bis 163 °C erhalten.

### Beispiel 13:

### 3-Aminomethyl-1-(3-phenylpropyl)-3,4,5,6-tetrahydro-6-oxo-1H-azepino[5,4,3-cd]indol

2 g 3-Trifluoracetamidomethyl-3,4,5,6-tetrahydro-6-oxo-1H-azepino[5,4,3-cd]indol (Herstellung siehe Beispiel 11A) wurden in 40 ml trockenem Dimethylformamid gelöst. Zu der Lösung wurden 0,8 g Natriumhydrid gegeben und das Reaktionsgemisch wurde 1 Stunde unter Stickstoffatmosphäre gerührt. Anschließend wurden 3,9 ml 3-Phenylpropylbromid hinzugetropft und das Reaktionsgemisch wurde 6 Stunden am Rückfluß erhitzt. Sodann wurde das Reaktionsgemisch unter vermindertem Druck (Wasserstrahlpumpenvakuum) eingedampft und der ölige Rückstand wurde in einer Mischung aus 40 ml Essigsäurethylester und 40 ml einer 5 %igen wäßrigen Zitronensäurelösung gelöst. Die organische Phase wurde abgetrennt und die wäßrige Phase wurde noch zweimal mit je 30 ml Essigsäureethylester extrahiert. Die vereinigten organischen Extrakte wurden über Natriumsulfat getrocknet, filtriert und eingeengt. Das erhaltene ölige rohe 3-Trifluoracetamidomethyl-1-(3-phenylpropyl)-3,4,5,6-tetrahydro-6-oxo-1H-azepino[5,4,3-cd]indol wurde durch Chromatographie unter mäßig erhöhtem Druck (Niederdruckchromatographie) an Kieselgel unter Verwendung von Cyclohexan/Essigsäureethylester 1:1 als Elutionsmittel gereinigt. Die das gesäuberte Produkt enthaltenden Fraktionen wurden zusammengefaßt und eingeengt. Zur Abspaltung der Trifluoracetylschutzgruppe wurde das erhaltene Produkt in 40 ml Methanol gelöst und die Lösung wurde mit 40 ml gesättigter wäßriger Kaliumcarbonatlösung versetzt und 8 Stunden bei Raumtemperatur gerührt. Anschließend wurde das Reaktionsgemisch nach der in Beispiel 11E beschriebenen Methode aufgearbeitet. Die erhaltene ölige Titelbase wurde in Isopropanol gelöst und die Lösung mit etherischer Salzsäurelösung versetzt. Es wurden 1,0 g 3-Aminomethyl-1-(3-phenylpropyl)-3,4,5,6-tetrahydro-6-oxo-1H-azepino[5,4,3-cd]indol-hydrochlorid mit einem Schmelzpunkt von 118 °C erhalten.

### Beispiel 14:

### 3-(Piperidin-1-ylmethyl)-3,4,5,6-tetrahydro-6-oxo-1H-azepino[5,4,3-cd]indol

4 g 3-Aminomethyl-3,4,5,6-tetrahydro-6-oxo-1H-azepino[5,4,3-cd]indol-hydrat (Herstellung siehe Beispiel 1C) wurden in 40 ml Dimethylformamid gelöst. Zu der Lösung wurden 2,6 ml Triethylamin hinzugegeben und anschließend bei Raumtemperatur unter Rühren 2,4 ml 1,5-Dibrompentan zugesetzt. Das Reaktionsgemisch wurde für 3,5 Stunden auf 50 °C erhitzt. Zur Aufarbeitung wurde das Reaktionsgemisch unter vermindertem Druck (Wasserstrahlpumpenvakuum) eingedampft und der Rückstand wurde in einem Gemisch aus Dichlormethan/Methanol 9:1 aufgenommen. Dann wurde gesättigte wäßrige Natriumcarbonatlösung bis zur Erreichung von pH 11 zugesetzt. Die organische Phase wurde abgetrennt und die wäßrige Phase noch zweimal mit je 50 ml des Dichlormethan/Methanol-Gemisches gewaschen. Die vereinigten organischen Phasen wurden über Natriumsulfat getrocknet, filtriert und eingedampft. Der Rückstand wurde in Ethanol gelöst und die Lösung wurde mit Diethylether bis zum Auftreten einer leichten Trübung versetzt. Das entstandene Kristallisat wurde abfiltriert und getrocknet. Es wurden 3,5 g kristallines 3-(Piperidin-1-ylmethyl)-3,4,5,6-tetrahydro-6-oxo-1H-azepino[5,4,3-cd]indol mit einem Schmelzpunkt von 119 bis 122 °C erhalten.

### Beispiel 15:

### 3-(Dimethylaminomethylidenaminomethyl)-3,4,5,6-tetrahydro-6-oxo-1H-azepino[5,4,3-cd]indol

2 g 3-Aminomethyl-3,4,5,6-tetrahydro-6-oxo-1H-azepino[5,4,3-cd]indol-hydrat (Herstellung siehe Beispiel 1C) wurden bei Raumtemperatur in 20 ml Dimethylformamid gelöst. Zu der Lösung wurden unter Rühren 2,6 ml Dimethylformamid-dimethylacetal zugesetzt. Das Reaktionsgemisch wurde 4 Stunden bei Raumtemperatur gerührt. Anschließend wurde das Reaktionsgemisch unter vermindertem Druck (Wasserstrahlpumpenvakuum) eingeengt und der ölige Rückstand wurde in 50 ml Essigsäureethylester gelöst. Die Lösung wurde bis zur Bildung einer leichten Trübung mit Diethylether versetzt. Es kristallisierten 2,2 g der rohen Titelbase aus.

2,2 g der vorstehend erhaltenen rohen Titelbase wurden in Methanol gelöst und die Lösung wurde durch Zusatz von etherischer Salzsäurelösung auf einen pH-Wert von etwa 1 eingestellt. Dann wurde die Lösung eingedampft und das zurückbleibende Hydrochlorid der Titelverbindung wurde aus Butylacetat unter Zusatz von Isopropanol kristallisiert. Nach Trocknung des Kristallisates wurden 1,3 g 3-(Dimethylaminomethylidenaminomethyl)-3,4,5,6-tetrahydro-6-oxo-1H-azepino[5,4,3-cd]indol-hydrochlorid mit einem Schmelzpunkt von 178 bis 179 °C erhalten.

### Beispiel 16:

### 3-(Dimethylaminomethylidenaminomethyl)-3,4,5,6-tetrahydro-6-oxo-1H-azepino[5,4,3-cd]indol.

1 g 3-Aminomethyl-3,4,5,6-tetrahydro-6-oxo-1H-azepino[5,4,3-cd]indol-hydrat (Herstellung siehe Beispiel 1C) wurden bei Raumtemperatur in 10 ml Dimethylformamid gelöst. Zu dieser Lösung wurden 1 g N-Chlormethylen-N,N-dimethyliminiumchlorid hinzugefügt. Anschließend wurde das Reaktionsgemisch 2 Stunden bei Raumtemperatur gerührt. Zur Aufarbeitung wurde das Reaktionsgemisch unter vermindertem Druck (Wasserstrahlpumpenvakuum) eingedampft und der Rückstand in einem Gemisch aus gleichen Teilen Wasser und Essigsäureethylester aufgenommen. Durch Zugabe von gesättigter Natriumhydrogencarbonatlösung wurde die wäßrige Phase auf einen pH-Wert von 8,5 gebracht, dann wurde die organische Phase abgetrennt, und die wäßrige Phase noch dreimal mit je 50 ml Essigsäureethylester extrahiert. Die vereinigten organischen Extrakte wurden bis auf 25 ml eingeengt. Die so erhaltene Lösung der Titelverbindung wurde wie in Beispiel 15 beschrieben weiter aufgearbeitet. Es wurden 0,5 g 3-(Dimethylaminomethylidenaminomethyl)-3,4,5,6-tetrahydro-6-oxo-1H-azepino[5,4,3-cd]indol-hydrochlorid mit einem Schmelzpunkt von 178 bis 179 °C erhalten.

### Beispiel 17:

### 4-(4-Phenylpiperazin-1-ylmethyl)-3,4,5,6-tetrahydro-6-oxo-1H-azepino[5,4,3-cd]indol.

2,2 g 4-(p-Toluolsulfonyloxymethyl)-3,4,5,6-tetrahydro-6-oxo-1H-azepino[5,4,3-cd]indol (Herstellung siehe Beispiel 7E) wurden mit 9 ml 1-Phenylpiperazin gemischt und das Gemisch wurde 4 Stunden bei einer Temperatur von 80 °C gerührt. Bei dem anschließenden Abkühlen kristallisierte die rohe Titelverbindung aus. Sie wurde abfiltriert und mit einem Gemisch aus gleichen Teilen Methanol und Wasser gewaschen. Nach dem Trocknen wurden 1,7 g 4-(4-Phenylpiperazin-1-ylmethyl)-3,4,5,6-tetrahydro-6-oxo-1H-azepino[5,4,3-cd]indol mit einem Schmelzpunkt von 184 bis 185 °C erhalten.

### Beispiel 18:

### 1-Benzyl-3-benzylaminomethyl-5-methyl-3,4,5,6-tetrahydro-6-oxo-1H-azepino[5,4,3-cd]indol.

A) 4 g öliges 1-Benzyl-3-aminomethyl-3,4,5,6-tetrahydro-6-oxo-1H-azepino[5,4,3-cd]indol (Herstellung siehe Beispiel 10) wurden nach der in Beispiel 2 beschriebenen Methode mit Benzaldehyd umgesetzt und die erhaltene Schiff'sche Base wurde nach der in Beispiel 2 beschriebenen Methode reduziert. Das Reaktionsgemisch wurde wie in Beispiel 2 beschrieben aufgearbeitet. Es wurden 4,3 g 1-Benzyl-3-benzylaminomethyl-3,4,5,6-tetrahydro-6-oxo-1H-azepino[5,4,3-cd]indol erhalten.
B) 4,3 g des vorstehend erhaltenen Produktes wurden nach der in Beispiel 11 A beschriebenen Methode mit Trifluoressigsäureanhydrid umgesetzt und das Reaktionsgemisch wurde wie in Beispiel 11 A beschrieben aufgearbeitet. Es wurden 4,1 g 1-Benzyl-3-[N-(benzyl)-trifluoracetamidomethyl]-3,4,5,6-tetrahydro-6-oxo-1H-azepino[5,4,3-cd]indol als schaumiges Produkt erhalten.
C) 4,1 g des vorstehend erhaltenen Produktes wurden in 100 ml absolutem Tetrahydrofuran gelöst. Die Lösung wurde bei einer Temperatur von - 20 °C unter Stickstoffatmosphäre tropfenweise mit einer Lösung von 1,15 g Kalium-tert.-butylat in 10 ml Tetrahydrofuran versetzt. Das Reaktionsgemisch wurde 1 Stunde bei - 20 °C gerührt und anschließend wurde bei dieser Temperatur eine Lösung von 0,85 ml Dimethylsulfat in 5 ml Tetrahydrofuran tropfenweise zugegeben. Das Reaktionsgemisch wurde eine weitere Stunde bei - 20 °C unter Stickstoffatmosphäre gerührt. Anschließend wurde die Kühlung entfernt. Nach Erreichen der Raumtemperatur wurde das Reaktionsgemisch bis auf ca. 20 ml unter vermindertem Druck (Wasserstrahlpumpenvakuum) eingeengt und anschließend mit 100 ml Wasser und 50 ml Dichlormethan verdünnt. Dem zweiphasigen Gemisch wurde dann gesättigte wäßrige Kaliumcarbonatlösung bis zum Erreichen eines pH-Wertes von ungefähr 11 zugesetzt. Das Gemisch wurde anschließend 4 Stunden lang heftig gerührt,so daß eine gute Durchmischung der beiden Phasen stattfand, wobei auch die Trifluoracetylschutzgruppe hydrolytisch abgespalten wurde. Anschließend wurde die organische Phase abgetrennt, und die wäßrige Phase wurde noch zweimal mit je 50 ml Dichlormethan extrahiert. Die vereinigten organischen Phasen wurden über Natriumsulfat getrocknet, filtriert und eingedampft. Das zurückbleibende Rohprodukt wurde durch Chromatographie an Kieselgel unter leicht erhöhtem Druck (Flash-Chromatographie) unter Verwendung von Essigsäureethylester/Cyclohexan 7:3 als Elutionsmittel gereinigt. Es wurden 2,1 g 1-Benzyl-3-benzylaminomethyl-5-methyl-3,4,5,6-tetrahydro-6-oxo-1H-azepino[5,4,3-cd]indol als schaumiges Produkt erhalten.

### Beispiel 19:

### 3-[N-(4-Hydroxybenzyl)-aminomethyl]-3,4,5,6-tetrahydro-6-oxo-1H-azepino[5,4,3-cd]indol.

Zu einer Mischung aus 1,5 g 3-[N-(4-Methoxybenzyl)-aminomethyl]-3,4,5,6-tetrahydro-6-oxo-1H-azepino[5,4,3-cd]indol (Herstellung siehe Beispiel 21) und 2,5 g 1,4-Diazabicyclo[2,2,2]octan (=DABCO) wurden 2 ml Trimethylsilyljodid gegeben. Das Reaktionsgemisch wurde unter Stickstoffatmosphäre für 24 Stunden auf 150 °C erhitzt. Nach Abkühlen auf Raumtemperatur wurde das Reaktionsgemisch mit 25 ml Methanol verdünnt und mit 20-%iger wäßriger Salzsäure angesäuert. Dann wurde das Gemisch 2 Stunden bei Raumtemperatur stark gerührt. Zur Aufarbeitung wurde das Reaktionsgemisch mit 30 ml Wasser verdünnt, die wäßrige Phase abgetrennt und die organische Phase noch zweimal mit je 20 ml Wasser gewaschen. Die vereinigten wäßrigen Phasen wurden mit verdünnter Natronlauge neutralisiert und anschließend unter vermindertem Druck (Wasserstrahlpumpenvakuum) eingedampft. Das als öliger Rückstand verbleibende Rohprodukt wurde durch Chromatographie an Kieselgel unter leicht erhöhtem Druck (Flash-Chromatographie) unter Verwendung von Methanol/25-%ige wäßrige Ammoniaklösung 98:2 als Elutionsmittel gereinigt. Es wurden 0,6 g 3-[N-(4-Hydroxybenzyl)-aminomethyl]-3,4,5,6-tetrahydro-6-oxo-1H-azepino[5,4,3-cd]indol als Öl erhalten.

0,6 g der vorstehend erhaltenen Titelbase wurden in 20 ml Methanol gelöst und zu der Lösung wurden 0,27 g Weinsäure gegeben. Nach Eindampfen dieser Mischung wurden 0,87g amorphes 3-[N-(4-Hydroxybenzyl)-aminomethyl]-3,4,5,6-tetrahydro-6-oxo-1H-azepino[5,4,3-cd]indol-hydrogentartrat erhalten.

### Beispiel 20:

### 4-Aminomethyl-3,4,5,6-tetrahydro-6-oxo-1H-azepino[5,4,3-cd]indol.

A) 2,5 g 4-(p-Toluolsulfonyloxymethyl)-3,4,5,6-tetrahydro-6-oxo-1H-azepino[5,4,3-cd]indol wurden in 40 ml Methanol gelöst. Zu der Lösung wurden 1,4 g Kaliumphthalimid gegeben. Das Reaktionsgemisch wurde 6 Stunden am Rückfluß erhitzt. Zur Aufarbeitung wurde das Reaktionsgemisch anschließend unter vermindertem Druck (Wasserstrahlpumpenvakuum) zur Trockene eingedampft. Der verbleibende feste Rückstand wurde in einem Gemisch aus Dichlormethan und Wasser 1:1 gelöst. Die organische Phase wurde abgetrennt und die wäßrige Phase noch dreimal mit je 50 ml Dichlormethan gewaschen. Die vereinigten organischen Extrakte wurden über Natriumsulfat getrocknet, filtriert und eingedampft. Es wurden 1,9 g 4-(N-Phthalimidomethyl)-3,4,5,6-tetrahydro-6-oxo-1H-azepino[5,4,3-cd]indol erhalten, welches sofort in der nächsten Reaktionsstufe weiterverarbeitet wurde.
B) 1,9 g des vorstehend erhaltenen Produktes wurden in 50 ml Ethanol gelöst. Zu der Lösung wurde 1 ml Hydrazin-Hydrat gegeben. Das Reaktionsgemisch wurde 4 Stunden am Rückfluß erhitzt. Zur Aufarbeitung wurde auf Raumtemperatur abgekühlt und das gebildete Phthalhydrazid abfiltriert und zweimal mit je 20 ml Ethanol gewaschen. Das Filtrat und die Waschlösungen wurden vereinigt und eingedampft. Die verbleibende rohe Titelverbindung wurde durch Chromatographie unter mäßig erhöhtem Druck (=Niederdruckchromatographie = LPLC bei 3 bis 6 bar) an Kieselgel unter Verwendung von Methanol/wäßrige Ammoniaklösung 98:2 als Elutionsmittel gereinigt. Die so erhaltene Titelbase wurde nach der in Beispiel 7G beschriebenen Methode in ihr Hydrochlorid überführt. Es wurden 0,7 g 4-Aminomethyl-3,4,5,6-tetrahydro-6-oxo-1H-azepino[5,4,3-cd]indol-hydrochlorid mit einem Schmelzpunkt von 256 bis 260 °C erhalten.

Nach den in den vorstehenden Beispielen beschriebenen Verfahren können auch die in der nachfolgenden Tabelle 1 aufgeführten Verbindungen der Formel I hergestellt werden.

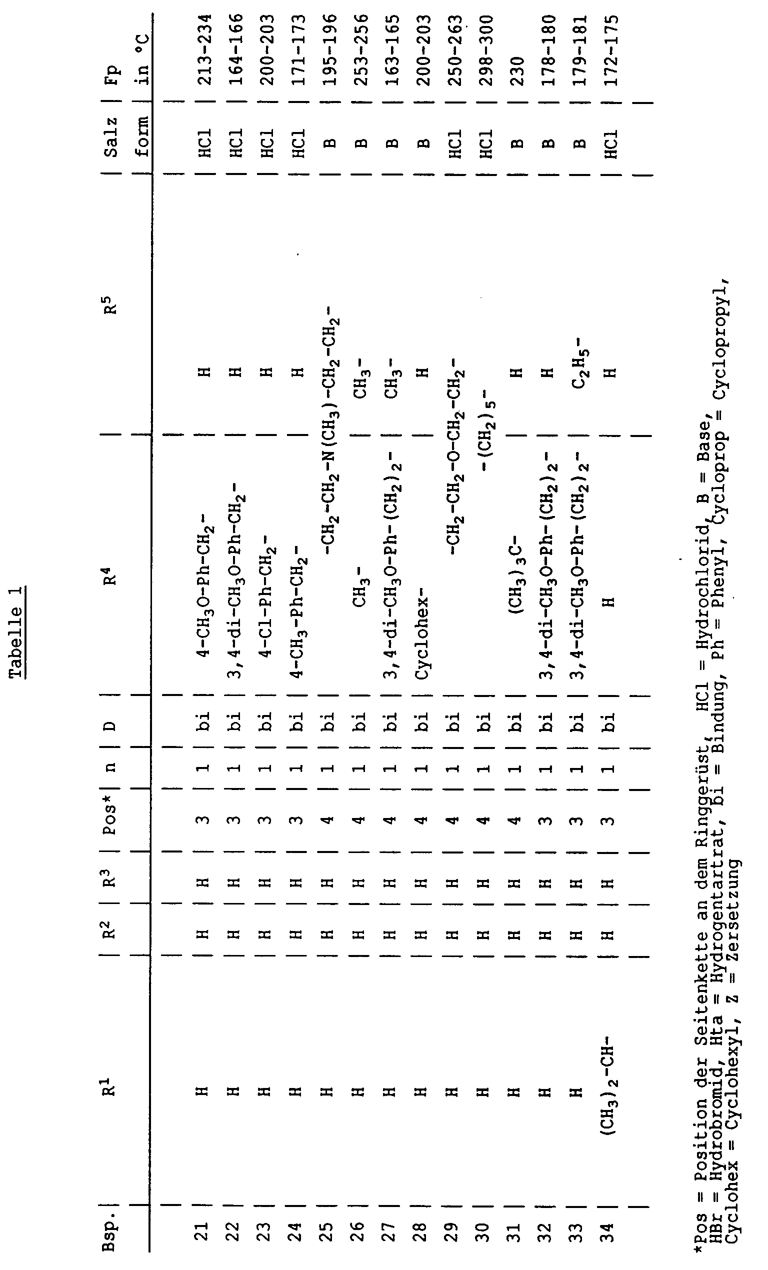

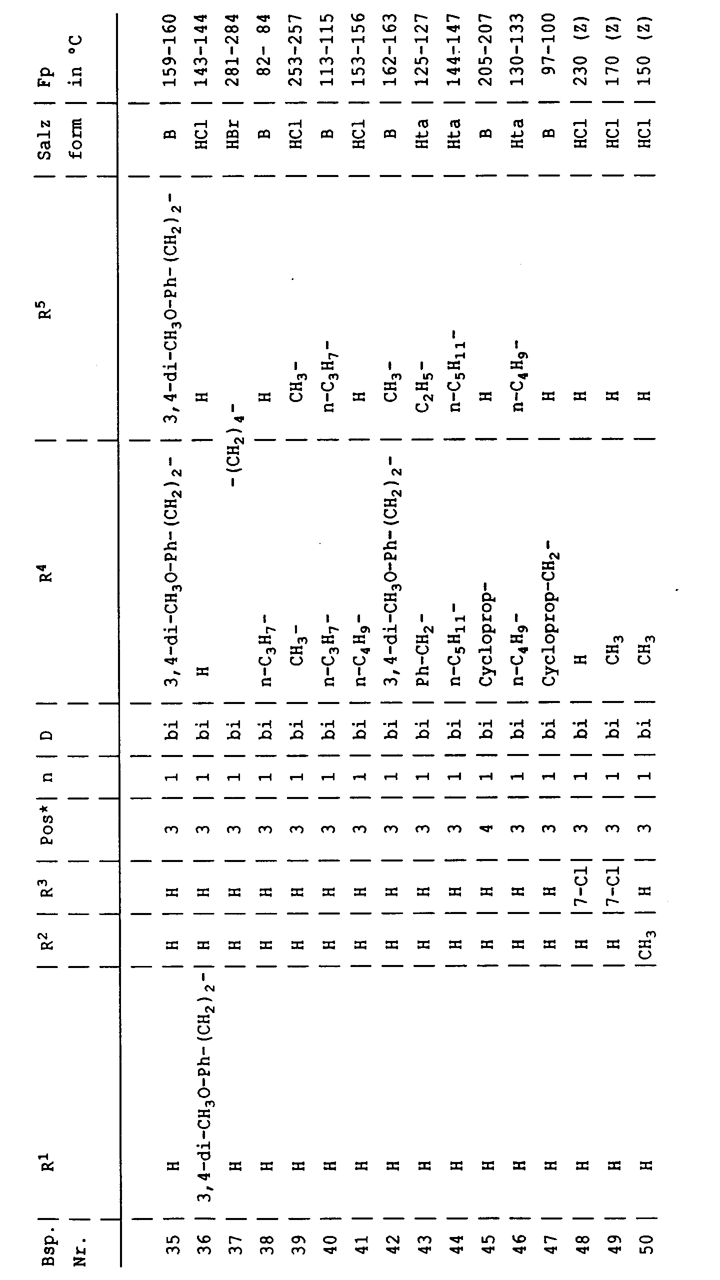

### Beispiel I:

### 3-Aminomethyl-3,4,5,6-tetrahydro-6-oxo-1H-azepino[5,4,3-cd]indol enthaltende Tabletten.

Man stellte Tabletten in folgender Zusammensetzung pro Tablette her:

| | |
|---|---|
| 3-Aminomethyl-3,4,5,6-tetrahydro-6-oxo-1H-azepino[5,4,3-cd]indol-hydrochlorid | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker wurden mit der 10%-igen Gelatine-Lösung eingedickt. Die Paste wurde zerkleinert und das entstandene Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

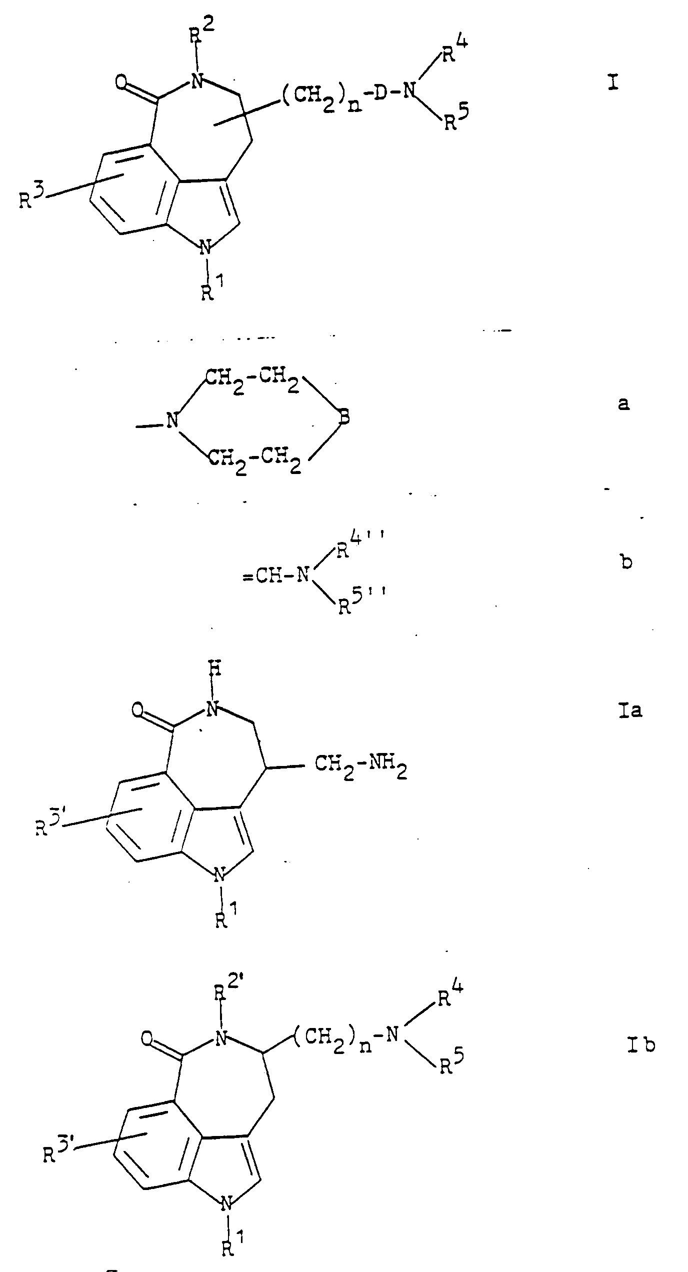

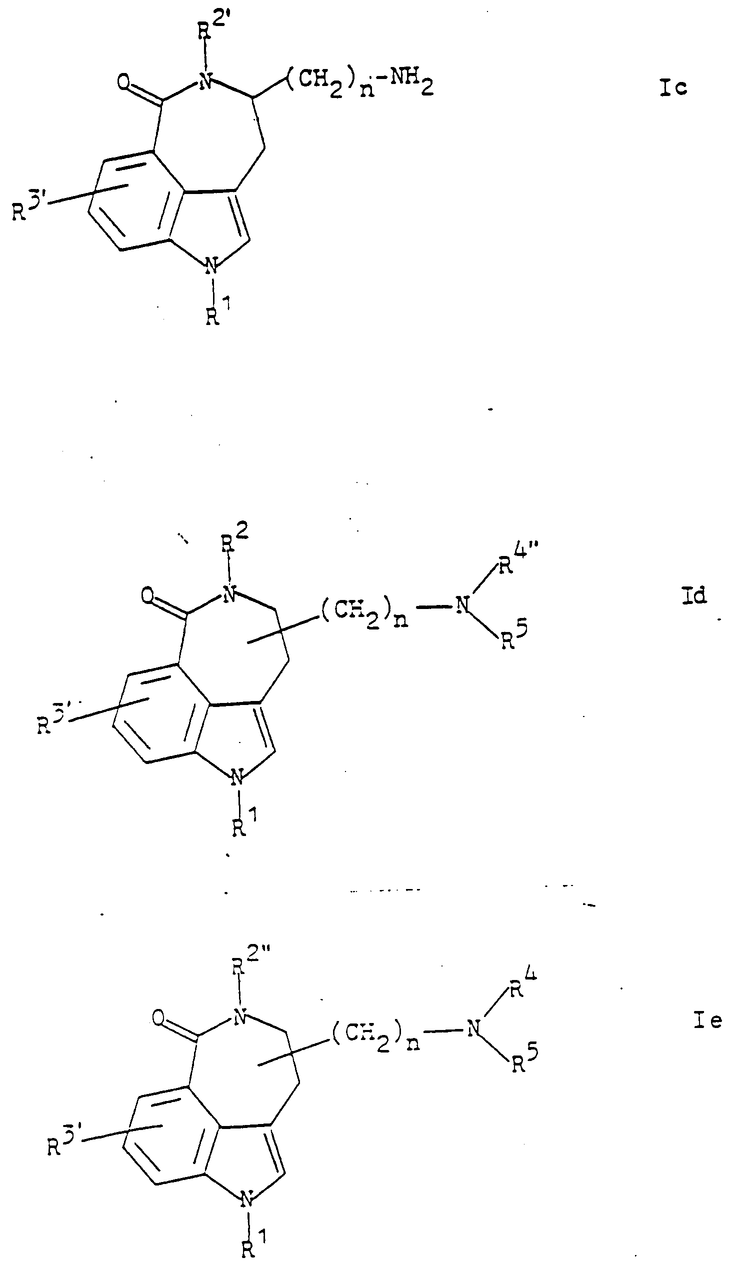

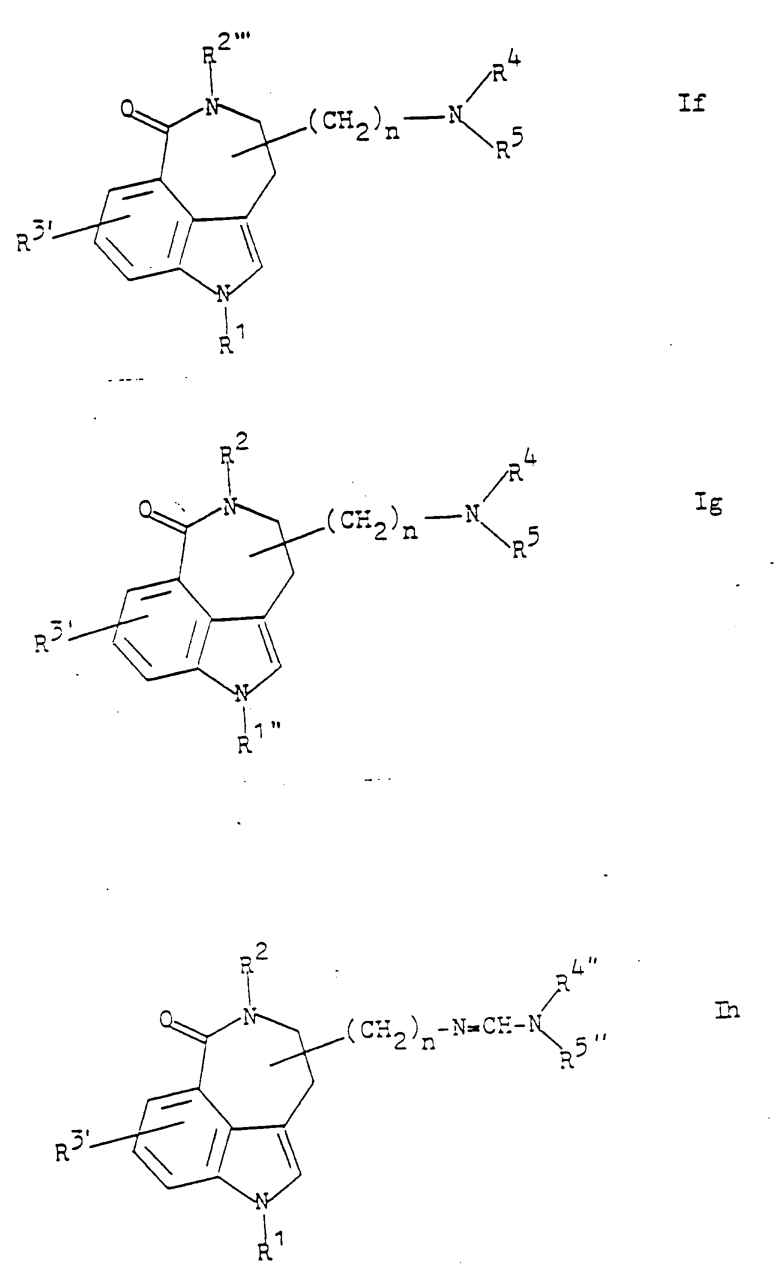

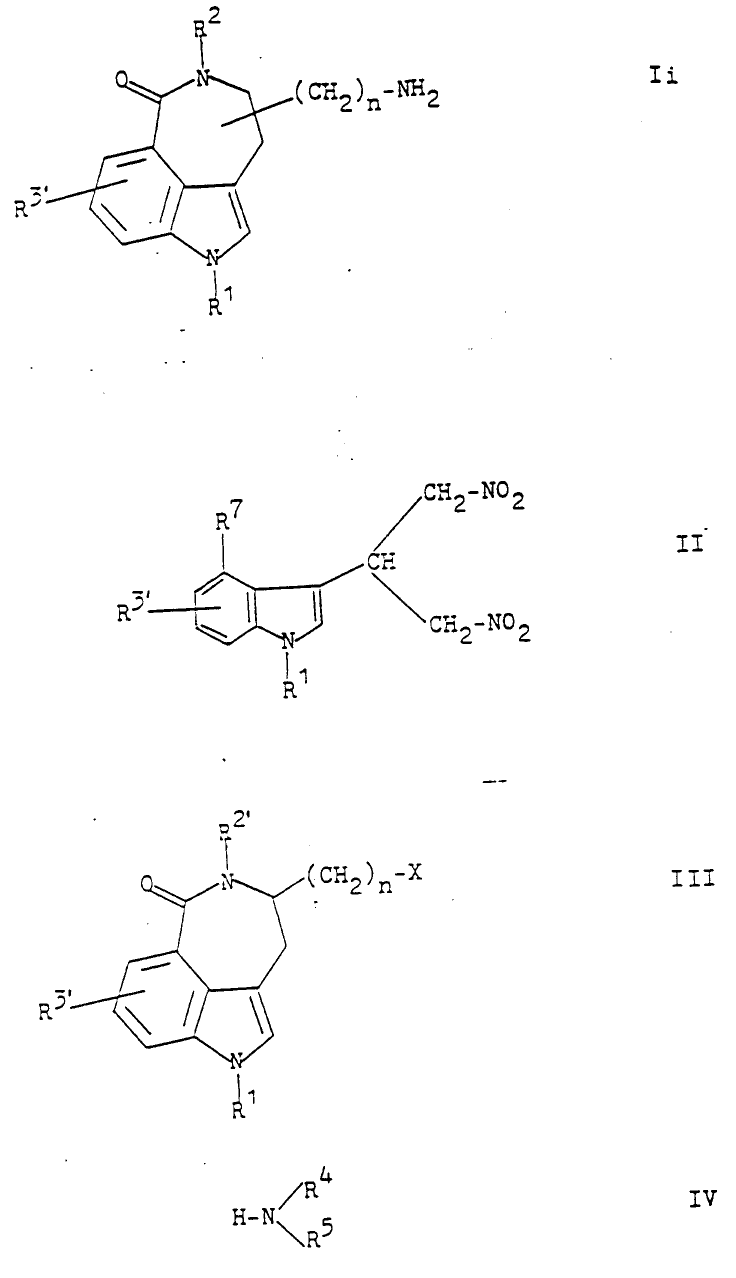

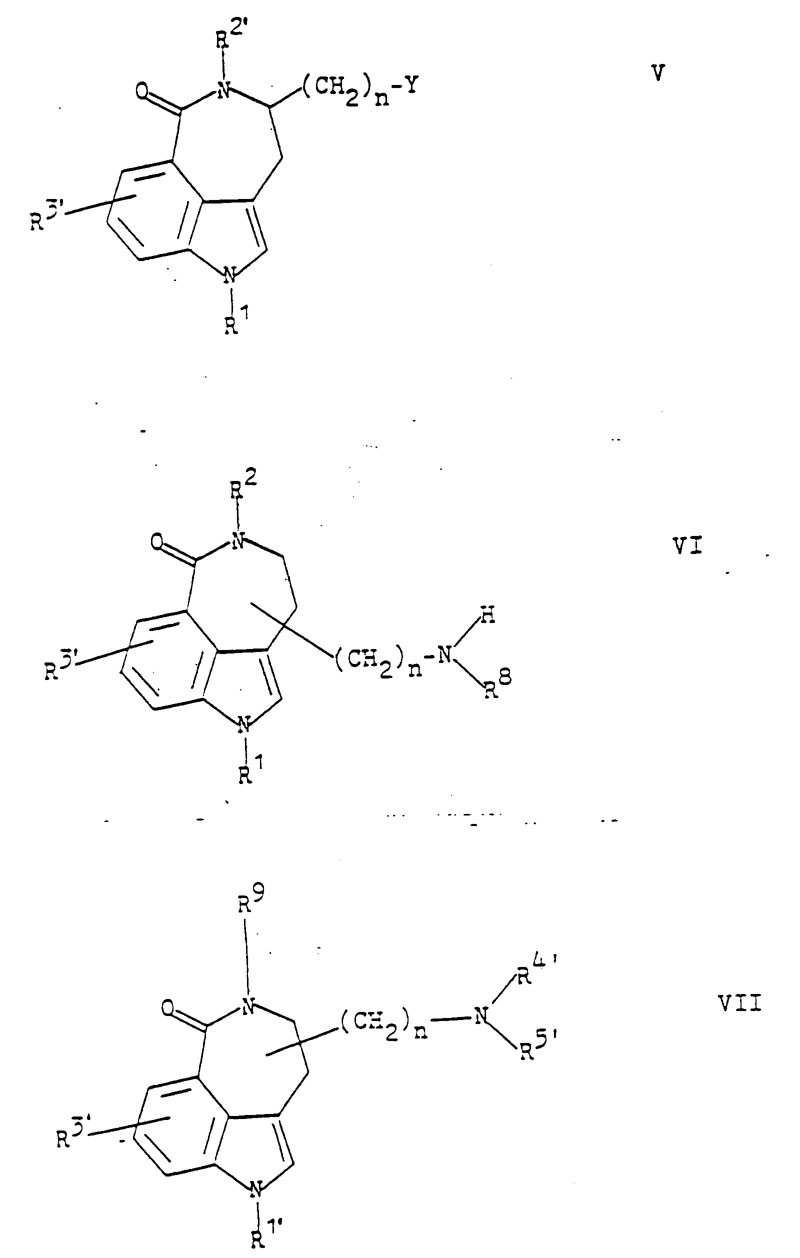

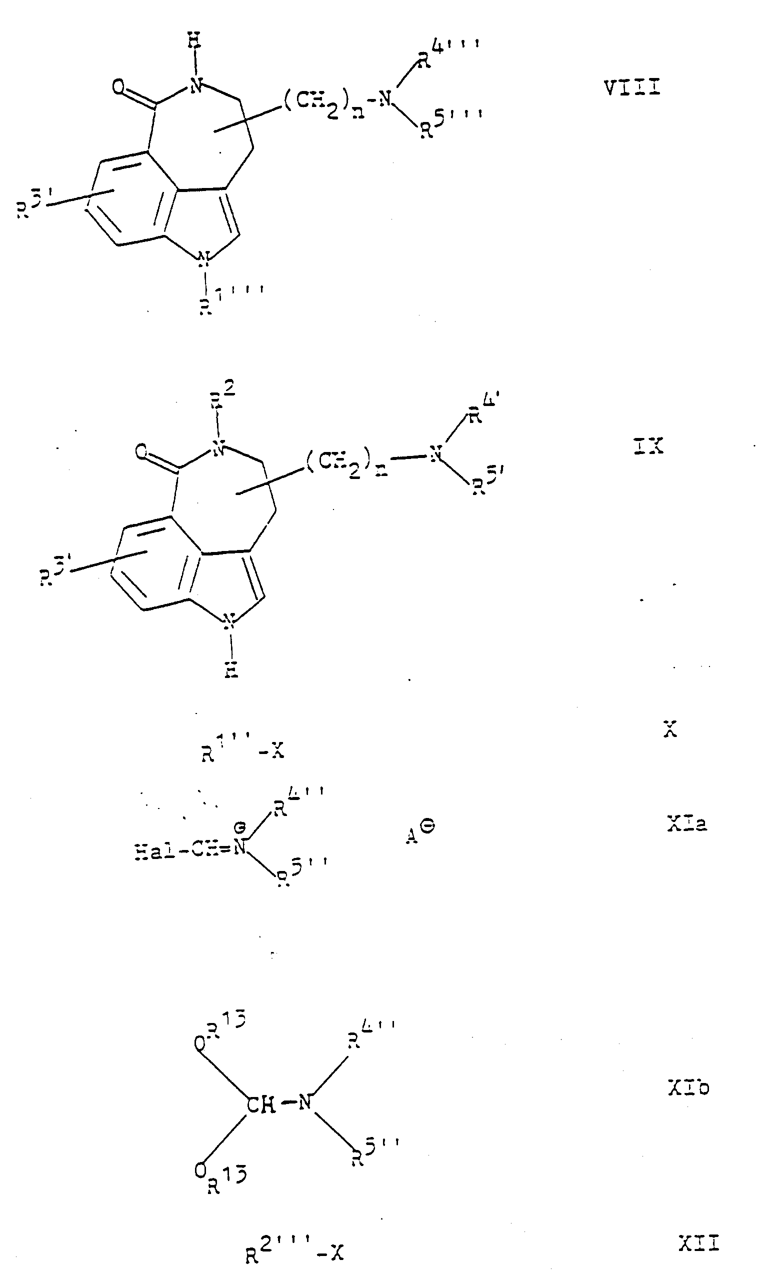

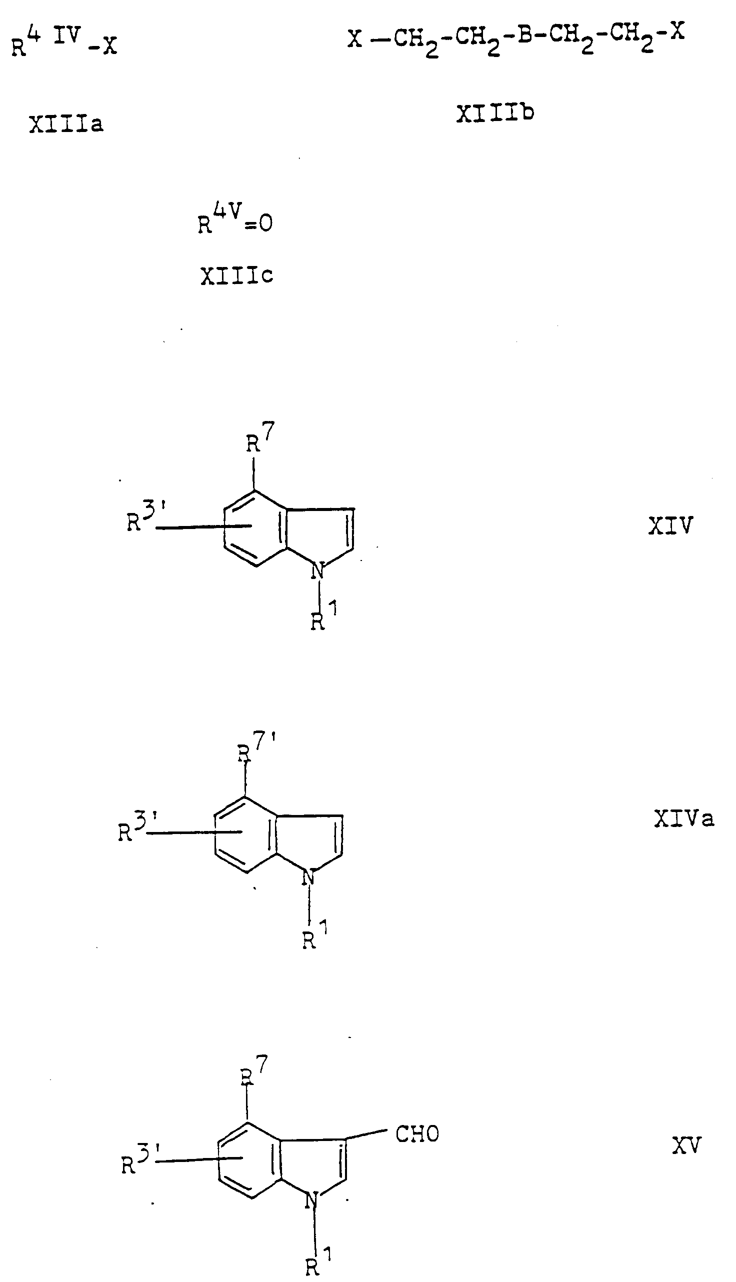

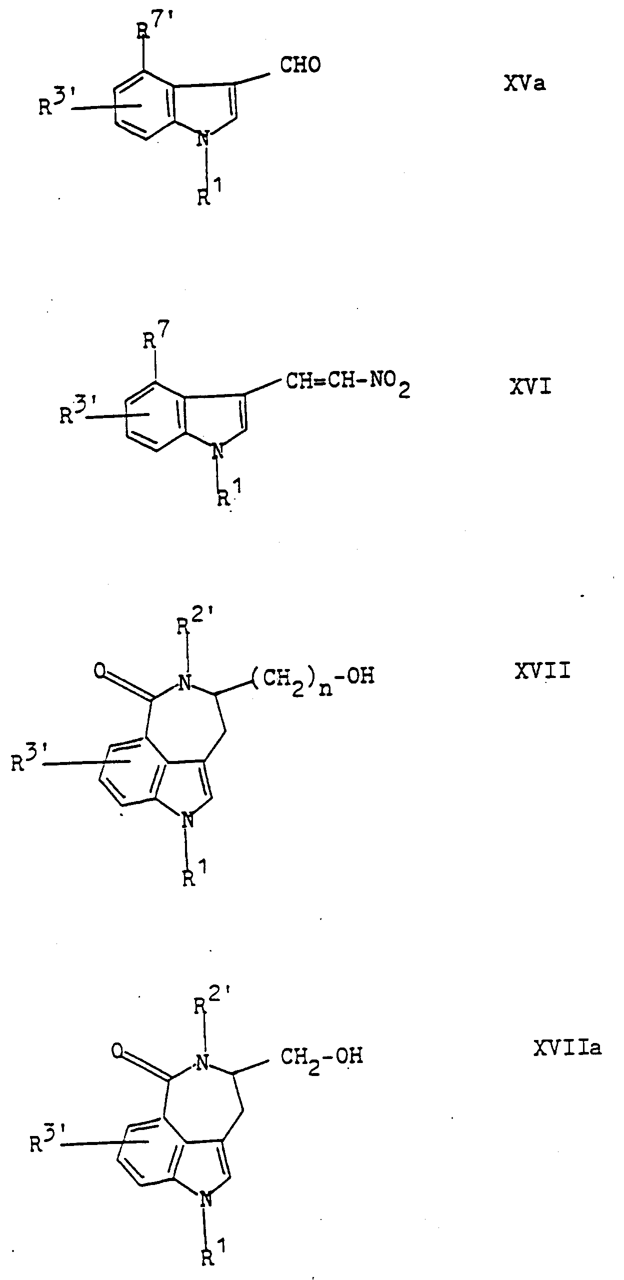

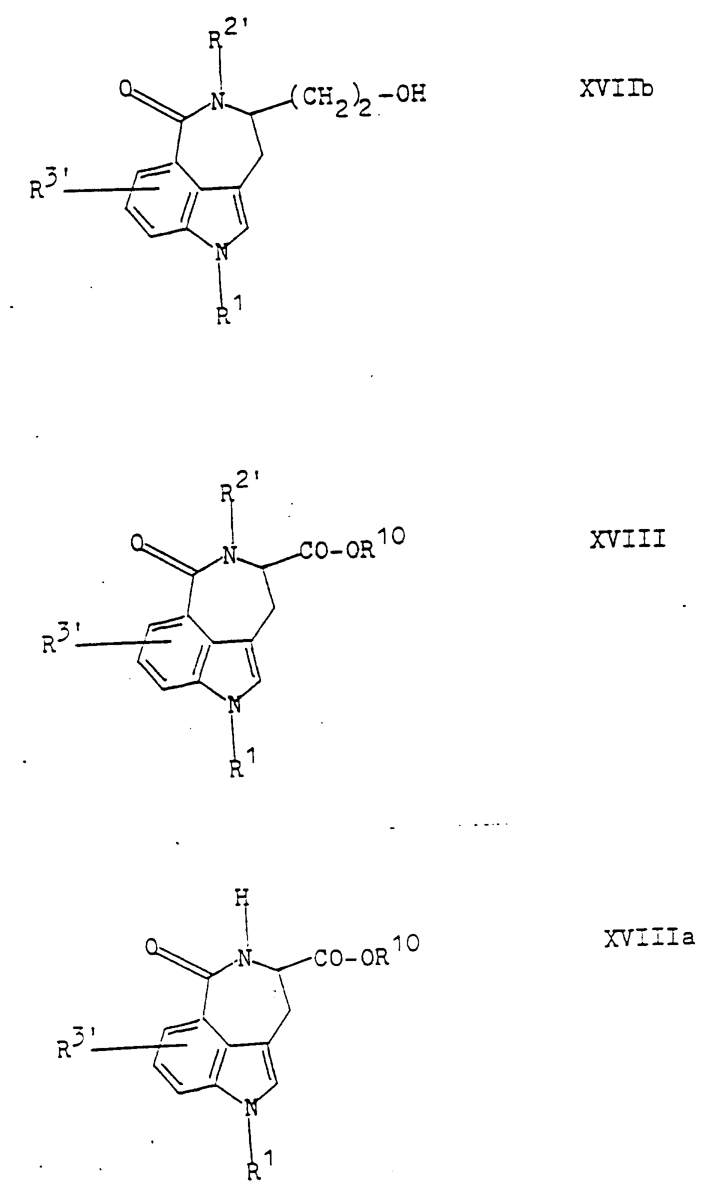

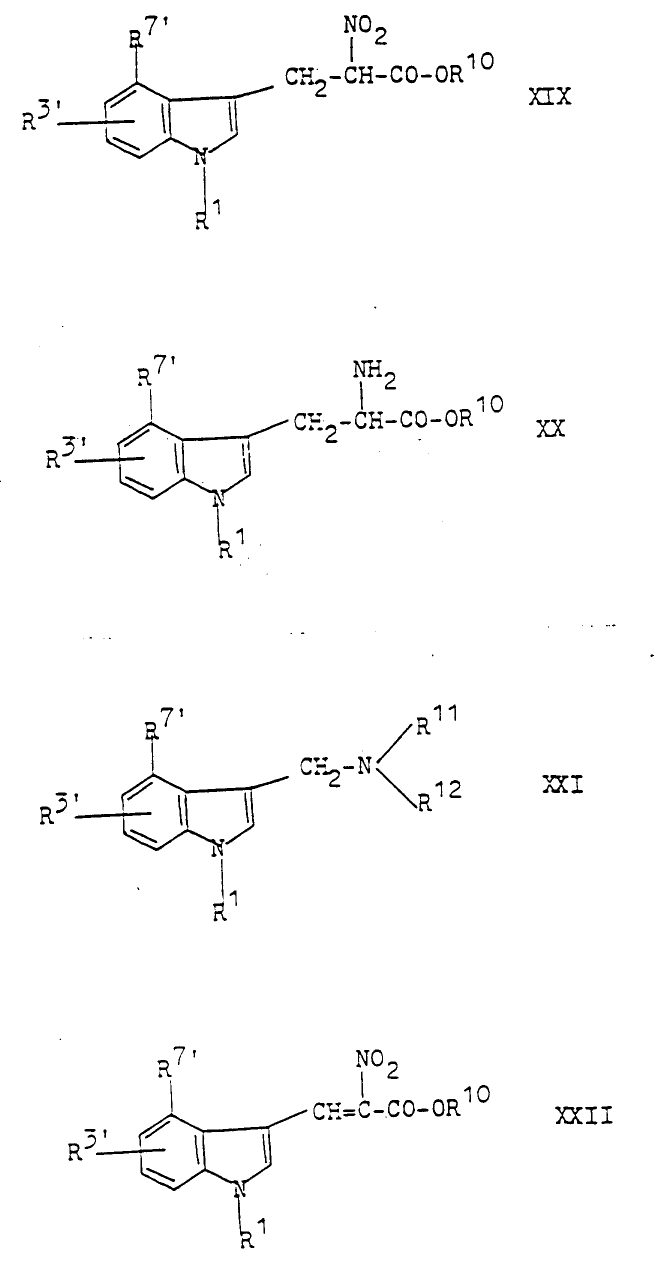

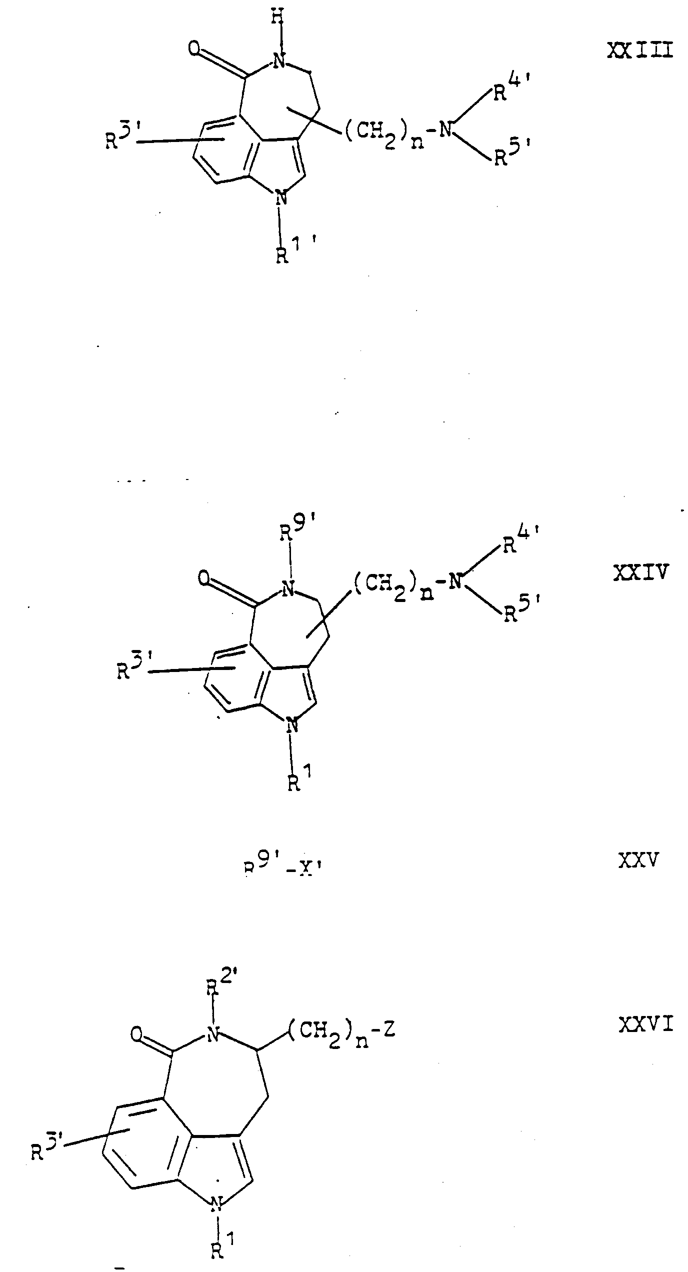

## Patentansprüche

1. Verbindungen der allgemeinen Formel I 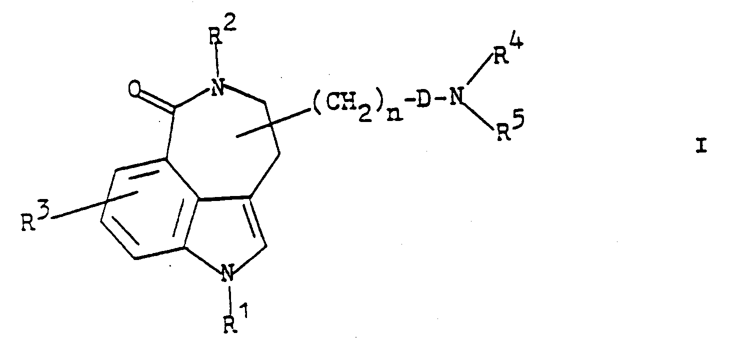 worin
R¹ für Wasserstoff, für eine niedere Alkyl- oder Cycloalkylalkylgruppe oder für eine Phenylniederalkylgruppe, welche gegebenenfalls im Phenylring durch niederes Alkoxy, Hydroxy, Halogen oder niederes Alkyl mono- oder disubstituiert sein kann, steht,
R² Wasserstoff oder eine gegebenenfalls in α-Stellung zu dem Stickstoffatom durch niederes Alkoxy substituierte niedere Alkylgruppe bedeutet,
R³ Wasserstoff, niederes Alkyl, niederes Alkoxy, Halogen oder, falls die Substituenten R¹, R², R⁴ und/oder R⁵ keine niederen Alkoxygruppen enthalten, auch Hydroxy bedeutet,
n für 1 oder, falls die -(CH₂)ₙ-Kette in 4-Stellung des Ringgerüstes angeordnet ist, auch für 2 steht,
R⁴ Wasserstoff, Alkyl mit 1 bis 5 Kohlenstoffatomen, Cycloalkyl mit 3 bis 6 Kohlenstoffatomen, Cycloalkylalkyl mit 4 bis 9 Kohlenstoffatomen oder eine gegebenenfalls im Phenylring durch niederes Alkyl, niederes Alkoxy, Hydroxy oder Halogen mono- oder disubstituierte Phenylniederalkylgruppe bedeutet, und
R⁵ Wasserstoff, Alkyl mit 1 bis 5 Kohlenstoffatomen, Cycloalkyl mit 3 bis 6 Kohlenstoffatomen, Cycloalkylalkyl mit 4 bis 9 Kohlenstoffatomen oder eine gegebenenfalls im Phenylring durch niederes Alkyl, niederes Alkoxy, Hydroxy oder Halogen mono- oder disubstituierte Phenylniederalkylgruppe bedeutet, oder
R⁴ und R⁵ gemeinsam mit dem Stickstoffatom, an welches sie gebunden sind, einen Heterocyclus der allgemeinen Formel a 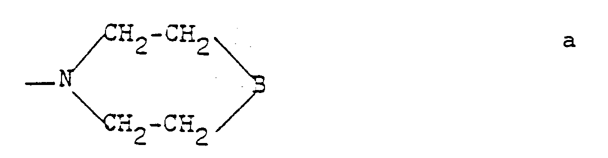 bilden, worin
B für eine Bindung, die Methylengruppe, Sauerstoff oder eine Iminogruppe -NR⁶-, worin R⁶ Wasserstoff, niederes Alkyl oder gegebenenfalls im Phenylring durch niederes Alkyl, niederes Alkoxy, Hydroxy oder Halogen substituiertes Phenyl oder Benzyl bedeutet, steht, und
D eine Bindung oder, falls R⁴ und R⁵ nicht Wasserstoff bedeuten, auch die -N=CH-Gruppe darstellt,
wobei sofern die Substituenten niedere Alkylgruppen darstellen, können diese 1 bis 4 Kohlenstoffatome enthalten, und deren physiologisch verträgliche Säureadditionssalze.

2. Verbindungen gemäß Anspruch 1, dadurch gekennzeichnet, daß R¹ Wasserstoff bedeutet.

3. Verbindungen gemäß Anspruch 1 oder 2, dadurch gekennzeichnet, daß R² Wasserstoff bedeutet.

4. Verbindungen gemäß Anspruch 1 bis 3, dadurch gekennzeichnet, daß R⁴ Wasserstoff, niederes Alkyl mit 1 bis 4 Kohlenstoffatomen oder gegebenenfalls substituiertes Benzyl und R⁵ Wasserstoff bedeuten.

5. Verbindungen gemäß Anspruch 1 bis 4, dadurch gekennzeichnet, daß D eine Bindung darstellt.

6. Verbindungen gemäß Anspruch 1 bis 5, dadurch gekennzeichnet, daß sie eine -CH₂-D-NR⁴R⁵ Gruppe in 3-Stellung des Ringgerüstes enthalten.

7. Arzneimittel enthaltend eine pharmakologisch wirksame Menge einer Verbindung gemäß Anspruch 1 und übliche pharmazeutische Hilfs- und/oder Trägerstoffe.

8. Verfahren zur Herstellung von Verbindungen der allgemeinen Formel I 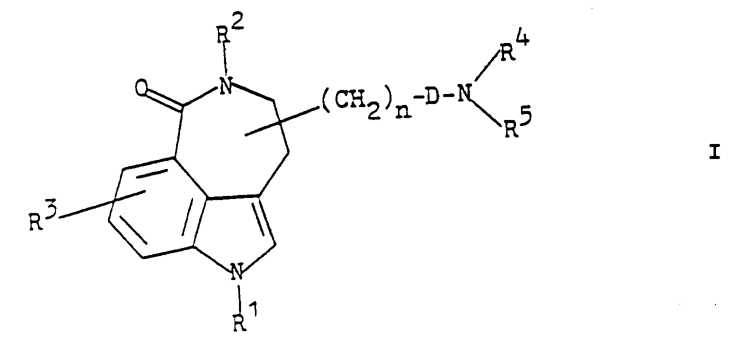 worin
R¹ für Wasserstoff, für eine niedere Alkyl- oder Cycloalkylalkylgruppe oder für eine Phenylniederalkylgruppe, welche gegebenenfalls im Phenylring durch niederes Alkoxy, Hydroxy, Halogen oder niederes Alkyl mono- oder disubstituiert sein kann, steht,
R² Wasserstoff oder eine gegebenenfalls in α-Stellung zu dem Stickstoffatom durch niederes Alkoxy substituierte niedere Alkylgruppe bedeutet,
R³ Wasserstoff, niederes Alkyl, niederes Alkoxy, Halogen oder, falls die Substituenten R¹, R², R⁴ und/oder R⁵ keine niederen Alkoxygruppen enthalten, auch Hydroxy bedeutet,
n für 1 oder, falls die -(CH₂)ₙ-Kette in 4-Stellung des Ringgerüstes angeordnet ist, auch für 2 steht,
R⁴ Wasserstoff, Alkyl mit 1 bis 5 Kohlenstoffatomen, Cycloalkyl mit 3 bis 6 Kohlenstoffatomen, Cycloalkylalkyl mit 4 bis 9 Kohlenstoffatomen oder eine gegebenenfalls im Phenylring durch niederes Alkyl, niederes Alkoxy, Hydroxy oder Halogen mono- oder disubstituierte Phenylniederalkylgruppe bedeutet, und
R⁵ Wasserstoff, Alkyl mit 1 bis 5 Kohlenstoffatomen, Cycloalkyl mit 3 bis 6 Kohlenstoffatomen, Cycloalkylalkyl mit 4 bis 9 Kohlenstoffatomen oder eine gegebenenfalls im Phenylring durch niederes Alkyl, niederes Alkoxy, Hydroxy oder Halogen mono- oder disubstituierte Phenylniederalkylgruppe bedeutet, oder
R⁴ und R⁵ gemeinsam mit dem Stickstoffatom, an welches sie gebunden sind, einen Heterocyclus der allgemeinen Formel a 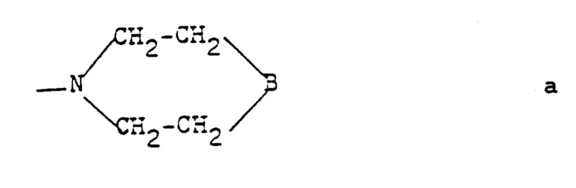 bilden, worin
B für eine Bindung, die Methylengruppe, Sauerstoff oder eine Iminogruppe -NR⁶-, worin R⁶ Wasserstoff, niederes Alkyl oder gegebenenfalls im Phenylring durch niederes Alkyl, niederes Alkoxy, Hydroxy oder Halogen substituiertes Phenyl oder Benzyl bedeutet, steht, und
D eine Bindung oder, falls R⁴ und R⁵ nicht Wasserstoff bedeuten, auch die -N=CH-Gruppe darstellt, wobei sofern die
Substituenten niedere Alkylgruppen darstellen, können diese 1 bis 4 Kohlenstoffatome enthalten, und deren physiologisch verträglichen Säureadditionssalzen, dadurch gekennzeichnet, daß man
a) zur Herstellung von Verbindungen der allgemeinen Formel Ia 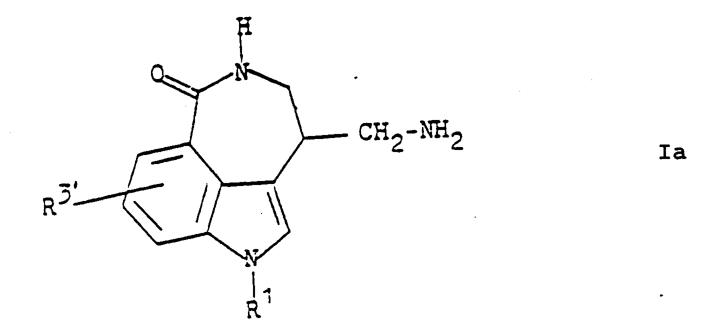 worin R¹ obige Bedeutung besitzt und R³' die für R³ angegebene Bedeutung mit Ausnahme von Hydroxy besitzt, Verbindungen der allgemeinen Formel II 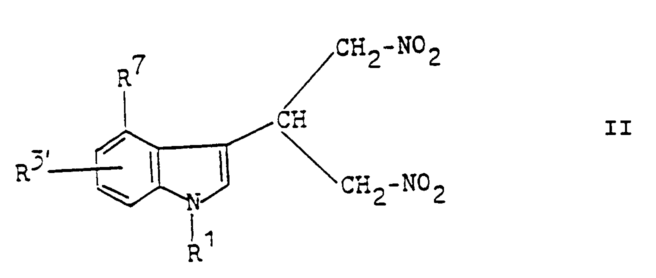 worin R¹ und R³' obige Bedeutung besitzen und R⁷ eine niedere Alkoxycarbonylgruppe oder die CN-Gruppe bedeutet, unter reduzierenden Bedingungen cyclisiert, oder
b) zur Herstellung von Verbindungen der allgemeinen Formel Ib 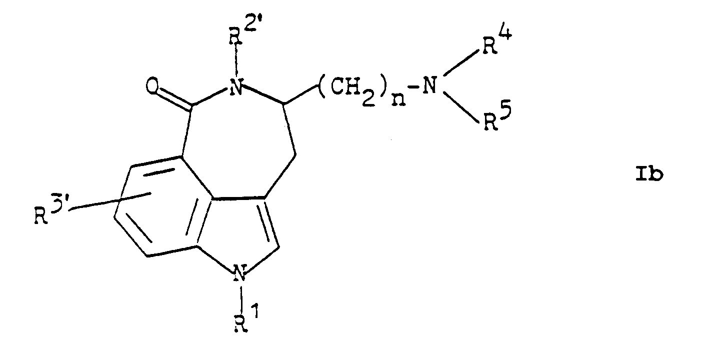 worin R¹, R³', R⁴, R⁵ und n obige Bedeutung besitzen und R²' Wasserstoff oder niederes Alkyl darstellt, Verbindungen der allgemeinen Formel III 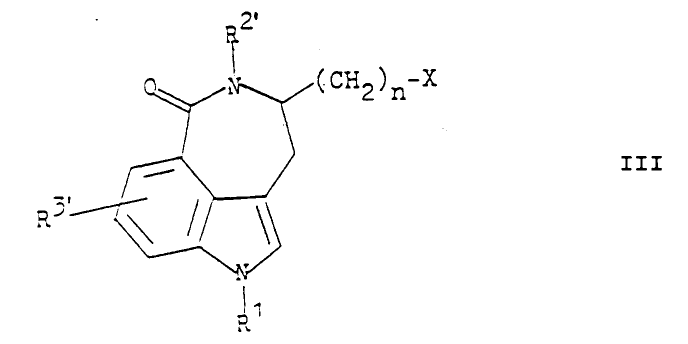 worin R¹, R²', R^{3'} und n obige Bedeutung besitzen und X eine nukleophil abspaltbare Fluchtgruppe darstellt, mit Verbindungen der allgemeinen Formel IV 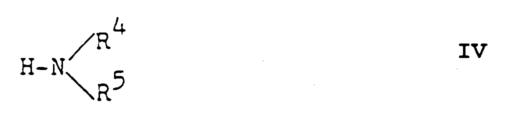 worin R⁴ und R⁵ obige Bedeutung besitzen, umsetzt, oder
c) zur Herstellung von Verbindungen der allgemeinen Formel Ic 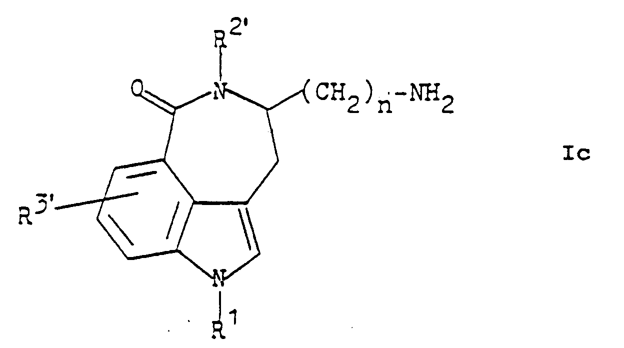 worin R¹, R²', R^{3'} und n obige Bedeutung besitzen, in Verbindungen der allgemeinen Formel V 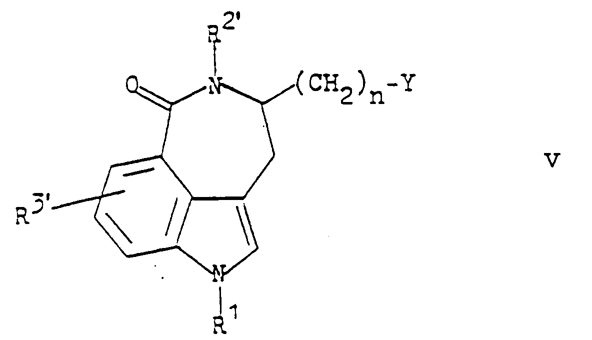 worin R¹, R²', R³' und n obige Bedeutung besitzen und Y für eine Azid- oder Phthalimidgruppe oder, falls n 1 ist, auch für die Cyangruppe steht, die Gruppe Y in die Aminogruppe überführt, oder
d) zur Herstellung von Verbindungen der allgemeinen Formel Id 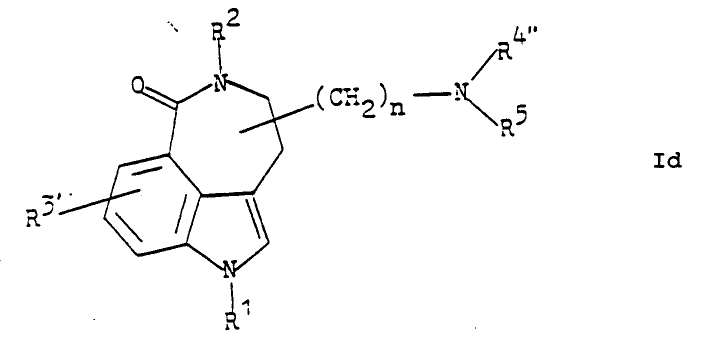 worin R¹, R², R³', R⁵ und n obige Bedeutung besitzen und R⁴'' die für R⁴ angegebene Bedeutung mit Ausnahme von Wasserstoff besitzt,
Verbindungen der allgemeinen Formel VI 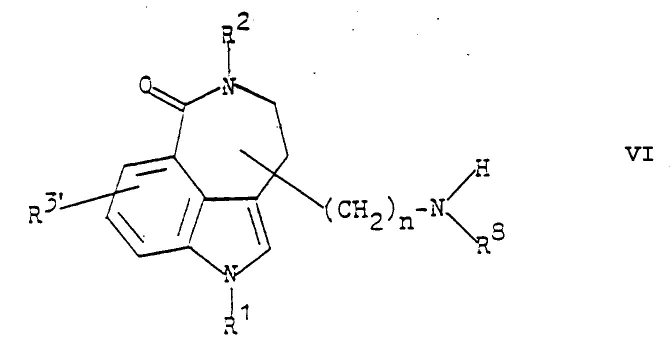 worin R¹, R², R³' und n obige Bedeutung besitzen und R⁸ Wasserstoff, Alkyl mit 1 bis 5 Kohlenstoffatomen, Cycloalkyl mit 3 bis 6 Kohlenstoffatomen, Cycloalkylalkyl mit 4 bis 9 Kohlenstoffatomen, eine gegebenenfalls im Phenylring durch niederes Alkyl, niederes Alkoxy, Hydroxy oder Halogen mono- oder disubstituierte Phenylniederalkylgruppe oder eine Aminoschutzgruppe bedeutet, alkyliert und eine allfällige Aminoschutzgruppe R⁸ wieder abspaltet, oder
e) zur Herstellung von Verbindungen der allgemeinen Formel Ie 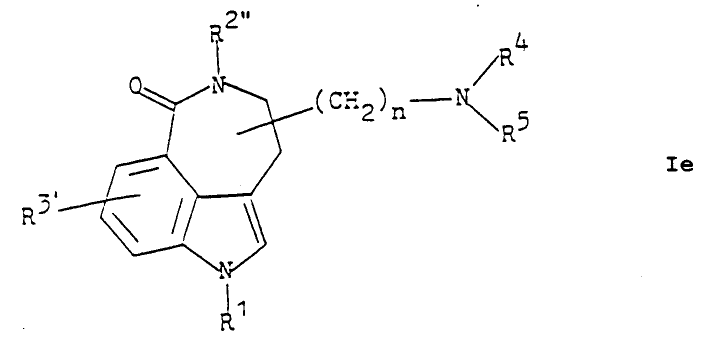 worin R¹, R³', R⁴, R⁵ und n obige Bedeutung besitzen und R²'' eine gegebenenfalls in α-Stellung zum Stickstoffatom durch niederes Alkoxy substituierte niedere Alkylgruppe bedeutet, in Verbindungen der allgemeinen Formel VII 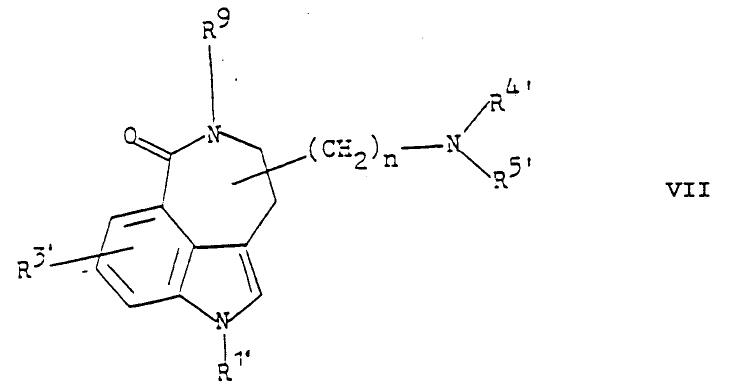 worin R³ 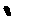 und n obige Bedeutung besitzen, R¹' die für R¹ angegebene Bedeutung besitzt oder für eine Aminoschutzgruppe steht, R⁴' und R⁵' die für R⁴ und R⁵ angegebenen Bedeutungen besitzen, wobei jedoch eine NR⁴R⁵-Gruppe, worin R⁴ und/oder R⁵ Wasserstoff bedeuten, durch mindestens eine leicht abspaltbare Aminoschutzgruppe derart geschützt ist, daß sie nicht mit Acylierungs- oder Alkylierungsmitteln reagiert, und R⁹ eine niedere 1-Hydroxyalkylgruppe darstellt, die 1-Hydroxyalkylgruppe in den Rest R²'' überführt und allfällige Aminoschutzgruppen wieder abspaltet, oder
f) zur Herstellung von Verbindungen der allgemeinen Formel If 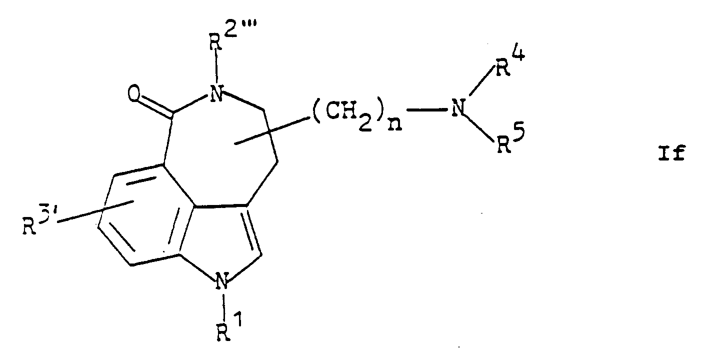 worin R¹, R³', R⁴, R⁵ und n obige Bedeutung besitzen und R²''' niederes Alkyl bedeutet,
Verbindungen der allgemeinen Formel VIII 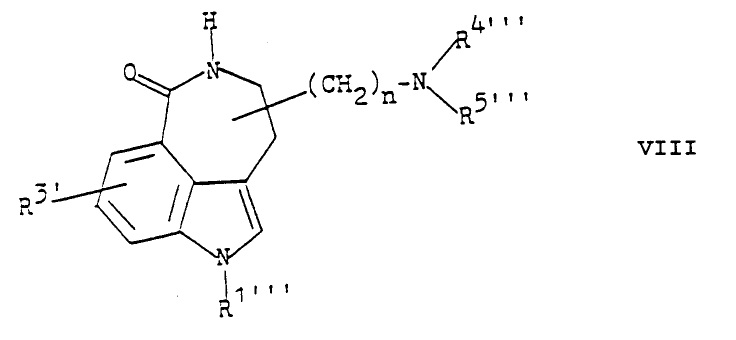 worin R³' und n obige Bedeutung besitzen und R¹''', R⁴''' und R⁵''' die für R¹, R⁴ und R⁵ angegebenen Bedeutungen mit Ausnahme von Wasserstoff besitzen oder eine Aminoschutzgruppe darstellen, mit Verbindungen der allgemeinen Formel XII
R²'''-X XII
worin R²''' und X obige Bedeutung besitzen, umsetzt und anschließend allfällige Aminoschutzgruppen wieder abspaltet, oder
g) zur Herstellung von Verbindungen der allgemeinen Formel Ig 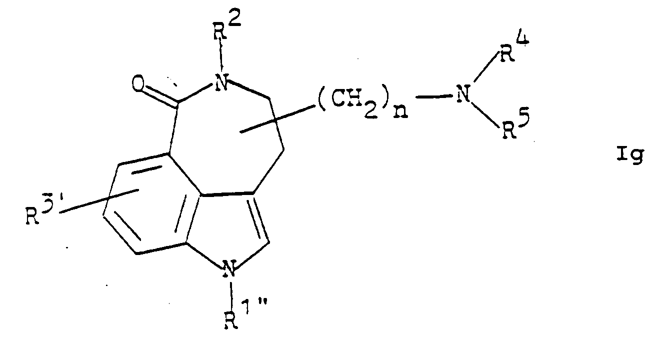 worin R², R³', R⁴, R⁵ und n obige Bedeutung besitzen und R¹'' die für R¹ angegebene Bedeutung mit Ausnahme von Wasserstoff besitzt, Verbindungen der allgemeinen Formel IX 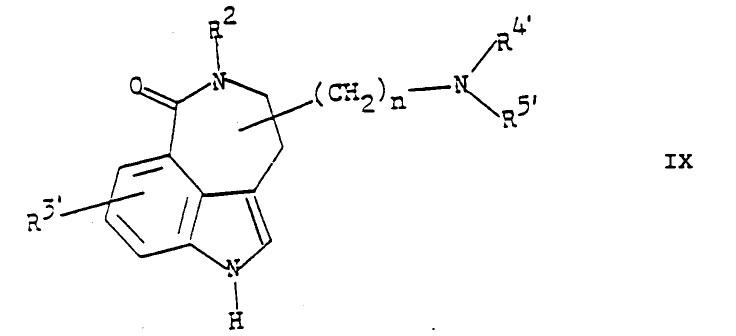 worin R², R³', R⁴', R^{5'} und n obige Bedeutung besitzen, mit Verbindungen der allgemeinen Formel X
R¹''-X X
worin R¹'' und X obige Bedeutung besitzen, umsetzt und anschließend allfällige Aminoschutzgruppen wieder abspaltet, oder
h) zur Herstellung von Verbindungen der allgemeinen Formel Ih 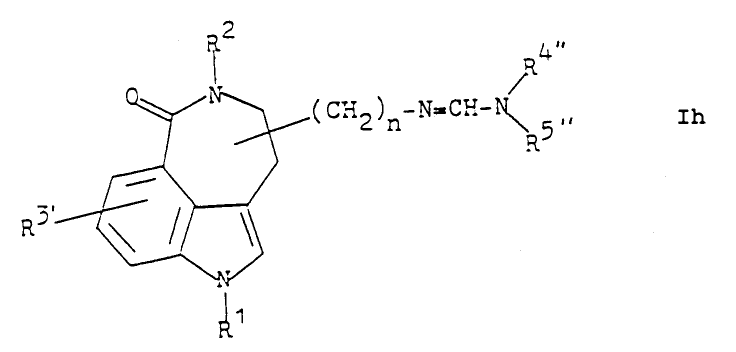 worin R¹, R², R³', R⁴'' und n obige Bedeutung besitzen und R⁵'' die für R⁵ angegebene Bedeutung mit Ausnahme von Wasserstoff besitzt, in Verbindungen der allgemeinen Formel Ii 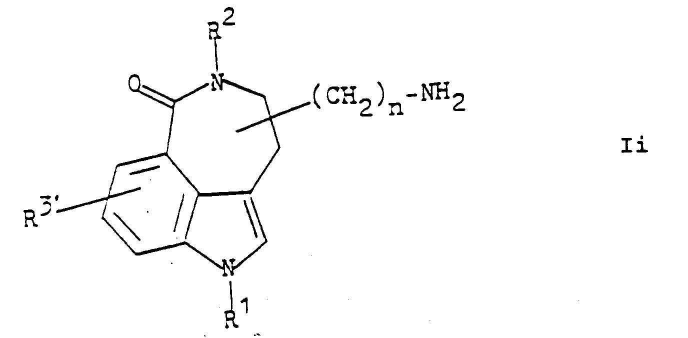 worin R¹, R², R³' und n obige Bedeutung besitzen, den Aminomethylenrest der allgemeinen Formel b 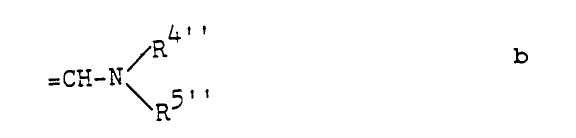 worin R⁴'' und R⁵'' obige Bedeutung besitzen, einführt, wobei sofern die Substituenten niedere Alkylgruppen
darstellen, können diese 1 bis 4 Kohlenstoffatome enthalten, und gewünschtenfalls in erhaltenen Verbindungen der Formel I, worin R³' Methoxy bedeutet und/oder R¹, R⁴ und/oder R⁵ eine Methoxyphenylgruppe enthalten, die Methoxygruppe zur Hydroxygruppe spaltet und/oder in erhaltenen Verbindungen der Formel I, worin R¹, R⁴, R⁵ und/oder R⁶ eine gegebenenfalls substituierte Benzylgruppe darstellen, diese Benzylgruppe hydrogenolytisch abspaltet und gewünschtenfalls freie Verbindungen der Formel I in ihre Säureadditionssalze überführt oder die Säureadditionssalze in die freien Verbindungen der Formel I überführt.

9. Verbindungen der allgemeinen Formel II 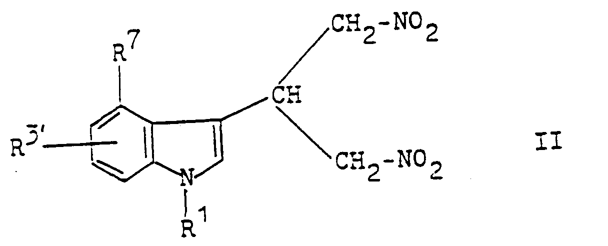 worin
R¹ für Wasserstoff, für eine niedere Alkyl- oder Cycloalkylalkylgruppe oder für eine Phenylniederalkylgruppe, welche gegebenenfalls im Phenylring durch niederes Alkoxy, Hydroxy, Halogen oder niederes Alkyl mono- oder disubstituiert sein kann, steht,
R³ 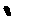 Wasserstoff, niederes Alkyl, niederes Alkoxy oder Halogen bedeutet, und
R⁷ eine niedere Alkoxycarbonylgruppe oder die CN-Gruppe bedeutet, wobei sofern die Substituenten niedere Alkylgruppen darstellen, können diese 1 bis 4 Kohlenstoffatome enthalten,

10. Verbindungen der allgemeinen Formel VII 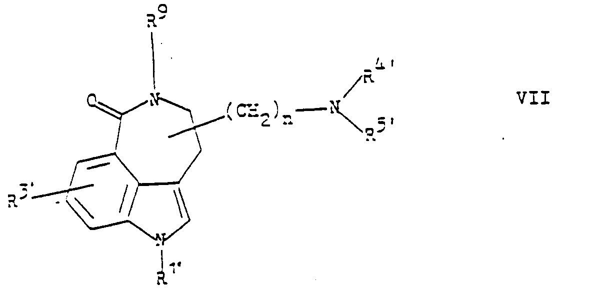 worin
R^{1'} für Wasserstoff, für eine niedere Alkyl- oder Cycloalkylalkylgruppe oder für eine Phenylniederalkylgruppe, welche gegebenenfalls im Phenylring durch niederes Alkoxy, Hydroxy, Halogen oder niederes Alkyl mono- oder disubstituiert sein kann, oder für eine Aminoschutzgruppe steht,
R^{3'} Wasserstoff, niederes Alkyl, niederes Alkoxy oder Halogen bedeutet,
n für 1 oder, falls die -(CH₂)ₙ-Kette in 4-Stellung des Ringgerüstes angeordnet ist, auch für 2 steht,
R^{4'} Wasserstoff, Alkyl mit 1 bis 5 Kohlenstoffatomen, Cycloalkyl mit 3 bis 6 Kohlenstoffatomen, Cycloalkylalkyl mit 4 bis 9 Kohlenstoffatomen oder eine gegebenenfalls im Phenylring durch niederes Alkyl, niederes Alkoxy, Hydroxy oder Halogen mono- oder disubstituierte Phenylniederalkylgruppe bedeutet, und
R^{5'} Wasserstoff, Alkyl mit 1 bis 5 Kohlenstoffatomen, Cycloalkyl mit 3 bis 6 Kohlenstoffatomen, Cycloalkylalkyl mit 4 bis 9 Kohlenstoffatomen oder eine gegebenenfalls im Phenylring durch niederes Alkyl, niederes Alkoxy, Hydroxy oder Halogen mono- oder disubstituierte Phenylniederalkylgruppe bedeutet, oder
R^{4'} und R^{5'} gemeinsam mit dem Stickstoffatom, an welches sie gebunden sind, einen Heterocyclus der allgemeinen Formel a 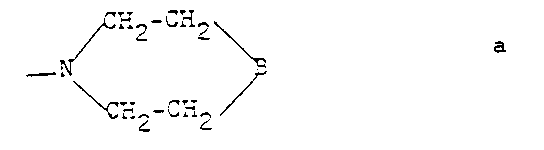 bilden, worin
B für eine Bindung, die Methylengruppe, Sauerstoff oder eine Iminogruppe -NR⁶-, worin R⁶ Wasserstoff, niederes Alkyl oder gegebenenfalls im Phenylring durch niederes Alkyl, niederes Alkoxy, Hydroxy oder Halogen substituiertes Phenyl oder Benzyl bedeutet, steht, wobei jedoch eine NR^{4'}R^{5'}-Gruppe, worin R^{4'} und/oder R^{5'} Wasserstoff bedeuten, durch mindestens eine leicht abspaltbare Aminoschutzgruppe geschützt ist, und
R⁹ eine niedere 1-Hydroxyalkylgruppe darstellt, wobei sofern die Substituenten niedere Alkylgruppen darstellen, können diese 1 bis 4 Kohlenstoffatome enthalten,

11. Verbindungen der allgemeinen Formel XXVI 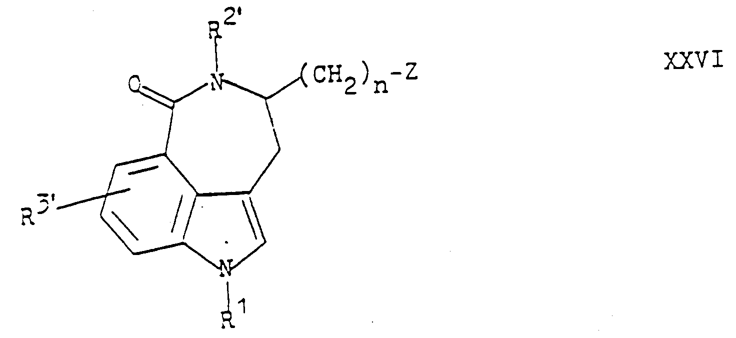 worin
R¹ für Wasserstoff, für eine niedere Alkyl- oder Cycloalkylalkylgruppe oder für eine Phenylniederalkylgruppe, welche gegebenenfalls im Phenylring durch niederes Alkoxy, Hydroxy, Halogen oder niederes Alkyl mono- oder disubstituiert sein kann, steht,
R^{2'} Wasserstoff oder eine niedere Alkylgruppe bedeutet,
R^{3'} Wasserstoff, niederes Alkyl, niederes Alkoxy oder Halogen bedeutet,
n für 1 oder 2 steht, und
Z für Hydroxy, für eine nukleophil abspaltbare Fluchtgruppe, für eine Azid- oder Phthalimidgruppe oder, falls n 1 ist, auch für die Cyangruppe steht, und wobei sofern die Substituenten niedere Alkylgruppen darstellen, können diese 1 bis 4 Kohlenstoffatome enthalten,

## Claims

1. Compounds of the general formula I 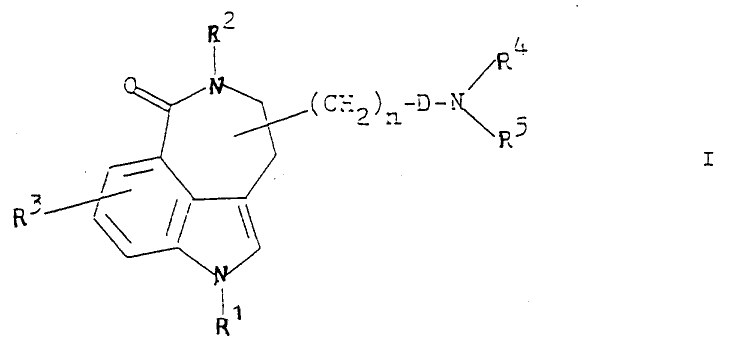 in which
R¹ represents hydrogen, a lower alkyl or cycloalkylalkyl group or a phenyl-lower alkyl group which can optionally be mono- or disubstituted in the phenyl ring by lower alkoxy, hydroxyl, halogen or lower alkyl,
R² represents hydrogen or a lower alkyl group optionally substituted in the α-position to the nitrogen atom by lower alkoxy,
R³ denotes hydrogen, lower alkyl, lower alkoxy, halogen or, if the substituents R¹, R², R⁴ and/or R⁵ do not contain any lower alkoxy groups, also denotes hydroxyl,
n represents 1 or, if the -(CH₂)ₙ- chain is in the 4-position of the ring structure, also represents 2,
R⁴ denotes hydrogen, alkyl having 1 to 5 carbon atoms, cycloalkyl having 3 to 6 carbon atoms, cycloalkylalkyl having 4 to 9 carbon atoms or a phenyl-lower alkyl group optionally mono- or disubstituted in the phenyl ring by lower alkyl, lower alkoxy, hydroxyl or halogen, and
R⁵ denotes hydrogen, alkyl having 1 to 5 carbon atoms, cycloalkyl having 3 to 6 carbon atoms, cycloalkylalkyl having 4 to 9 carbon atoms or a phenyl-lower alkyl group optionally mono- or disubstituted in the phenyl ring by lower alkyl, lower alkoxy, hydroxyl or halogen, or
R⁴ and R⁵, together with the nitrogen atom to which they are bonded, form a heterocycle of the general formula a 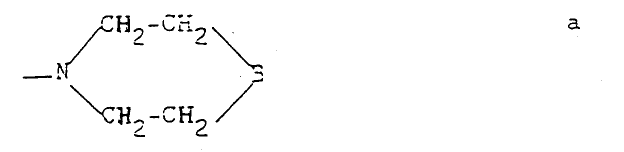 in which
B represents a bond, the methylene group, oxygen or an imino group -NR⁶-, in which R⁶ denotes hydrogen, lower alkyl or phenyl or benzyl optionally substituted in the phenyl ring by lower alkyl, lower alkoxy, hydroxyl or halogen, and
D represents a bond or, if R⁴ and R⁵ do not denote hydrogen, also represents the -N=CH- group, wherein if the substituents represent lower alkyl groups, these may contain 1 to 4 carbon atoms,
and their physiologically tolerable acid addition salts.

2. Compounds according to Claim 1, characterised in that R¹ denotes hydrogen.

3. Compounds according to Claim 1 or 2, characterised in that R² denotes hydrogen.

4. Compounds according to Claims 1 to 3, characterised in that R⁴ denotes hydrogen, lower alkyl having 1 to 4 carbon atoms or optionally substituted benzyl and R⁵ denotes hydrogen.

5. Compounds according to Claims 1 to 4, characterised in that D represents a bond.

6. Compounds according to Claims 1 to 5, characterised in that they contain a -CH₂-D-NR⁴R⁵ group in the 3-position of the ring structure.

7. Medicament containing a pharmacologically active amount of a compound according to Claim 1 and customary pharmaceutical auxiliaries and/or excipients.

8. Process for the preparation of compounds of the general formula I 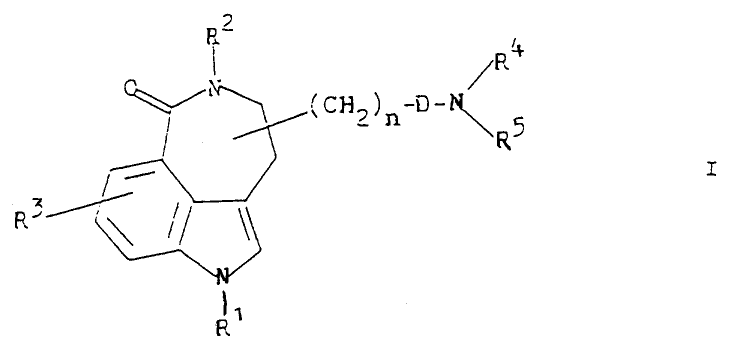 in which
R¹ represents hydrogen, a lower alkyl or cycloalkylalkyl group or a phenyl-lower alkyl group which can optionally be mono- or disubstituted in the phenyl ring by lower alkoxy, hydroxyl, halogen or lower alkyl,
R² represents hydrogen or a lower alkyl group optionally substituted in the α-position to the nitrogen atom by lower alkoxy,
R³ denotes hydrogen, lower alkyl, lower alkoxy, halogen or, if the substituents R¹, R², R⁴ and/or R⁵ do not contain any lower alkoxy groups, also denotes hydroxyl,
n represents 1 or, if the -(CH₂)ₙ- chain is in the 4-position of the ring structure, also represents 2,
R⁴ denotes hydrogen, alkyl having 1 to 5 carbon atoms, cycloalkyl having 3 to 6 carbon atoms, cycloalkylalkyl having 4 to 9 carbon atoms or a phenyl-lower alkyl group optionally mono- or disubstituted in the phenyl ring by lower alkyl, lower alkoxy, hydroxyl or halogen, and
R⁵ denotes hydrogen, alkyl having 1 to 5 carbon atoms, cycloalkyl having 3 to 6 carbon atoms, cycloalkylalkyl having 4 to 9 carbon atoms or a phenyl-lower alkyl group optionally mono- or disubstituted in the phenyl ring by lower alkyl, lower alkoxy, hydroxyl or halogen, or
R⁴ and R⁵, together with the nitrogen atom to which they are bonded, form a heterocycle of the general formula a 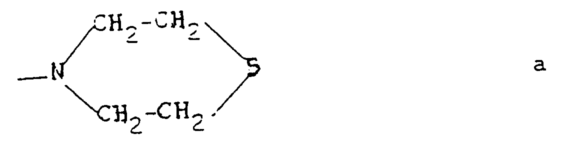 in which
B represents a bond, the methylene group, oxygen or an imino group -NR⁶-, in which R⁶ denotes hydrogen, lower alkyl or phenyl or benzyl optionally substituted in the phenyl ring by lower alkyl, lower alkoxy, hydroxyl or halogen, and
D represents a bond or, if R⁴ and R⁵ do not denote hydrogen, also represents the -N=CH- group, wherein if the substituents represent lower alkyl groups, these may contain 1 to 4 carbon atoms,
and their physiologically tolerable acid addition salts, characterised in that
a) for the preparation of compounds of the general formula Ia 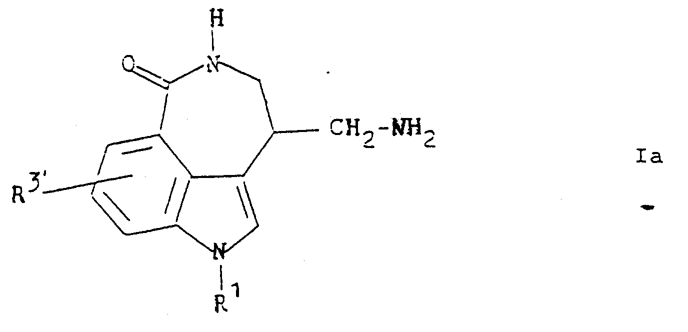 in which R¹ has the above meaning and R³' has the meaning given for R³ with the exception of hydroxyl, compounds of the general formula II 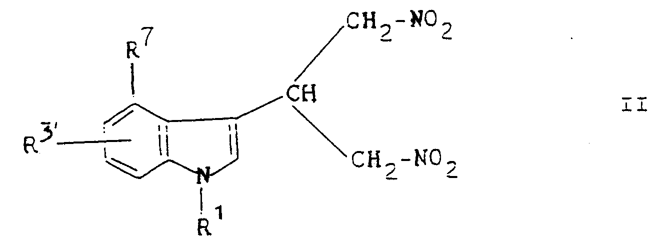 in which R¹ and R^{3'} have the above meaning and R⁷ denotes a lower alkoxycarbonyl group or the CN group, are cyclised under reducing conditions, or
b) for the preparation of compounds of the general formula Ib 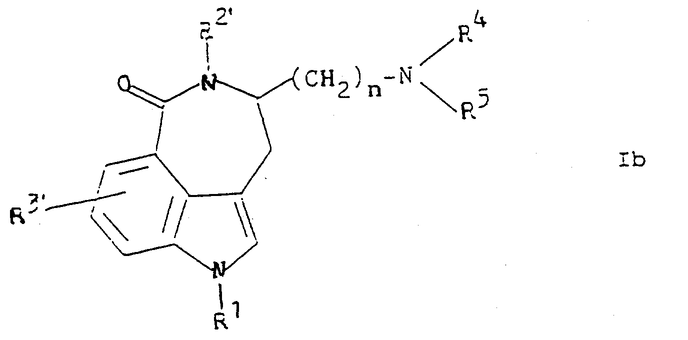 in which R¹, R^{3'}, R⁴, R⁵ and n have the above meaning and R^{2'} represents hydrogen or lower alkyl, compounds of the general formula III 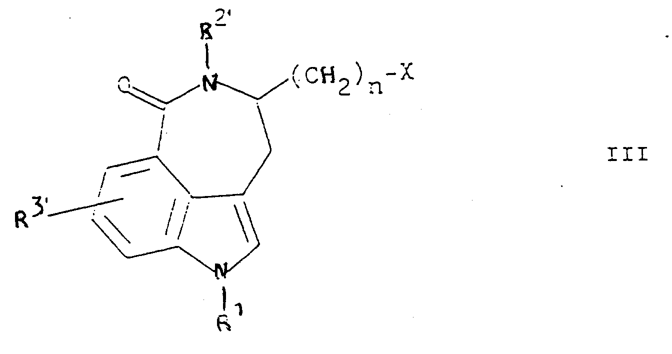 in which R¹, R²', R^{3'} and n have the above meaning and X represents a leaving group which can be removed nucleophilically, are reacted with compounds of the general formula IV 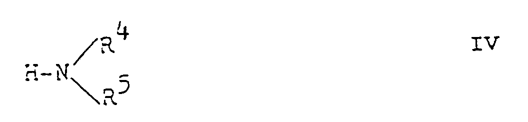 in which R⁴ and R⁵ have the above meaning, or
c) for the preparation of compounds of the general formula Ic 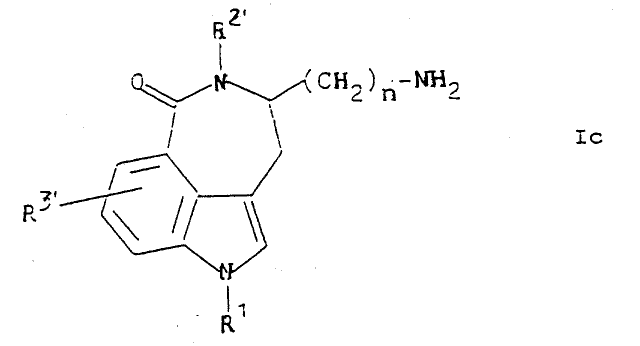 in which R¹, R^{2'}, R^{3'} and n have the above meaning, the group Y in compounds of the general formula V 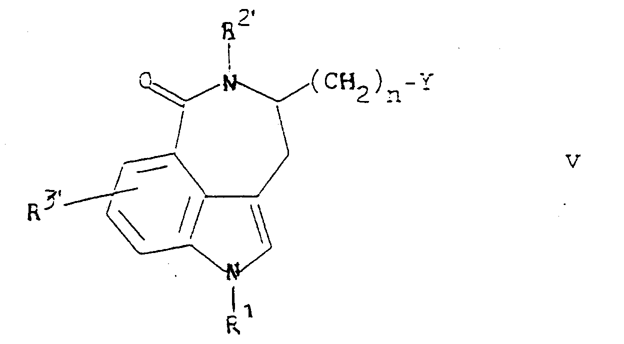 in which R¹, R^{2'}, R^{3'} and n have the above meaning and Y represents an azide or phthalimide group or, if n is 1, also represents the cyano group, is converted into the amino group, or
d) for the preparation of compounds of the general formula Id 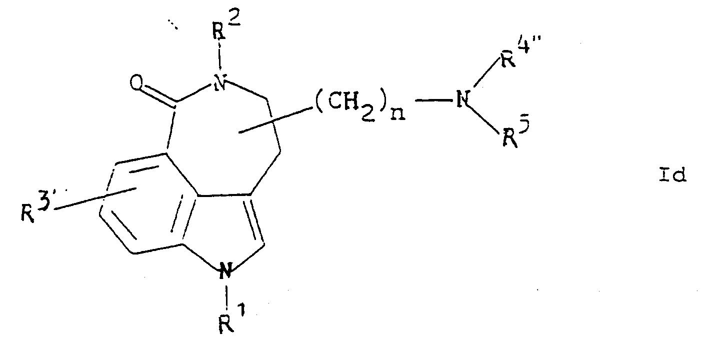 in which R¹, R², R^{3'}, R⁵ and n have the above meaning and R^{4''} has the meaning given for R⁴ with the exception of hydrogen,
compounds of the general formula VI 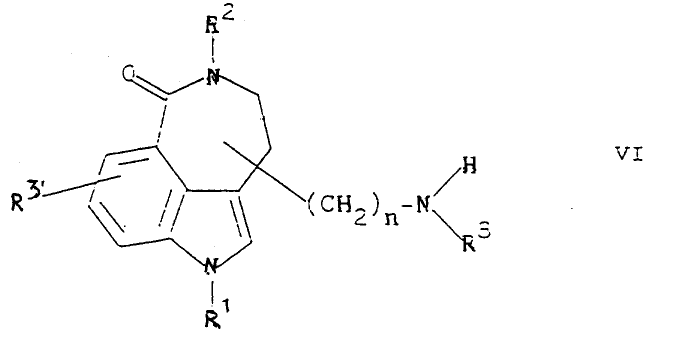 in which R¹, R², R^{3'} and n have the above meaning and R⁸ denotes hydrogen, alkyl having 1 to 5 carbon atoms, cycloalkyl having 3 to 6 carbon atoms, cycloalkylalkyl having 4 to 9 carbon atoms, a phenyl-lower alkyl group optionally mono- or disubstituted in the phenyl ring by lower alkyl, lower alkoxy, hydroxyl or halogen, or an amino protective group, are alkylated and any amino protective group R⁸ is removed again, or
e) for the preparation of compounds of the general formula Ie 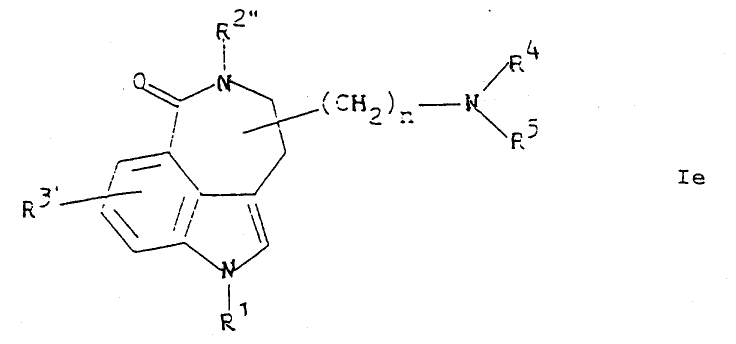 in which R¹, R^{3'}, R⁴, R⁵ and n have the above meaning and R^{2''} denotes a lower alkyl group optionally substituted in the α-position to the nitrogen atom by lower alkoxy, the 1-hydroxyalkyl group in compounds of the general formula VII 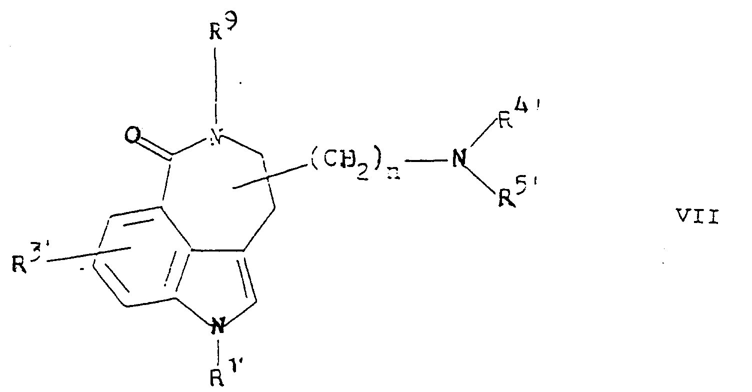 in which R^{3'} and n have the above meaning, R^{1'} has the meaning given for R¹ or represents an amino protective group, R^{4'} and R^{5'} have the meanings given for R⁴ and R⁵, where, however, an NR⁴R⁵ group in which R⁴ and/or R⁵ denote hydrogen is protected by at least one easily removable amino protective group in such a way that it does not react with acylating or alkylating agents, and R⁹ represents a lower 1-hydroxyalkyl group, is converted into the radical R^{2''} and any amino protective groups are removed again, or
f) for the preparation of compounds of the general formula If 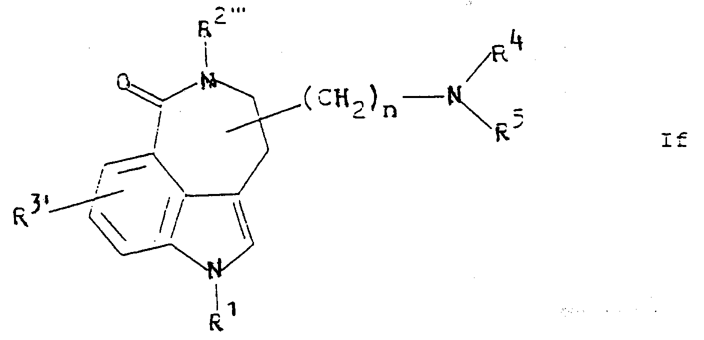 in which R¹, R^{3'}, R⁴, R⁵ and n have the above meaning and R^{2'''} denotes lower alkyl,
compounds of the general formula VIII 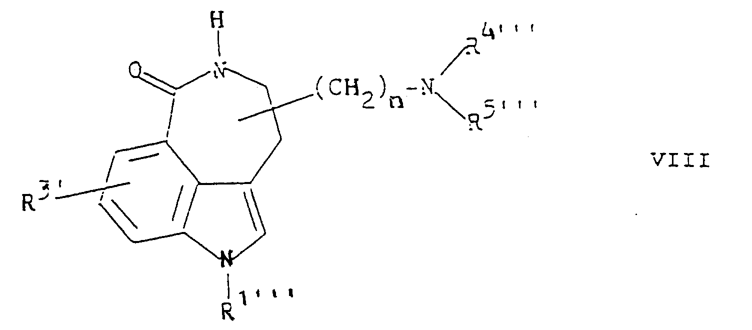 in which R^{3'} and n have the above meaning and R^{1'''}, R^{4'''} and R^{5'''} have the meanings given for R¹, R⁴ and R⁵ with the exception of hydrogen or represent an amino protective group, are reacted with compounds of the general formula XII
R^{2'''}-X XII
in which R^{2'''} and X have the above meaning, and then any amino protective groups are removed again, or
g) for the preparation of compounds of the general formula Ig 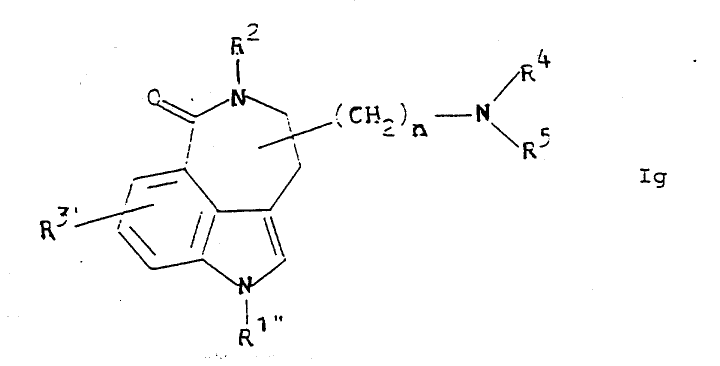 in which R², R^{3'}, R⁴, R⁵ and n have the above meaning and R^{1''} has the meaning given for R¹ with the exception of hydrogen, compounds of the general formula IX 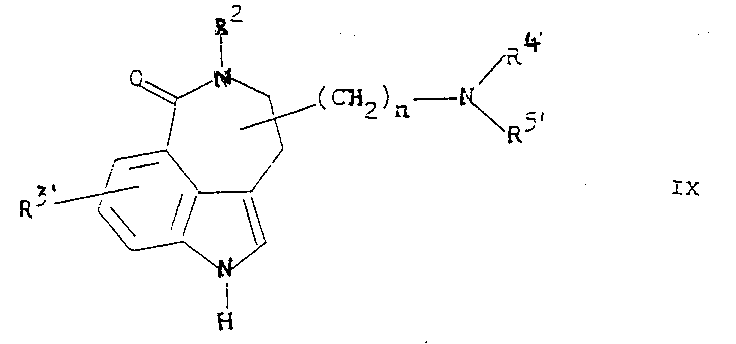 in which R², R^{3'}, R^{4'}, R^{5'} and n have the above meaning, are reacted with compounds of the general formula X
R^{1''}-X X
in which R^{1''} and X have the above meaning, and then any amino protective groups are removed again, or
h) for the preparation of compounds of the general formula Ih 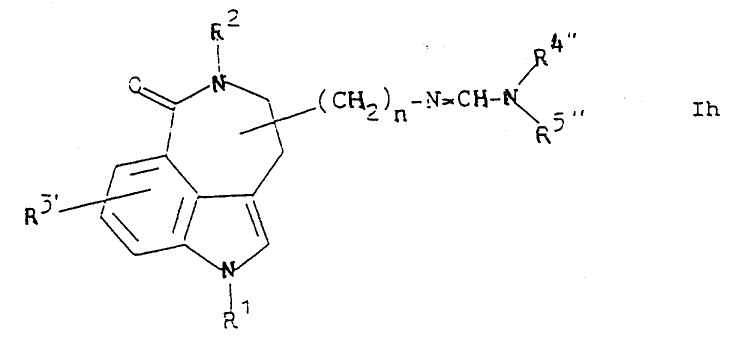 in which R¹, R², R^{3'}, R^{4''} and n have the above meaning and R^{5''} has the meaning given for R⁵ with the exception of hydrogen, there is introduced in compounds of the general formula Ii 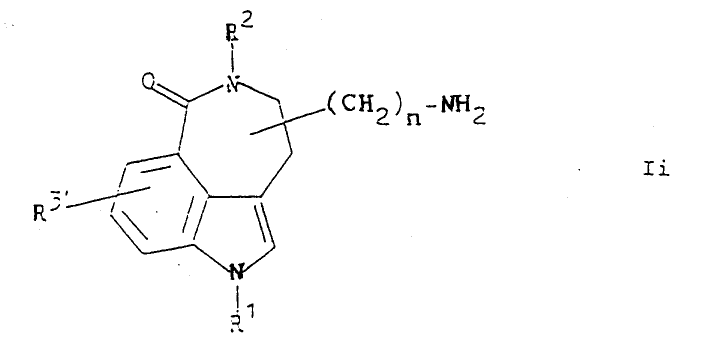 in which R¹, R², R^{3'} and n have the above meaning, the aminomethylene radical of the general formula b 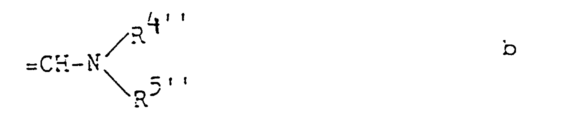 in which R^{4''} and R^{5''} have the above meaning, wherein if the substituents represent lower alkyl groups, these may contain 1 to 4 carbon atoms,
and, if desired, in resulting compounds of formula I in which R^{3'} denotes methoxy and/or R¹, R⁴ and/or R⁵ contain a methoxyphenyl group, the methoxy group is cleaved to give the hydroxyl group and/or in resulting compounds of formula I in which R¹, R⁴, R⁵ and/or R⁶ represent an optionally substituted benzyl group, this benzyl group is removed by hydrogenolysis and, if desired, free compounds of formula I are converted into their acid addition salts or the acid addition salts are converted into the free compounds of formula I.

9. Compounds of the general formula II 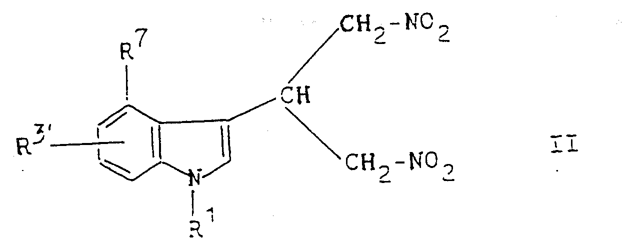 in which
R¹ represents hydrogen, a lower alkyl or cycloalkylalkyl group or a phenyl-lower alkyl group which can be optionally mono- or disubstituted in the phenyl ring by lower alkoxy, hydroxyl, halogen or lower alkyl,
R³' denotes hydrogen, lower alkyl, lower alkoxy or halogen, and
R⁷ denotes a lower alkoxycarbonyl group or the CN group, wherein if the substituents represent lower alkyl groups, these may contain 1 to 4 carbon atoms.

10. Compounds of the general formula VII 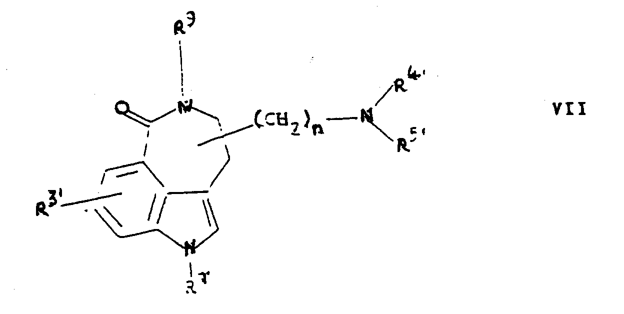 in which
R^{1'} represents hydrogen, a lower alkyl or cycloalkylalkyl group or a phenyl-lower alkyl group which can be optionally mono- or disubstituted in the phenyl ring by lower alkoxy, hydroxyl, halogen or lower alkyl, or an amino protective group,
R^{3'} denotes hydrogen, lower alkyl, lower alkoxy or halogen,
n represents 1 or, if the -(CH₂)ₙ- chain is in the 4-position of the ring structure, also represents 2,
R^{4'} denotes hydrogen, alkyl having 1 to 5 carbon atoms, cycloalkyl having 3 to 6 carbon atoms, cycloalkylalkyl having 4 to 9 carbon atoms or a phenyl-lower alkyl group which is optionally mono- or disubstituted in the phenyl ring by lower alkyl, lower alkoxy, hydroxyl or halogen, and
R^{5'} denotes hydrogen, alkyl having 1 to 5 carbon atoms, cycloalkyl having 3 to 6 carbon atoms, cycloalkylalkyl having 4 to 9 carbon atoms or a phenyl-lower alkyl group which is optionally mono- or disubstituted in the phenyl ring by lower alkyl, lower alkoxy, hydroxyl or halogen, or
R^{4'} and R^{5'}, together with the nitrogen atom to which they are bonded, form a heterocycle of the general formula a 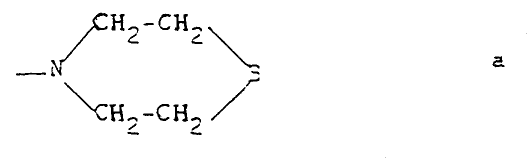 in which
B represents a bond, the methylene group, oxygen or an imino group -NR⁶-, in which R⁶ denotes hydrogen, lower alkyl or phenyl or benzyl optionally substituted in the phenyl ring by lower alkyl, lower alkoxy, hydroxyl or halogen, but where an NR⁴'R⁵' group in which R⁴' and/or R⁵' denote hydrogen, is protected by at least one easily removable amino protective group, and
R⁹ represents a lower 1-hydroxyalkyl group, wherein if the substituents represent lower alkyl groups, these may contain 1 to 4 carbon atoms.

11. Compounds of the general formula XXVI 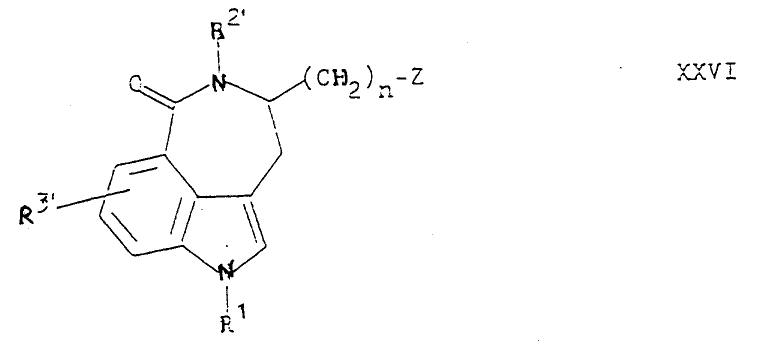 in which
R¹ represents hydrogen, a lower alkyl or cycloalkylalkyl group or a phenyl-lower alkyl group which can be optionally mono-or disubstituted in the phenyl ring by lower alkoxy, hydroxyl, halogen or lower alkyl,
R^{2'} denotes hydrogen or a lower alkyl group,
R^{3'} denotes hydrogen, lower alkyl, lower alkoxy or halogen,
n represents 1 or 2, and
Z represents hydroxyl, a leaving group which can be removed nucleophilically, an azide or phthalimide group or, if n is 1, also represents the cyano group, and wherein if the substituents represent lower alkyl groups, these may contain 1 to 4 carbon atoms.

## Revendications

1. Composés de formule générale I 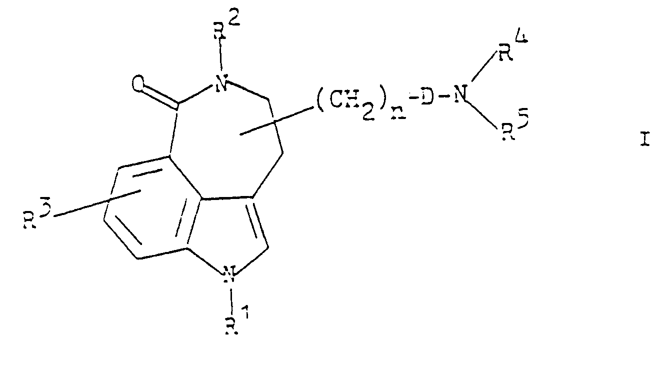 dans laquelle
R¹ représente un atome d*'*hydrogène ou un groupe alkyle inférieur ou cycloalkylalkyle inférieur ou un groupe phényl-alkyle inférieur qui peut éventuellement être mono- ou disubstitué sur le noyau phényle par un ou des atomes d*'*halogène ou groupes alcoxy inférieur, hydroxy ou alkyle inférieur;
R² représente un atome d*'*hydrogène ou un groupe alkyle inférieur éventuellement substitué, en position α par rapport à l*'*atome d*'*azote, par un groupe alcoxy inférieur;
R³ représente un atome d*'*hydrogène ou d*'*halogène ou un groupe alkyle inférieur, alcoxy inférieur ou, dans le cas où les substituants R¹, R², R⁴ et/ou R⁵ ne contiennent pas de groupes alcoxy inférieurs, également le groupe hydroxy;
n représente 1 ou, lorsque la chaîne -(CH₂)ₙ- est disposée en position 4 du squelette cyclique, également 2;
R⁴ représente un atome d*'*hydrogène ou un groupe alkyle ayant de 1 à 5 atomes de carbone, cycloalkyle ayant de 3 à 6 atomes de carbone, cycloalkylalkyle ayant de 4 à 9 atomes de carbone, ou un groupe phényl-alkyle inférieur éventuellement mono- ou disubstitué sur le noyau phényle par un ou des atomes d*'*halogène ou groupes alkyle inférieur, alcoxy inférieur ou hydroxy, et
R⁵ représente un atome d'hydrogène ou un groupe alkyle ayant de 1 à 5 atomes de carbone, cycloalkyle ayant de 3 à 6 atomes de carbone, cycloalkylalkyle ayant de 4 à 9 atomes de carbone, ou un groupe phényl-alkyle inférieur éventuellement mono- ou disubstitué sur le noyau phényle par un ou des atomes d'halogène ou groupes alkyle inférieur, alcoxy inférieur ou hydroxy, ou
R⁴ et R⁵ forment, conjointement avec l'atome d'azote auquel ils sont liés, un hétérocycle de formule générale a 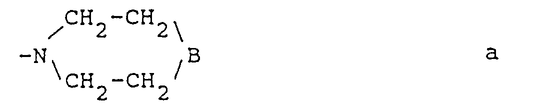 dans laquelle
B représente une liaison, le groupe méthylène, un atome d'oxygène ou un groupe imino -NR⁶-, R⁶ représentant un atome d'hydrogène, un groupe alkyle inférieur ou un groupe phényle ou benzyle éventuellement substitué sur le noyau phényle par un ou des groupes alkyle inférieur, alcoxy inférieur, hydroxy ou par un ou des atomes d'halogène, et
D représente une liaison ou, lorsque R⁴ et R⁵ ne représentent pas des atomes d'hydrogène, également le groupe -N=CH-, et dans le cas où les substituants sont des groupes alkyle inférieurs, ces derniers renferment de 1 à 4 atomes de carbone,
et les sels d'addition avec des acides physiologiquement acceptables de ceux-ci.

2. Composés selon la revendication 1, caractérisés en ce que R¹ représente un atome d'hydrogène.

3. Composés selon la revendication 1 ou 2, caractérisés en ce que R² représente un atome d'hydrogène.

4. Composés selon l'une quelconque des revendications 1 à 3, caractérisés en ce que R⁴ représente un atome d'hydrogène ou un groupe alkyle inférieur ayant de 1 à 4 atomes de carbone ou un groupe benzyle éventuellement substitué, et R⁵ représente un atome d'hydrogène.

5. Composés selon l'une quelconque des revendications 1 à 4, caractérisés en ce que D représente une liaison.

6. Composés selon l'une quelconque des revendications 1 à 5, caractérisés en ce qu'ils contiennent un groupe -CH₂-D-NR⁴R⁵ en position 3 du squelette cyclique.

7. Médicament contenant une quantité pharmacologiquement efficace d'un composé selon la revendication 1 et des adjuvants et/ou véhicules pharmaceutiques usuels.

8. Procédé pour la préparation de composés de formule générale I 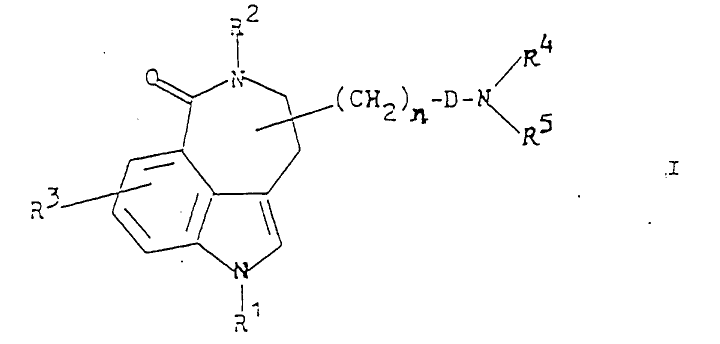 dans laquelle
R¹ représente un atome d'hydrogène ou un groupe alkyle inférieur ou cycloalkylalkyle ou un groupe phényl-alkyle inférieur qui peut éventuellement être mono- ou disubstitué sur le noyau phényle par un ou des atomes d'halogène ou groupe alcoxy inférieur, hydroxy ou alkyle inférieur ;
R² représente un atome d'hydrogène ou un groupe alkyle inférieur éventuellement substitué, en position α par rapport à l'atome d'azote, par un radical alcoxy inférieur ;
R³ représente un atome d'hydrogène ou d'halogène ou un groupe alkyle inférieur, alcoxy inférieur ou, dans le cas où les substituants R¹, R², R⁴ et/ou R⁵ ne contiennent pas de groupes alcoxy inférieurs, également le groupe hydroxy ;
n représente 1 ou, lorsque la chaîne -(CH₂)ₙ- est disposée en position 4 du squelette cyclique, également 2;
R⁴ représente un atome d'hydrogène ou un groupe alkyle ayant de 1 à 5 atomes de carbone, cycloalkyle ayant de 3 à 6 atomes de carbone, cycloakylalkyle ayant de 4 à 9 atomes de carbone, ou un groupe phényl-alkyle inférieur éventuellement mono- ou disubstitué sur le noyau phényle par un ou des atomes d'halogène ou groupes alkyle inférieur, alcoxy inférieur ou hydroxy, et
R⁵ représente un atome d'hydrogène ou un groupe alkyle ayant de 1 à 5 atomes de carbone, cycloalkyle ayant de 3 à 6 atomes de carbone, cycloakylalkyle ayant de 4 à 9 atomes de carbone, ou un groupe phényl-alkyle inférieur éventuellement mono- ou disubstitué sur le noyau phényle par un ou des atomes d'halogène ou groupes alkyle inférieur, alcoxy inférieur ou hydroxy, ou
R⁴ et R⁵ forment, conjointement avec l'atome d'azote auquel ils sont liés, un hétérocycle de formule générale a 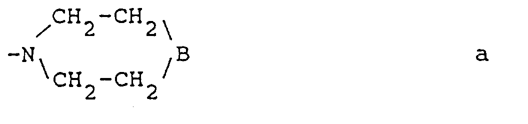 dans laquelle
B représente une liaison, le groupe méthylène, un atome d'oxygène ou un groupe imino -NR⁶-, R⁶ représentant un atome d'hydrogène, un groupe alkyle inférieur ou un groupe phényle ou benzyle éventuellement substitué sur le noyau phényle par un ou des groupes alkyle inférieur, alcoxy inférieur, hydroxy ou par un ou des atomes d'halogène, et
D représente une liaison ou, lorsque R⁴ et R⁵ ne représentent pas des atomes d'hydrogène, également le groupe -N=CH-, et dans le cas où les substituants sont des groupes alkyle inférieurs, ces derniers renferment de 1 à 4 atomes de carbone.
et de leurs sels d'addition avec des acides physiologique ment acceptables, caractérisé en ce que
a) pour la préparation de composés de formule générale Ia 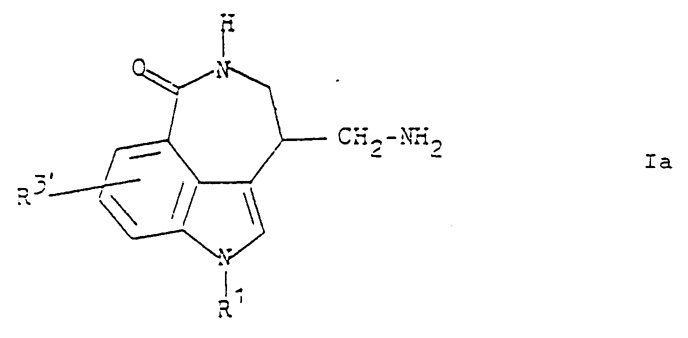 dans laquelle R¹ a la signification donnée plus haut et R^{3'} a la signification donnée pour R³, à l*'*exception du groupe hydroxy, on cyclise, dans des conditions réductrices, des composés de formule générale II 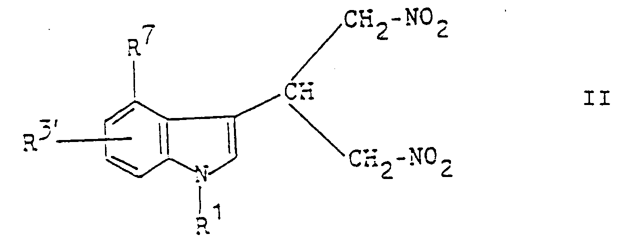 dans laquelle R¹ et R^{3'} ont les significations données plus haut, et R⁷ représente un groupe alcoxycarbonyle inférieur ou le groupe CN, ou
b) pour la préparation de composés de formule générale Ib 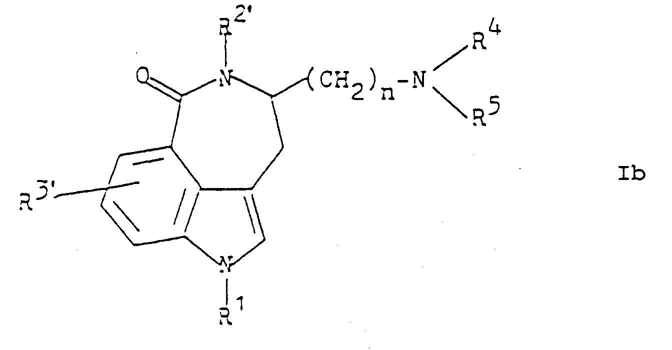 dans laquelle R¹, R^{3'}, R⁴, R⁵ et n ont les significations données plus haut, et R^{2'} représente un atome d*'*hydrogène ou un groupe alkyle inférieur, on fait réagir des composés de formule générale III 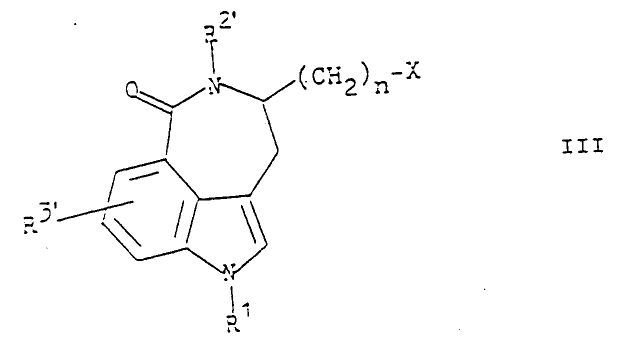 dans laquelle R¹, R^{2'}, R^{3'} et n ont les significations données plus haut et X représente un groupe séparable par substitution nucléophile, avec des composés de formule générale IV 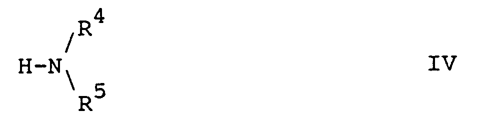 dans laquelle R⁴ et R⁵ ont les significations données plus haut, ou
c) pour la préparation de composés de formule générale Ic 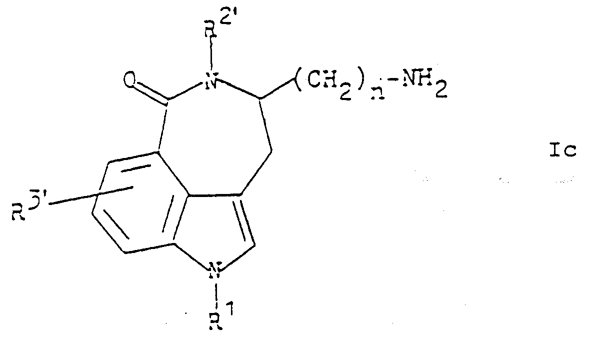 dans laquelle R¹, R^{2'}, R^{3'} et n ont les significations données plus haut, on convertit le groupe Y en groupe amino dans des composés de formule générale V 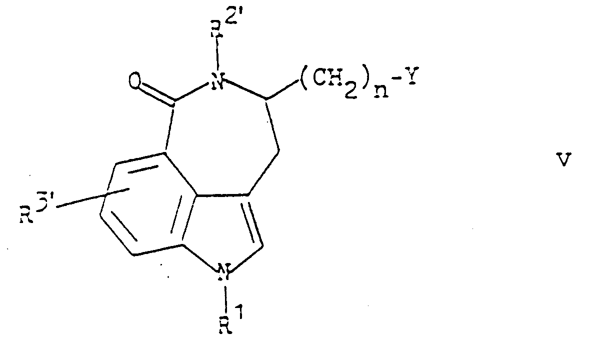 dans laquelle R¹, R^{2'}, R^{3'} et n ont les significations données plus haut et Y représente un groupe azide ou phtalimide, ou, lorsque n est 1, également le groupe cyano, ou
d) pour la préparation de composés de formule générale Id 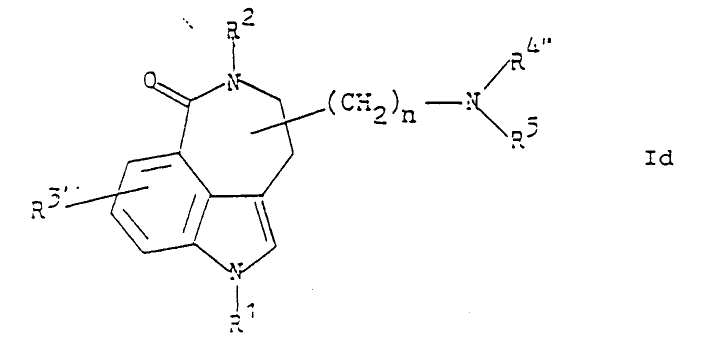 dans laquelle R¹, R², R^{3'}, R⁵ et n ont les significations données plus haut, et R^{4''} a la signification donnée pour R⁴, à l*'*exception d*'*un atome d*'*hydrogène, on soumet à une alkylation des composés de formule générale VI 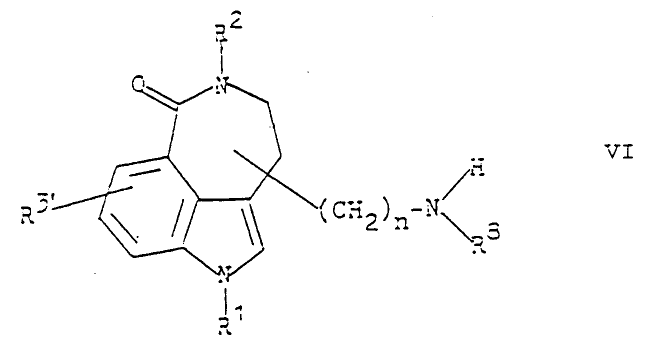 dans laquelle R¹, R², R^{3'} et n ont les significations données plus haut, et R⁸ représente un atome d*'*hydrogène ou un groupe alkyle ayant de 1 à 5 atomes de carbone, cycloalkyle ayant de 3 à 6 atomes de carbone, cycloalkylalkyle ayant de 4 à 9 atomes de carbone, un groupe phénylalkyle inférieur éventuellement mono- ou disubstitué sur le noyau phényle par un ou des atomes d*'*halogène ou groupes alkyle inférieur, alcoxy inférieur ou hydroxy, et on élimine à nouveau un groupe R⁸ protecteur de fonction amino, éventuellement présent, ou
e) pour la préparation de composés de formule générale Ie 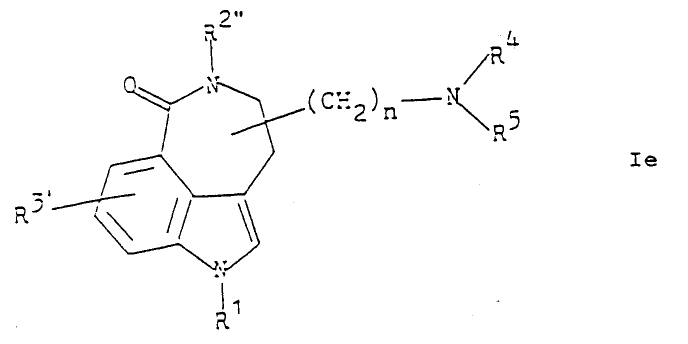 dans laquelle R¹, R^{3'}, R⁴, R⁵ et n ont les significations données plus haut, et R^{2''} représente un groupe alkyle inférieur éventuellement substitué par un groupe alcoxy inférieur en position α par rapport à l*'*atome d*'*azote, on convertit en radical R^{2''} le groupe 1-hydroxyalkyle dans des composés de formule générale VII 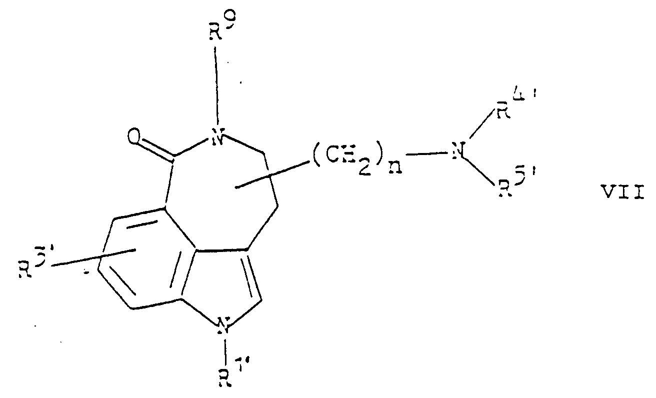 dans laquelle R^{3'} et n ont les significations données plus haut, R^{1'} a la signification donnée pour R¹ ou représente un groupe protecteur de fonction amino, R^{4'} et R^{5'} ont les significations données pour R⁴ et R⁵, un groupe NR⁴R⁵, dans lequel R⁴ et/ou R⁵ représente(nt) un ou des atomes d*'*hydrogène, étant toutefois protégé par au moins un groupe protecteur de fonction amino, aisément séparable, de manière qu*'*il ne réagisse pas avec des agents d*'*acylation ou d*'*alkylation, et R⁹ représente un groupe 1-hydroxyalkyle inférieur, et on élimine à nouveau des groupes protecteurs de fonction amino, éventuellement présents, ou
f) pour la préparation de composés de formule générale If 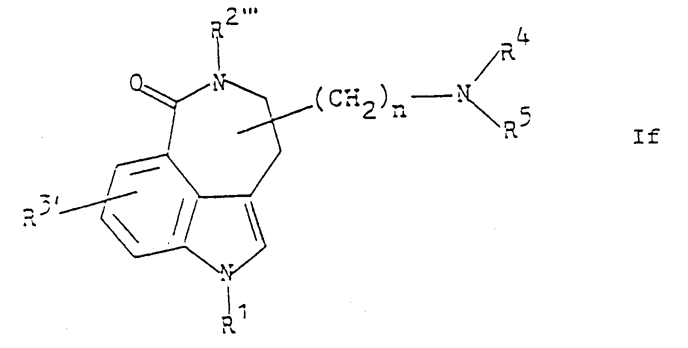 dans laquelle R¹, R^{3'}, R⁴, R⁵ et n ont les significations données plus haut, et R^{2'''} représente un groupe alkyle inférieur, on fait réagir des composés de formule générale VIII 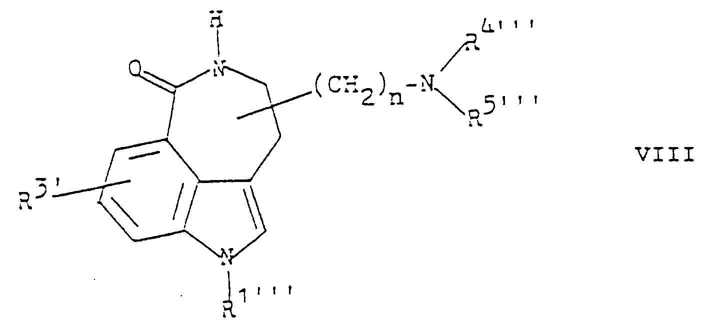 dans laquelle R^{3'} et n ont les significations données plus haut, et R^{1'''}, R^{4'''} et R^{5'''} ont les significations données pour R¹, R⁴ et R⁵, à l*'*exception d*'*un atome d*'*hydrogène, ou représentent un groupe protecteur de fonction amino, avec des composés de formule générale XII
R^{2'''}-X XII
dans laquelle R^{2'''} et X ont les significations données plus haut, et ensuite on élimine à nouveau des groupes protecteurs de fonction amino éventuellement présents, ou
g) pour la préparation de composés de formule générale Ig 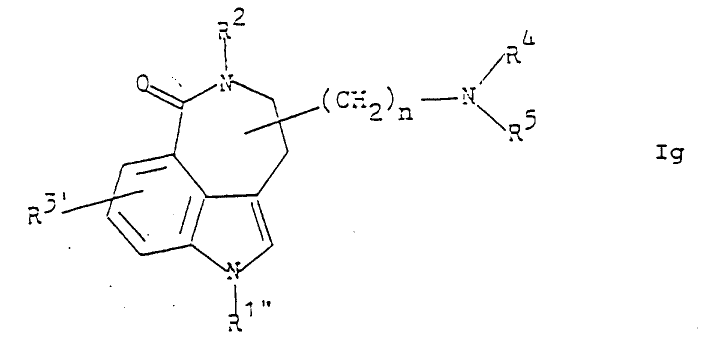 dans laquelle R², R^{3'}, R⁴, R⁵ et n ont les significations données plus haut, et R^{1''} a la signification donnée pour R¹, à l*'*exception d'un atome d*'*hydrogène, on fait réagir des composés de formule générale IX 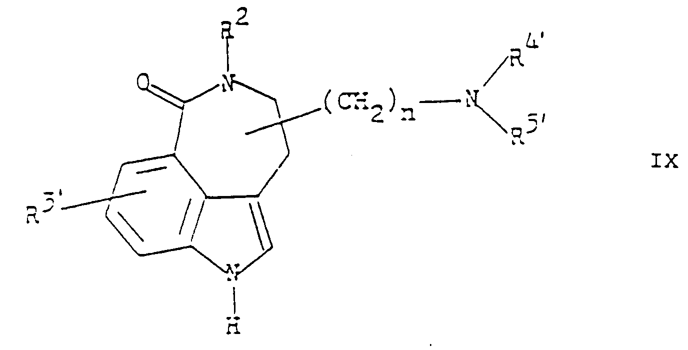 dans laquelle R², R^{3'}, R^{4'}, R^{5'} et n ont les significations données plus haut, avec des composés de formule générale X
R^{1''}-X X
dans laquelle R^{1''} et X ont les significations données plus haut, et ensuite on élimine à nouveau des groupes protecteurs de fonction amino éventuellement présents, ou
h) pour la préparation de composés de formule générale Ih 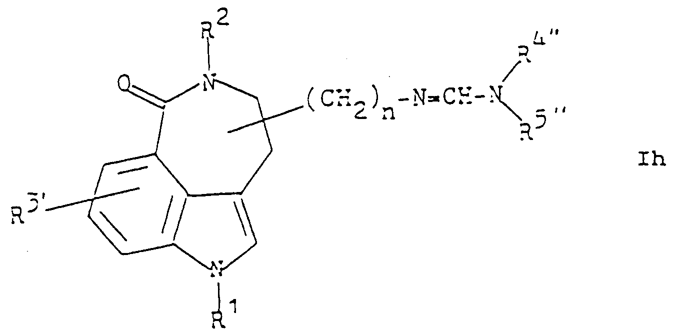 dans laquelle R¹, R², R^{3'}, R^{4''} et n ont les significations données plus haut et R^{5''} a la signification donnée pour R⁵, à l*'*exception d*'*un atome d*'*hydrogène, on introduit le radical aminométhyle de formule générale b 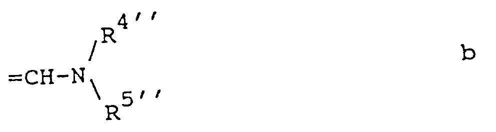 dans laquelle R^{4''} et R^{5''} ont les significations données plus haut, dans des composés de formule générale Ii 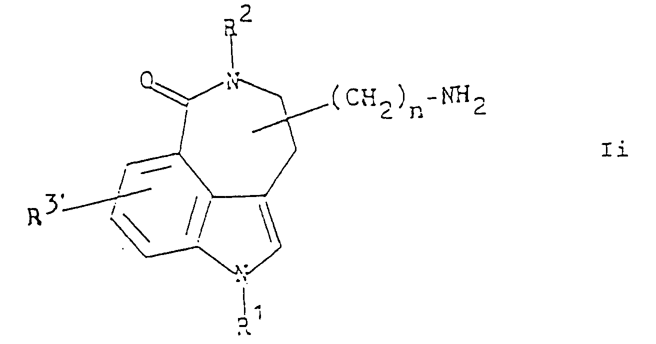 dans laquelle R¹ R², R^{3'} et n ont les significations données plus haut, et dans le cas où les substituants sont des groupes alkyls inférieurs, ces derniers renferment de 1 à 4 atomes de carbone,
et éventuellement dans des composés de formule I obtenus, dans lesquels R^{3'} représente le groupe méthoxy et/ou R¹, R⁴ et/ou R⁵ contiennent un groupe méthoxyphényle, on dissocie le groupe méthoxy en le groupe hydroxy, et/ou dans des composés de formule I obtenus, dans lesquels R¹, R⁴, R⁵ et/ou R⁶ représentent un groupe benzyle éventuellement substitué, on élimine par hydrogénolyse ce groupe benzyle, et, si on le désire, on convertit des composés de formule I libres en leurs sels d'addition avec des acides, ou on convertit les sels d'addition avec des acides en composés de formule I libres.

9. Composés de formule générale II 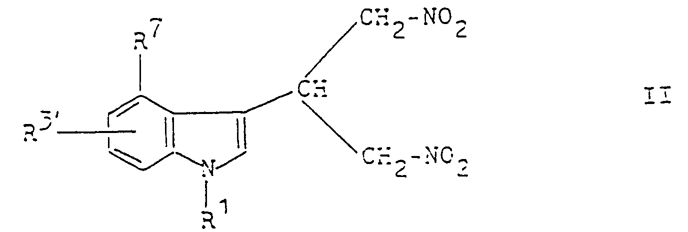 dans laquelle
R¹ représente un atome d'hydrogène ou un groupe alkyle inférieur ou cycloalkylalkyle ou un groupe phénylalkyle inférieur qui peut éventuellement être mono- ou disubstitué sur le noyau phényle par un ou des atomes d'halogène ou groupes alcoxy inférieur, hydroxy ou alkyle inférieur ;
R^{3'} représente un atome d'hydrogène ou d'halogène ou un groupe alkyle inférieur ou alcoxy inférieur, et
R⁷ représente un groupe alcoxycarbonyle inférieur ou le groupe CN, et dans le cas où les substituants sont des groupes alkyls inférieurs, ces derniers renfermant de 1 à 4 atomes de carbone.

10. Composés de formule générale VII 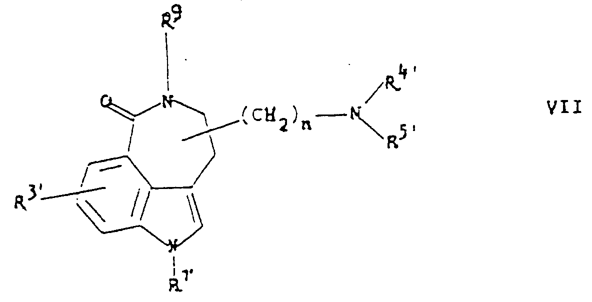 dans laquelle
R^{1'} représente un atome d'hydrogène ou un groupe alkyle inférieur ou cycloalkylalkyle ou un groupe phényl-alkyle inférieur qui peut éventuellement être mono- ou disubstitué sur le noyau phényle par un ou des atomes d'halogène ou groupes alcoxy inférieur, hydroxy ou alkyle inférieur ou représente un groupe protecteur de fonction amino ;
R^{3'} représente un atome d'hydrogène ou d'halogène ou un groupe alkyle inférieur ou alcoxy inférieur ;
n représente 1 ou, lorsque la chaîne -(CH₂)ₙ- est disposée en position 4 du squelette cyclique, également 2;
R^{4'} représente un atome d'hydrogène ou un groupe alkyle ayant de 1 à 5 atomes de carbone, cycloalkyle ayant de 3 à 6 atomes de carbone, cycloalkylalkyle ayant de 4 à 9 atomes de carbone, ou un groupe phényl-alkyle inférieur éventuellement mono- ou disubstitué sur le noyau phényle par un ou des atomes d'halogène ou groupes alkyle inférieur, alcoxy inférieur ou hydroxy; et
R^{5'} représente un atome d'hydrogène ou un groupe alkyle ayant de 1 à 5 atomes de carbone, cycloalkyle ayant de 3 à 6 atomes de carbone, cycloalkylalkyle ayant de 4 à 9 atomes de carbone, ou un groupe phényl-alkyle inférieur éventuellement mono- ou disubstitué sur le noyau phényle par un ou des atomes d'halogène ou groupes alkyle inférieur, alcoxy inférieur ou hydroxy, ou
R^{4'} et R^{5'} forment, conjointement avec l'atome d'azote auquel ils sont liés, un hétérocycle de formule générale a 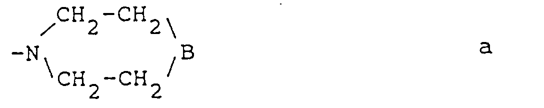 dans laquelle
B représente une liaison, le groupe méthylène, un atome d'oxygène ou un groupe imino - NR⁶-, R⁶ représentant un atome d'hydrogène, un groupe alkyle inférieur ou un groupe phényle ou benzyle éventuellement substitué sur le noyau phényle par un ou des groupes alkyle inférieur, alcoxy inférieur, hydroxy ou par un ou des atomes d'halogène, un groupe NR^{4'}R⁵(, dans le quel R^{4'} et/ou R^{5'} représente(nt) un atome d'hydrogène, étant toutefois protégé par au moins un groupe protecteur de fonction amino aisément séparable, et
R⁹ représente un groupe 1-hydroxyalkyle inférieur et dans le cas où les substituants sont des groupes alkyle inférieurs, ces derniers renferment de 1 à 4 atomes de carbone.

11. Composés de formule générale XXVI 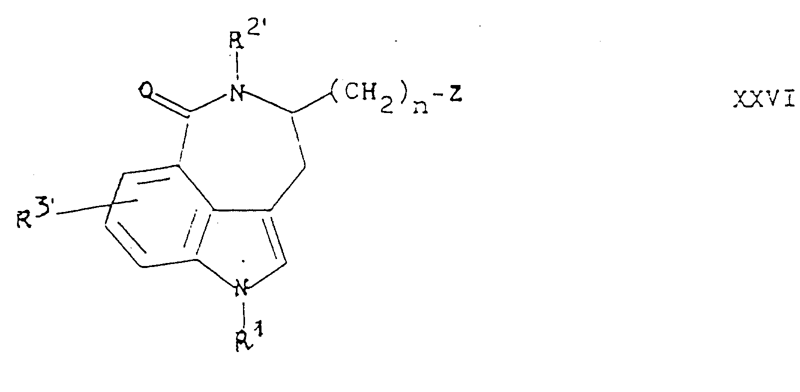 dans laquelle
R¹ représente un atome d'hydrogène ou un groupe alkyle inférieur ou cycloalkylalkyle ou un groupe phényl-alkyle inférieur qui peut éventuellement être mono- ou disusbtitué sur le noyau phényle par un ou des atomes d'halogène ou groupes alcoxy inférieur, hydroxy ou alkyle inférieur;
R^{2'} représente un atome d'hydrogène ou un groupe alkyle inférieur;
R^{3'} représente un atome d'hydrogène ou d'halogène ou un groupe alkyle inférieur ou alcoxy inférieur ;
n représente 1 ou 2; et
Z représente le groupe hydroxy, un groupe séparable pouvant être éliminé par substitution nucléophile, un groupe azide ou phatlimide ou, lorsque n est 1, également le groupe cyano, et dans le cas où les substituants sont des groupes alkyle inférieurs, ces derniers renferment de 1 à 4 atomes de carbone.
